(19) Europäisches Patentamt
European Patent Office
Office européen des brevets

(11) Numéro de publication: **0 373 061 B1**

(12) # FASCICULE DE BREVET EUROPEEN

(45) Date de publication de fascicule du brevet: **16.02.94**

(51) Int. Cl.[5]: **C07D 495/04**, A61K 31/40, //(C07D495/04,337:00,209:00), (C07D495/04,335:00,209:00)

(21) Numéro de dépôt: **89403356.2**

(22) Date de dépôt: **05.12.89**

(54) **Nouveaux dérivés de l'indole, leur procédé de préparation et les compositions pharmaceutiques qui les contiennent.**

(30) Priorité: **06.12.88 FR 8815937**

(43) Date de publication de la demande: **13.06.90 Bulletin 90/24**

(45) Mention de la délivrance du brevet: **16.02.94 Bulletin 94/07**

(84) Etats contractants désignés: **AT BE CH DE ES FR GB GR IT LI LU NL SE**

(56) Documents cités:
**EP-A- 0 035 259**
**FR-A- 2 426 689**
**GB-A- 2 153 821**

**INDIAN JOURNAL OF CHEMISTRY, vol. 20B, août 1981, pages 672-679, IN; K. NAGARAJAN et al.: "Derivatives of 3-mercaptoindole - synthesis of a potent vasoconstrictor, 3-(2-imidazolin-2-ylthio)indole (tinazoline)"**

(73) Titulaire: **ADIR ET COMPAGNIE**
**1 rue Carle Hébert**
**F-92415 Courbevoie Cédex(FR)**

(72) Inventeur: **Malen, Charles**
**3 allée Traversière**
**F-94260 Fresnes(FR)**
Inventeur: **Lacoste, Jean-Michel**
**103 rue Brancas**
**F-92310 Sèvres(FR)**
Inventeur: **Laubie, Michel**
**35 avenue Foch**
**F-92420 Vaucresson(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 373 061 B1

## Description

La présente invention concerne de nouveaux dérivés de l'indole, leur procédé de préparation et les compositions pharmaceutiques qui les contiennent.

Plus particulièrement l'invention concerne des dérivés possédant des propriétés antagonistes des récepteurs à la 5 - hydroxytryptamine (5 - HT) plus particulièrement aux récepteurs 5 - $HT_3$, récepteurs que l'on sait impliqués dans de nombreux troubles, tant centraux que périphériques.

On connaît déjà les propriétés antagonistes aux récepteurs 5 - $HT_3$ d'un certain nombre de tétrahydrocarbazolones - 4 décrites notamment dans les demandes de brevets européens n° 0 191 562, 0 210 840, 0 219 929, 0 275 668, 0 275 669 ainsi que dans la demande de brevet français 2 601 951. Parmi l'ensemble des composés décrits dans ces demandes de brevets l'un d'eux, référencé GR 38032 possède une puissante activité antagoniste aux récepteurs 5 - $HT_3$. Il s'agit du dérivé de formule :

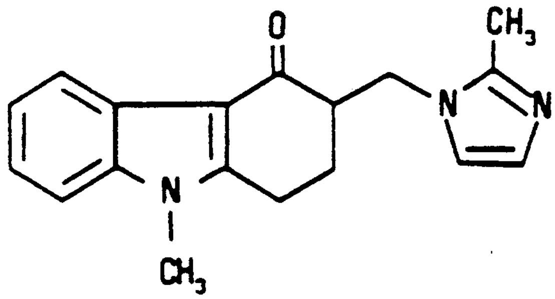

La demanderesse a présentement découvert de nouveaux dérivés de l'indole dont l'activité antagoniste aux récepteurs 5 - $HT_3$ est significativement supérieure à celle du composé de référence GR 38032. En outre leur durée d'action est significativement plus longue, ce qui les rend plus apte à une application thérapeutique.

Plus spécifiquement, la présente invention concerne des dérivés de formule (I) :

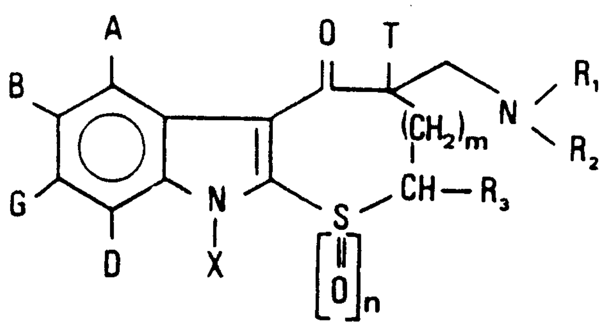

(I)

dans laquelle :

- A, B, G, D : identiques ou différents représentent chacun un atome d'hydrogène ou un atome d'halogène, un groupement alkoxy inférieur ou encore un groupement alkyle inférieur linéaire ou ramifié éventuellement substitué par un ou plusieurs atomes d'halogène,
- X : représente un atome d'hydrogène, un groupement alkyle inférieur linéaire ou ramifié ou un groupement $SO_2E$ dans laquelle E représente un groupement alkyle inférieur linéaire ou ramifié ou phényle éventuellement substitué par un groupement alkyle inférieur linéaire ou ramifié,
- T : représente un atome d'hydrogène ou un groupement alkyle inférieur,
- $R_3$ : représente un atome d'hydrogène ou un groupement alkyle inférieur linéaire ou ramifié ou un groupement phényle éventuellement substitué par un ou plusieurs groupements alkyle inférieurs linéaires ou ramifiés,
- n et m : identiques ou différents représentent chacun 0 ou 1,
- $R_1$ et $R_2$ : identiques ou différents représentent chacun un atome d'hydrogène ou un groupement

alkyle inférieur linéaire ou ramifié,

ou bien,

$R_1$ et $R_2$ forment ensemble avec l'atome d'azote auxquels ils sont liés un système hétérocyclique azoté, mono ou bicyclique, saturé ou non, chaque cycle étant à cinq ou six sommets, comprenant éventuellement un ou deux autres hétéroatomes choisis parmi azote, oxygène ou soufre, éventuellement substitué par un ou plusieurs groupements alkyle ou alkoxy inférieur, linéaires ou ramifiés ou par un groupement aryle éventuellement lui-même substitué par un ou plusieurs groupements alkyle inférieurs, alkoxy inférieurs, trifluorométhyle ou bien encore un ou plusieurs atomes d'halogène,

ou bien,

$R_1$ représente un groupement

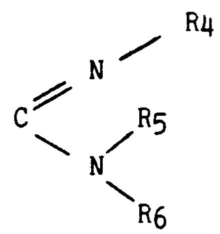

dans laquelle $R_4$, $R_5$, $R_6$ identiques ou différents représentent chacun un atome d'hydrogène ou un groupement alkyle inférieur linéaire ou ramifié ou bien $R_4$ forme avec $R_5$ un pont $(CH_2)p$, p étant compris entre 2 et 4,

ou bien encore $R_1$ représente un ensemble dihydro - 10,11 [5H] dibenzo [a,d] cycloheptényl - 5 et $R_2$ représente un atome d'hydrogène,

le cas échéant leurs isomères ainsi que leurs sels d'addition à un acide pharmaceutiquement acceptable,

étant entendu que par groupement alkyle inférieur ou alkoxy inférieur, on entend des groupements comprenant de 1 à 6 atomes de carbone.

Parmi les acides que l'on peut ajouter aux composés de formule (I) pour former un sel d'addition, on peut citer, à titre d'exemple, les acides chlorhydrique, sulfurique, phosphorique, tartrique, malique, maléique, fumarique, oxalique, succinique, méthane-sulfonique, éthane-sulfonique, camphorique, citrique, etc...

L'invention s'étend aussi au procédé d'obtention des composés de formule (I), caractérisé en ce que l'on condense un dérivé de formule (II) :

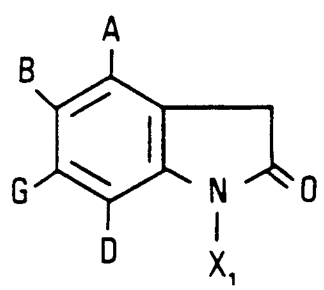

(II)

dans laquelle A, B, G, D ont la même signification que dans la formule (I),

et $X_1$ représente un atome d'hydrogène ou un groupement alkyle inférieur,

que l'on traite en présence d'un agent de thionation tel que le pentasulfure de phosphore pour obtenir un dérivé de formule (III) :

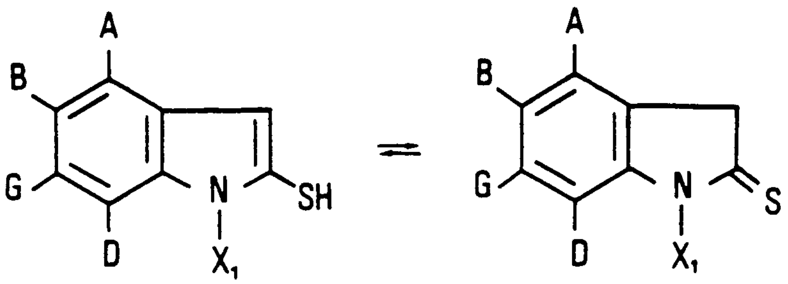

(III)

dans laquelle A, B, G, D et $X_1$ ont la même signification que précédemment,
que l'on traite par un dérivé de formule (IV) :

$JCHR_3—(CH_2)_m—CTK—COOR$ (IV)

dans laquelle :
ou bien
m représente 0 et dans ce cas J et K représentent ensemble une liaison $\pi$,
ou bien
m représente 1 et dans ce cas J représente un atome d'halogène, et K représente un atome d'hydrogène,
R représente un atome d'hydrogène ou un groupement alkyle inférieur linéaire ou ramifié et T et $R_3$ ont la même signification que dans la formule (I),
pour obtenir un dérivé de formule (V) :

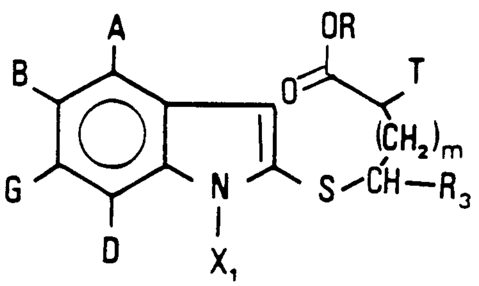

(V)

dans laquelle A, B, G, D, T, R, $R_3$, $X_1$ et m ont la même signification que précédemment,
qui, lorsque R est différent de H est soumis à l'action d'un agent alcalin pour conduire à un dérivé de formule (Va), cas particulier des dérivés de formule (V) pour lesquels R représente H,

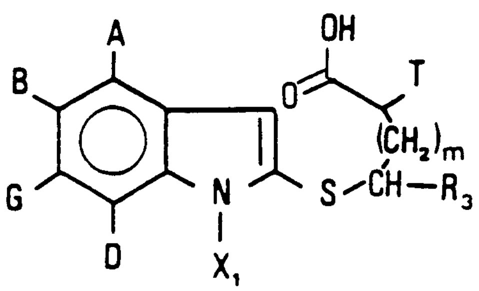

(Va)

que l'on soumet à l'action de polyphosphate ester préparé selon W. POLLMAN et G. SCHRAMM (Biochem Biophysica Acta, 1963, 80, 1) préférentiellement sous atmosphère d'azote pour conduire à un dérivé de formule (VI) :

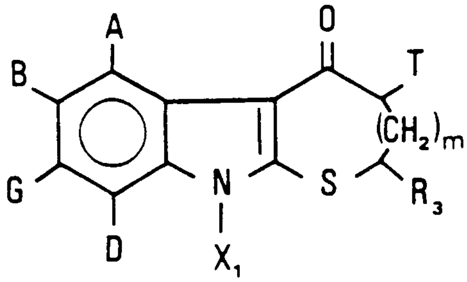

(VI)

dans lequel A, B, G, D, T, m $X_1$ et $R_3$ ont la même signification que précédemment,
que l'on traite ensuite par la paraformaldéhyde préférentiellement sous atmosphère d'azote,
en présence d'une dialkylamine,

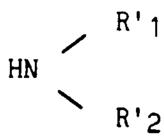

ou d'un de ses sels d'acide fort,
dans laquelle $R'_1$ et $R'_2$ signifient alkyle inférieur,
pour conduire à un dérivé de formule (I/A), cas particulier des dérivés de formule (I) :

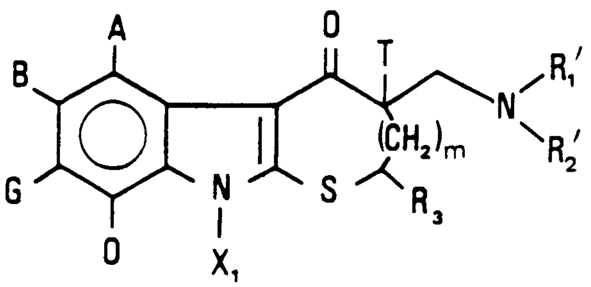

(I/A)

dans laquelle A, B, G, D, $R_3$, T, m et $X_1$ ont la même signification que précédemment et $R'_1$ et $R'_2$ signifient un groupement alkyle inférieur, que l'on purifie si nécessaire par chromatographie ou/et cristallisation qui,

- lorsque, dans le dérivé de formule (I) que l'on souhaite obtenir, $R_1$ et $R_2$ forment avec l'atome d'azote auquel ils sont attachés un système hétérocyclique tel que défini dans la formule (I), est traité ensuite par un composé hétérocyclique azoté, l'atome d'azote étant lié à un atome d'hydrogène, le composé hétérocyclique étant mono ou bicyclique, saturé ou non, chaque cycle comprenant cinq ou six sommets et éventuellement un ou deux autres hétéroatomes choisis parmi azote oxygène ou soufre, et éventuellement substitué par un ou plusieurs groupements alkyle ou alkoxy inférieurs linéaires ou ramifiés, ou par un groupement aryle éventuellement lui-même substitué par un ou plusieurs groupements alkyle inférieurs, alkoxy inférieurs, trifluorométhyle, ou bien encore un ou plusieurs atomes d'halogène pour donner un composé de formule (I/B) :

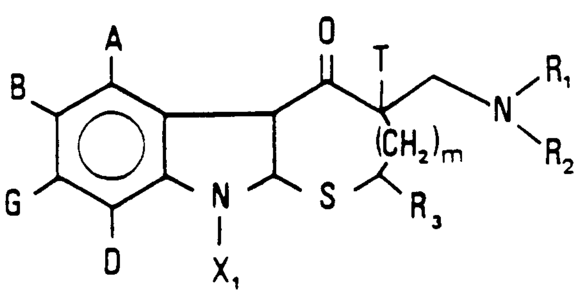

(I/B)

cas particulier des dérivés de formule (I) dans lesquels A, B, G, D, $R_3$, T, m et $X_1$ ont la même signification que précédemment et $R_1$, $R_2$ forment avec l'atome d'azote auquel ils sont attachés un système hétérocyclique tel que défini précédemment,

. lorsque dans le dérivé de formule (I) que l'on souhaite obtenir $R_1$ et $R_2$ représentent chacun un atome d'hydrogène, est traité par la dihydro - 10,11 - [5H] dibenzo [a,d] cycloheptenyl - 5 amine pour conduire après purification par chromatographie et cristallisation, à un dérivé de formule (I/C1) :

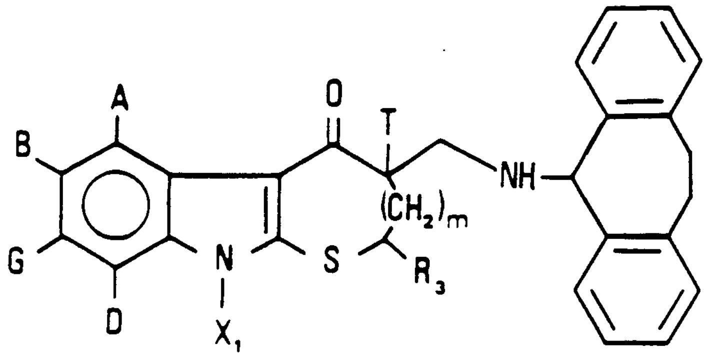

(I/C1)

cas particulier de la formule (I) dans laquelle A, B, G, D, $R_3$, T, m et $X_1$ ont la même signification que précédemment, que l'on traite par chauffage dans l'acide acétique, éventuellement dilué, pour conduire après éventuelle cristallisation à un dérivé de formule (I/C) :

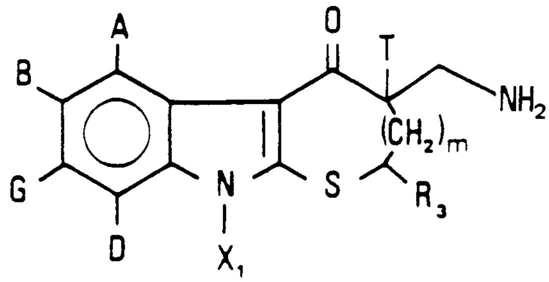

(I/C)

cas particulier des dérivés de formule (I) dans laquelle A, B, G, D, $R_3$, T, m et $X_1$ ont la même signification que précédemment,
que l'on purifie si nécessaire par chromatographie et/ou cristallisation et qui, lorsque dans le dérivé de formule (I) que l'on souhaite obtenir $R_1$ représente un groupement :

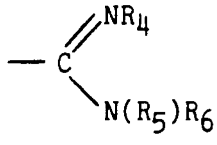

dans lequel $R_4$, $R_5$ et $R_6$ ont la même définition que dans la formule (I), est traité par un sel d'acide fort d'un dérivé de formule (VII) :

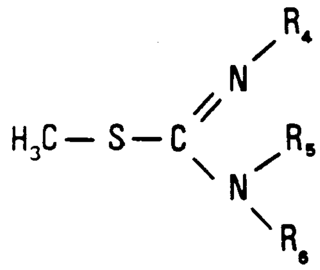

(VII)

dans laquelle $R_4$, $R_5$ et $R_6$ ont la même définition que dans la formule (I),
pour conduire, après éventuelle purification par chromatographie, traitement par un sel de métal lourd, et éventuelle cristallisation, à un dérivé de formule (I/D) :

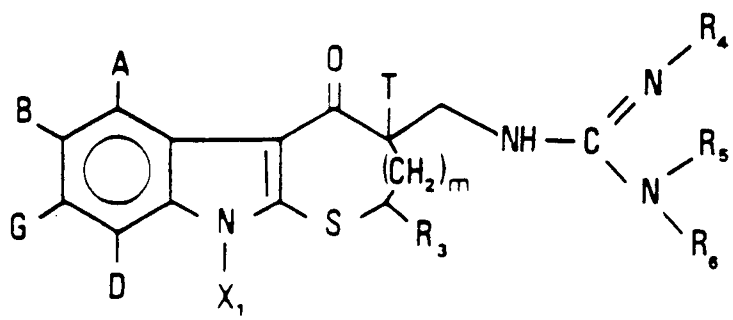

(I/D)

cas particulier de dérivé de formule (I) dans laquelle $R_1$ représente un groupement :

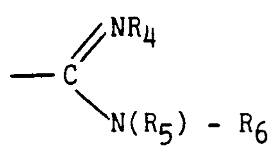

et $R_2$ représente un atome d'hydrogène,
et où A, B, G, D, $R_3$, T, m et $X_1$ ont la même signification que précédemment,
qui, dans le cas où $X_1$ représente un atome d'hydrogène peut être soumis, en fonction du composé de formule (I) que l'on désire obtenir, à l'action d'un composé de formule (VIII) :

Hal—$SO_2E$ (VIII)

dans laquelle E a la même signification que dans la formule (I) et Hal représente un atome d'halogène, en présence d'hydrure de sodium, pour conduire après extraction et éventuelle purification par chromatographie sur colonne à un dérivé de formule (I/E) :

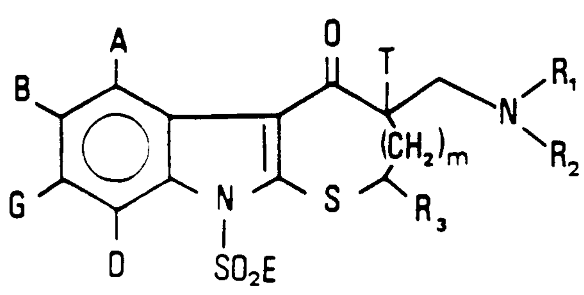

(I/E)

cas particulier des dérivés de formule (I) dans lequel X représente $SO_2E$ et où A, B, G, D, $R_1$, $R_2$, $R_3$, T, m et E ont la même signification que dans la formule (I),
dérivé de formule (I/A), (I/B), (I/C1), (I/C), (I/D) ou (I/E) qui, dans le cas où dans le composé de formule (I) que l'on souhaite obtenir, n représente 1, est traité par un agent oxydant, préférentiellement l'acide métachloroperbenzoïque pour conduire après éventuelle purification par chromatographie et/ou cristallisation au dérivé de formule (I/F):

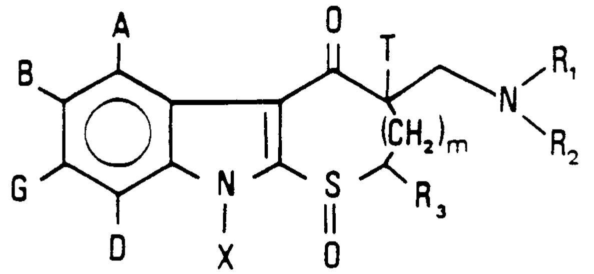

(I/F)

cas particulier des dérivés de formule (I) où A, B, G, D, X, T, $R_1$, $R_2$, $R_3$ et m ont la même signification que dans la formule (I), n représentant ici 1,
dérivé de formule (I/A), (I/B), (I/C1), (I/C), (I/D), (I/E) ou (I/F) qui, si on le désire peut être le cas échéant séparé en ses isomères et/ou salifié soit pour en faciliter la purification pour les éventuelles étapes ultérieures, soit salifié par un acide pharmaceutiquement acceptable et éventuellement cristallisé pour usage à des fins thérapeutiques.

Les composés de formule (I) ainsi que leurs sels d'addition à un acide pharmaceutiquement acceptable possèdent d'intéressantes propriétés pharmacologiques.

En particulier, les composés de la présente invention montrent une activité antagoniste des récepteurs sérotoninergiques 5-$HT_3$ particulièrement intense et dont la durée est étonnamment longue.

Notamment, les composés de la présente invention administrés par voie intraveineuse chez le rat, à des doses comprises entre 10 $\mu$g/kg et 1 mg/kg antagonisent de façon intense et prolongée l'effet Bezold-Jarish (bradycardie et hypotension après injection de sérotonine). Cette propriété est indicatrice d'un effet antagoniste des récepteurs 5-$HT_3$.

Par conséquent, les composés de la présente invention trouvent leurs applications thérapeutiques dans le traitement des états découlant d'un dysfonctionnement du système sérotoninergique et dans lesquels le blocage des récepteurs 5-$HT_3$, - qu'ils soient centraux ou périphériques - est réputé comme pouvant avoir des effets bénéfiques.

Parmi ces états, on peut citer la migraine et plus généralement les épisodes douloureux, l'anxiété, la schizophrénie, les vomissements et notamment ceux consécutifs à l'administration de traitements anticancéreux, les troubles du rythme cardiaque, certains désordres gastro-intestinaux, ainsi que les troubles cognitifs et particulièrement les états démentiels et les troubles de la mémoire.

L'invention a également pour objet les compositions pharmaceutiques contenant un composé de formule (I) ou un de ses sels d'addition à un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration parentérale, per ou transcutanée, nasale, rectale, perlinguale, oculaire ou respiratoire, et notamment les préparations injectables, les aérosols, les gouttes oculaires ou nasales, les capsules, les gélules, les comprimés, les comprimés sublinguaux, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 microgramme et 1 gramme par prise ou par application.

Les exemples suivants illustrent l'invention, et ne la limitent en aucune façon.

Les points de fusion indiqués sont mesurés selon la technique du micro-Kofler. Les spectres de résonance magnétique nucléaire $^1$H ont été enregistrés en utilisant le TMS comme référence interne.

**EXEMPLE 1 :[(METHYL - 2 IMIDAZOLYL - 1) METHYL] - 3 OXO - 4 METHYL - 9 TETRAHYDRO - 2,3,4,9 THIOPYRANO [2,3-b] INDOLE (CHLORHYDRATE) ainsi que le composé libre et d'autres sels pharmaceutiquement acceptables de ce dernier.**

**STADE A : METHYL - 1 INDOLINE THIONE - 2**

A une suspension de 130 g (0,8 mole) de méthyl - 1 indolinone - 2 préparé selon R.A. ABRAMOVITCH, DH. HEY (J. Chem. Soc., 1954, 1699) et de 133 g (0,3 mole) de pentasulfure de phosphore dans 1,30 l de tétrahydrofuranne anhydre, ajouter sous vive agitation et à température ambiante, 148 g (1,76 mole) de bicarbonate de sodium par portions de 20 grammes. Après 10 heures d'agitation à température ambiante, filtrer et concentrer le filtrat au bain-marie sous vide. Reprendre le résidu par un mélange eau/glace et extraire à trois reprises au chloroforme. Laver la phase organique à l'eau puis par une solution aqueuse saturée en chlorure de sodium, sécher et évaporer à sec. Le résidu est recristallisé dans le méthanol.

Rendement : 93 %

Point de fusion : 117 - 118 °C

| Analyse : | | | | | | |
|---|---|---|---|---|---|---|
| Calculée | C | 66,22 | N | 8,58 | S | 19,64 |
| Trouvée | C | 66,62 | N | 8,63 | S | 19,76 |

**STADE B : ACIDE (METHYL - 1 INDOLYL - 2 THIO) - 3 PROPIONIOUE**

A une suspension fortement agitée de 100 g (0,613 mole) de méthyl - 1 indoline thione - 2 obtenue au stade A, dans 46,8 g (0,65 mole) d'acide acrylique, ajouter lentement 600 ml de triéthylamine. Chauffer à reflux 12 heures, laisser refroidir et concentrer sous vide. Reprendre le résidu par une solution aqueuse de bicarbonate de sodium à 10 % et filtrer. Traiter le filtrat par de l'acide chlorhydrique dilué au demi jusqu'à l'obtention d'un pH voisin de 3. Essorer le précipité obtenu, laver à l'eau et sécher. Recristalliser dans le toluène.

Rendement : 70%

Point de fusion : 117 - 119 °C

| Analyse : | | | | | | | | |
|---|---|---|---|---|---|---|---|---|
| Calculée | C | 61,25 | H | 5,57 | N | 5,95 | S | 13,63 |
| Trouvée | C | 61,66 | H | 5,64 | N | 5,72 | S | 13,71 |

**STADE C : OXO - 4 METHYL - 9 TETRAHYDRO - 2,3,4,9 THIOPYRANO [2,3-b] INDOLE**

Placer 88,2 g (0,375 mole) d'acide (méthyl - 1 indolyl - 2 thio) - 3 propionique obtenu au stade B et 300 g de polyphosphate ester préparé selon W. POLLMANN, et G. SCHRAMM (Biochem. Biophysica Acta, 80, 1 (1963)) dans 3,5 l de chloroforme. Agiter à température ambiante et sous atmosphère d'azote pendant 16 heures. Après hydrolyse par 3 litres d'eau glacée, décanter la phase organique et extraire la phase aqueuse à 2 reprises par du chloroforme. Réunir les phases organiques, laver par une solution de bicarbonate de sodium à 10 %, puis à l'eau, sécher et évaporer le milieu organique au bain-marie sous vide. Recristalliser le résidu dans l'acétonitrile.

Rendement : 87 %

Point de fusion : 164 - 165 °C

| Analyse : | | | | | | | | |
|---|---|---|---|---|---|---|---|---|
| Calculée | C | 66,33 | H | 5,10 | N | 6,45 | S | 14,76 |
| Trouvée | C | 66,18 | H | 5,02 | N | 6,55 | S | 14,72 |

**STADE D : (DIMETHYLAMINO) METHYL - 3 OXO - 4 METHYL - 9 TETRAHYDRO - 2,3,4,9 THIOPYRANO [2,3-b] INDOLE**

Placer en suspension 21,7 g (0,1 mole) d'oxo - 4 méthyl - 9 tétrahydro - 2,3,4,9 thiopyrano [2,3-b] indole, 16,3 g (0,2 mole) de chlorhydrate de diméthylamine et 6 g de paraformaldéhyde dans 280 ml d'acide acétique. Chauffer à 100 °C sous vive agitation et sous atmosphère d'azote pendant 2 heures. Après refroidissement, évaporer le solvant et reprendre le résidu par un mélange acide acétique eau (20/80). Laver à l'acétate d'éthyle et alcaliniser par l'ammoniaque concentrée. Essorer le précipité obtenu, laver à l'eau, sécher et recristalliser dans l'éthanol.

Rendement : 65 %
Point de fusion : 110 - 112 °C

**STADE E : (DIMETHYLAMINO) METHYL - 3 OXO - 4 METHYL - 9 TETRAHYDRO 2,3,4,9 THIOPYRANO [2,3-b] INDOLE (CHLORHYDRATE)**

Dissoudre le (diméthylamino) méthyl - 3 oxo - 4 méthyl - 9 tétrahydro - 2,3,4,9 thiopyrano [2,3-b] indole obtenu au stade D, dans 200 ml de tétrahydrofuranne. Traiter par une solution éthérée d'acide chlorhydrique pendant une nuit. Essorer le produit formé, laver à l'éther éthylique, sécher et recristalliser dans l'éthanol.

Rendement : 80 %
Point de fusion : 280 - 283 °C

| Analyse : | | | | | | | | |
|---|---|---|---|---|---|---|---|---|
| Calculée | C | 57,95 | H | 6,16 | N | 9,01 | S | 10,31 |
| Trouvée | C | 57,53 | H | 6,48 | N | 8,94 | S | 10,31 |

**STADE F : [(METHYL - 2 IMIDAZOLYL - 1) METHYL] - 3 OXO - 4 METHYL - 9 TETRAHYDRO - 2,3,4,9 THIOPYRANO [2,3-b] INDOLE (CHLORHYDRATE)**

Chauffer à reflux pendant 20 heures une solution de 1,3 g (0,0158 mole) de méthyl - 2 imidazole et de 1,80 g (0,0057 mole) de chlorhydrate de [(diméthylamino) méthyl] - 3 oxo - 4 méthyl - 9 tétrahydro - 2,3,4,9 thiopyrano [2,3-b] indole obtenu au stade précédent dans 25 ml d'eau. Après refroidissement, essorer, laver le résidu obtenu à l'eau et sécher. Dissoudre dans l'éthanol chaud et traiter par une solution éthanolique d'acide chlorhydrique. Evaporer et cristalliser dans l'éthanol.

Rendement : 75 %
Point de fusion : 232 - 234 °C

| Analyse : | | | | | | | | | | |
|---|---|---|---|---|---|---|---|---|---|---|
| Calculée | C | 58,70 | H | 5,22 | N | 12,08 | S | 9,22 | Cl | 10,19 |
| Trouvée | C | 58,51 | H | 5,29 | N | 12,07 | S | 9,30 | Cl | 10,33 |

Caractéristiques spectrales :

RMN $^1H$ Solvant $D_2O$ $\delta$(ppm)
$\delta$ = 2,40, singulet, 3H, = N -C ($CH_3$) = N
$\delta$ = 2,60 - 3,40 ppm, massif, 3H, S - $CH_2$ et CO - CH
$\delta$ = 3,20 ppm, singulet, 3H, = N - $CH_3$
$\delta$ = 4,10 - 4,40 ppm, multiplet, 2H, - $CH_2$ - N
$\delta$ = 7,10 - 7,80 ppm, massif, 6H, aromatiques et éthyléniques

**EXEMPLE 2 :DIMETHYL - 2,9 [(METHYL - 2 IMIDAZOLYL - 1) METHYL] - 3 OXO - 4 TETRAHYDRO - 2,3,4,9 THIOPYRANO [2,3-b] INDOLE (CHLORHYDRATE)**

**STADE A : ACIDE (METHYL - 1 INDOLYL - 2 THIO) - 3 METHYL - 3 PROPIONIOUE**

Porter à 140 °C environ sous agitation pendant six heures un mélange de 16,30 g (0,01 mole) de méthyl - 1 indoline thione - 2 obtenue dans l'exemple 1, stade A, de 8,61 g (0,01 mole) d'acide crotonique et de 5 gouttes de pipéridine. Laisser refroidir et reprendre par 500 ml de soude à 5 %, laver la phase aqueuse à l'acétate d'éthyle, acidifier par l'acide chlorhydrique et extraire au chlorure de méthylène à trois reprises. Sécher les phases organiques et concentrer au bain-marie sous vide. Le résidu est utilisé tel quel au stade suivant.

Rendement : 70 %

**STADE B : DIMETHYL - 2,9 OXO - 4 TETRAHYDRO - 2,3,4,9 THIOPYRANO [2,3-b] INDOLE**

Agiter à température ambiante sous atmosphère d'azote pendant 3 heures un mélange de 16,20 g (0,065 mole) d'acide (méthyl - 1 indolyl - 2 thio) - 3 méthyl - 3 propanoïque obtenu au stade précédent et de 70 g de polyphosphate ester dans 500 ml de chloroforme. Diluer par 800 ml d'eau glacée, décanter la phase organique et extraire la phase aqueuse à trois reprises au chloroforme. Réunir les phases organiques, laver par une solution aqueuse de bicarbonate à 10 % puis à l'eau, sécher et évaporer le solvant. Recristalliser le résidu dans l'acétonitrile.

Rendement : 60 %

Point de fusion : 159 - 160 °C

| Analyse : | | | | | | | | |
|---|---|---|---|---|---|---|---|---|
| Calculée | C | 67,50 | H | 5,66 | N | 6,06 | S | 13,86 |
| Trouvée | C | 67,44 | H | 5,74 | N | 6,14 | S | 13,85 |

**STADE C : DIMETHYL - 2,9 (DIMETHYLAMINO) METHYL - 3 OXO - 4 TETRAHYDRO - 2,3,4,9 THIOPYRANO [2,3-b] INDOLE (CHLORHYDRATE)**

Agiter à 100 °C sous atmosphère d'azote pendant 2H30 une suspension de 6 g (0,026 mole) de diméthyl - 2,9 oxo - 4 tétrahydro - 2,3,4,9 thiopyrano [2,3-b] indole obtenu au stade précédent, de 4,29 g (0,052 mole) de chlorhydrate de diméthylamine et de 0,40 g de paraformaldéhyde dans 100 ml d'acide acétique. Refroidir, évaporer le solvant et reprendre par 250 ml d'eau. Laver la phase aqueuse à l'acétate d'éthyle, alcaliniser à l'ammoniaque concentrée et extraire à trois reprises au chlorure de méthylène. Réunir les phases organiques, laver par une solution saturée en chlorure de sodium, sécher et évaporer le solvant. Dissoudre le résidu dans 50 ml de tétrahydrofuranne et traiter par une solution éthérée d'acide chlorhydrique. Essorer le précipité formé, laver à l'éther et recristalliser dans l'éthanol.

Rendement : 60 %

Point de fusion : 242 - 244 °C

| Analyse : | | | | | | | | | | |
|---|---|---|---|---|---|---|---|---|---|---|
| Calculée | C | 59,15 | H | 6,52 | N | 8,62 | S | 9,87 | Cl | 10.91 |
| Trouvée | C | 58,82 | H | 6,83 | N | 8,46 | S | 9,83 | Cl | 10,78 |

**STADE D : DIMETHYL - 2,9 [(METHYL - 2 IMIDAZOLYL - 1) METHYL] - 3 OXO - 4 TETRAHYDRO - 2,3,4,9 THIOPYRANO [2,3-b] INDOLE (CHLORHYDRATE)**

Agiter à reflux sous atmosphère d'azote pendant 16 heures une solution de 2 g (0,062 mole) de chlorhydrate de diméthyl - 2,9 (diméthylamino) méthyl - 3 oxo - 4 tétrahydro - 2,3,4,9 thiopyrano - [2,3-b] indole et de 1,64 g (0,020 mole) de méthyl - 2 imidazole dans 30 ml d'eau. Après refroidissement, essorer le précipité formé, laver à l'eau et sécher. Dissoudre la poudre obtenue dans 30 ml de tétrahydrofuranne et

traiter par une solution d'éther chlorhydrique. Le précipité formé est essoré, lavé à l'éther, séché et recristallisé dans l'éthanol. On obtient les deux isomères cis-trans dans la proportion 50/50.

Rendement : 75 %

Point de fusion : 205-207 °C

| Analyse : | | | | | | | | | | |
|---|---|---|---|---|---|---|---|---|---|---|
| Calculée | C | 59,74 | H | 5,57 | N | 11,61 | S | 8,86 | Cl | 9,80 |
| Trouvée | C | 59,66 | H | 5,54 | N | 11,23 | S | 8,63 | Cl | 9,94 |

**EXEMPLE 3 : [(METHYL - 2 IMIDAZOLYL - 1) METHYL] - 3 OXO - 4 METHOXY - 6 METHYL - 9 TETRAHYDRO - 2,3,4,9 THIOPYRANO [2,3-b] INDOLE (CHLORHYDRATE)**

**STADE A : METHYL - 1 METHOXY - 5 INDOLINE THIONE - 2**

A une solution de 19,5 g (0,11 mole) de méthoxy - 5 méthyl - 1 indolinone - 2 préparée selon A. H. BECKETT, R.W. DAISLEY et J. WALKER (Tetrahedron 1968, 24, 6093) et de 16,5 g (0,037 mole) de pentasulfure de phosphore dans 200 ml de tétrahydrofuranne, ajouter 18,5 g (0,22 mole) de bicarbonate de sodium. Chauffer à une température de 50 °C pendant 1H30 puis filtrer le milieu réactionnel et concentrer le milieu réactionnel au bain-marie sous vide. Reprendre le résidu par l'eau glacée et extraire au chlorure de méthylène. Laver la phase organique par une solution saturée de chlorure de sodium, sécher et évaporer. Le résidu est utilisé tel quel dans l'étape suivante.

Rendement : 94 %

Point de fusion : 142 - 144 °C

| Analyse : | | | | | | | | |
|---|---|---|---|---|---|---|---|---|
| Calculée | C | 62,15 | H | 5,74 | N | 7,25 | S | 16,59 |
| Trouvée | C | 62,18 | H | 5,74 | N | 7,06 | S | 16,64 |

**STADE B : ACIDE (METHYL - 1 METHOXY - 5 INDOLYL - 2 THIO) - 3 PROPANOÏOUE**

Ajouter 90 ml de triéthylamine goutte à goutte à une suspension fortement agitée de 19,5 g (0,1 mole) de méthoxy - 5 méthyl - 1 indoline thione - 2, obtenue au stade précédent, dans 7,55 ml (0,11 mole) d'acide acrylique. Chauffer le milieu réactionnel à reflux pendant 6 heures puis refroidir et concentrer sous vide. Reprendre le résidu par une solution aqueuse de bicarbonate à 10 %, filtrer et acidifier la solution obtenue par l'acide chlorhydrique. Après essorage du précipité formé, lavage à l'eau et séchage, recristalliser dans le toluène.

Rendement : 91 %

Point de fusion : 136 - 138 °C

| Analyse : | | | | | | | | |
|---|---|---|---|---|---|---|---|---|
| Calculée | C | 58,85 | H | 5,70 | N | 5,28 | S | 12,08 |
| Trouvée | C | 58,57 | H | 5,80 | N | 5,29 | S | 11,89 |

**STADE C : OXO - 4 METHOXY - 6 METHYL - 9 TETRAHYDRO - 2,3,4,9 THIOPYRANO [2,3-b] INDOLE**

Agiter un mélange de 24 g (0,09 mole) d'acide (méthyl - 1 méthoxy - 5 indolyl - 2 thio) - 3 propanoïque obtenu au stade précédent et de 120 g de polyphosphate ester dans 80 ml de chloroforme pendant 16 heures, sous atmosphère d'azote et à température ambiante. Verser le mélange réactionnel sur un litre d'eau glacée, décanter la phase organique et extraire à trois reprises la phase aqueuse au chloroforme. Réunir les phases organiques, laver par une solution aqueuse de bicarbonate à 10 % puis à l'eau, sécher et évaporer. Recristalliser le résidu dans l'acétate d'éthyle.

Rendement : 60 %
Point de fusion : 165 - 166 °C

| Analyse : | | | | | | | | |
|---|---|---|---|---|---|---|---|---|
| Calculée | C | 63,14 | H | 5,30 | N | 5,66 | S | 12,96 |
| Trouvée | C | 62,75 | H | 5,48 | N | 5,82 | S | 12,87 |

**STADE D : (DIMETHYLAMLNO) METHYL - 3 OXO - 4 METHOXY - 6 METHYL - 9 TETRAHYDRO - 2,3,4,9 THIOPYRANO [2,3-b] INDOLE (CHLORHYDRATE)**

Chauffer à 100 °C pendant 1 heure sous atmosphère d'azote et forte agitation une suspension de 5,80 g (0,0235 mole) d'oxo - 4 méthoxy - 6 méthyl - 9 tétrahydro - 2,3,4,9 thiopyrano [2,3-b] indole obtenu au stade précédent, de 3,83 g (0,047 mole) de chlorhydrate de diméthylamine et de 1,30 g de paraformaldéhyde dans 70 ml d'acide acétique. Après refroidissement, évaporer le solvant et dissoudre le résidu dans 250 ml d'eau. Laver la phase aqueuse à deux reprises par 50 ml d'acétate d'éthyle et alcaliniser par l'ammoniaque concentrée. Essorer le précipité, laver à l'eau, sécher et dissoudre dans 60 ml de tétrahydrofuranne. Traiter par une solution éthérée d'acide chlorhydrique. Essorer le précipité formé, laver à l'éther éthylique, sécher et recristalliser dans l'éthanol.
Rendement : 35 %
Point de fusion : 190 - 192 °C

| Analyse : | | | | | | | | | | |
|---|---|---|---|---|---|---|---|---|---|---|
| Calculée | C | 56,38 | H | 6,21 | N | 8,22 | S | 9,41 | Cl | 10,40 |
| Trouvée | C | 56,62 | H | 5,98 | N | 8,12 | S | 9,65 | Cl | 10,48 |

**STADE E : [(METHYL - 2 IMIDAZOLYL - 1) METHYL] - 3 OXO - 4 METHOXY - 6 METHYL - 9 TETRAHYDRO - 2,3,4,9 THIOPYRANO [2,3-b] INDOLE (CHLORHYDRATE)**

Chauffer à reflux pendant 16 heures sous atmosphère d'azote une solution de 2,10 g (0,0062 mole) de chlorhydrate de (diméthylamino) méthyl - 3 oxo - 4 méthoxy - 6 méthyl - 9 tétrahydro - 2,3,4,9 thiopyrano [2,3-b] indole et de 1,53 g (0,0186 mole) de méthyl - 2 imidazole dans 30 ml d'eau. Après refroidissement, essorer, laver le précipité à l'eau et sécher. Dissoudre la poudre obtenue dans 20 ml d'acétate d'éthyle et traiter par une solution d'éthanol chlorhydrique. Evaporer et recristalliser dans l'éthanol.
Rendement : 40 %
Point de fusion : 196 - 197 °C

| Analyse : | | | | | | | | | | |
|---|---|---|---|---|---|---|---|---|---|---|
| Calculée | C | 57,21 | H | 5,33 | N | 11,12 | S | 8,48 | Cl | 9,38 |
| Trouvée | C | 56,42 | H | 5,11 | N | 10,72 | S | 8,47 | Cl | 9,28 |

**EXEMPLE 4 : [(METHYL - 2 IMIDAZOLYL - 1) METHYL] - 3 OXO - 4 METHOXY - 5 METHYL - 9 TETRAHYDRO - 2,3,4,9 THIOPYRANO [2,3-b] INDOLE (MALEATE)**

**STADE A : METHYL - 1 METHOXY - 4 INDOLINE THIONE - 2**

En procédant comme dans l'exemple 3, mais en remplaçant la méthoxy - 5 méthyl - 1 indolinone - 2 par la méthoxy - 4 méthyl - 1 indolinone - 2, on obtient le produit attendu.
Rendement : 80 %
Point de fusion : 144 - 145 °C

| Analyse : | | | | | | | | |
|---|---|---|---|---|---|---|---|---|
| Calculée | C | 62,15 | H | 5,74 | N | 7,25 | S | 16,59 |
| Trouvée | C | 61,79 | H | 5,59 | N | 7,10 | S | 16,75 |

**STADE B : ACIDE (METHYL - 1 METHOXY - 4 INDOLYL - 2 THIO) - 3 PROPANOÏOUE**

En procédant comme dans l'exemple 3, stade B, mais en remplaçant la méthyl - 1 méthoxy - 5 indoline thione - 2 par la méthyl - 1 méthoxy - 4 indoline thione 2 obtenue au stade précédent, on obtient le produit attendu.

Rendement : 84 %
Point de fusion : 126 - 128 °C

| Analyse : | | | | | | | | |
|---|---|---|---|---|---|---|---|---|
| Calculée | C | 58,85 | H | 5,70 | N | 5,28 | S | 12,08 |
| Trouvée | C | 58,70 | H | 5,47 | N | 5,28 | S | 12,03 |

**STADE C : OXO - 4 METHOXY - 5 METHYL - 9 TETRAHYRO - 2,3,4,9 THIOPYRANO [2,3-b] INDOLE**

En procédant comme dans l'exemple 3, stade C, mais en remplaçant l'acide (méthyl - 1 méthoxy - 5 indolyl - 2 thio) - 3 propanoïque par l'acide (méthyl - 1 méthoxy - 4 indolyl - 2 thio) - 3 propanoïque obtenu au stade précédent, on obtient le produit attendu.

Rendement : 84 %
Point de fusion : 162 - 163 °C

| Analyse : | | | | | | | | |
|---|---|---|---|---|---|---|---|---|
| Calculée | C | 63,14 | H | 5,30 | N | 5,66 | S | 12,96 |
| Trouvée | C | 63,02 | H | 5,40 | N | 5,61 | S | 12,61 |

**STADE D : (DIMETHYLAMINO) METHYL - 3 OXO - 4 METHOXY - 5 METHYL - 9 TETRAHYDRO - 2,3,4,9 THIOPYRANO [2,3-b] INDOLE (CHLORHYDRATE)**

En procédant comme dans l'exemple 3, stade D, mais en remplaçant l'oxo - 4 méthoxy - 6 méthyl - 9 tétrahydro - 2,3,4,9 thiopyrano [2,3-b] indole par l'oxo - 4 méthoxy - 5 méthyl - 9 tétrahydro - 2,3,4,9 thiopyrano [2,3-b] indole, on obtient le produit attendu.

Rendement : 44 %
Point de fusion : 200 - 202 °C

**STADE E : [(METHYL - 2 [1H] IMIDAZOLYL - 1) METHYL] - 3 OXO - 4 METHOXY - 5 METHYL - 9 TETRAHYDRO - 2,3,4,9 THIOPYRANO [2,3-b] INDOLE (MALEATE)**

En procédant comme dans l'exemple 3, stade E, mais en remplaçant le (diméthylamino) méthyl - 3 oxo - 4 méthoxy - 6 méthyl - 9 tétrahydro - 2,3,4,9 thiopyrano [2,3-b] indole (chlorhydrate) par le (diméthylamino) méthyl - 3 oxo - 4 méthoxy - 5 méthyl - 9 tétrahydro - 2,3,4,9 thiopyrano [2,3-b] indole (chlorhydrate), on obtient le produit attendu, la salification étant effectué ici par une solution alcoolique d'acide maléique et non plus par une solution éthérée d'acide chlorhydrique.

Rendement : 41 %
Point de fusion : 168 - 170 °C

| Analyse : | | | | | | | | |
|---|---|---|---|---|---|---|---|---|
| Calculée | C | 57,76 | H | 5,07 | N | 9,18 | S | 7,01 |
| Trouvée | C | 58,18 | H | 5,02 | N | 8,94 | S | 7,08 |

**EXEMPLE 5 : [(METHYL - 2 IMIDAZOLYL - 1) METHYL] - 3 OXO - 4 METHOXY - 7 METHYL - 9 TETRAHYDRO - 2,3,4,9 THIOPYRANO [2,3-b] INDOLE (CHLORHYDRATE)**

**STADE A : METHYL - 1 METHOXY - 6 INDOLINE THIONE - 2**

En procédant comme dans l'exemple 3, stade A, mais en remplaçant la méthoxy - 5 méthyl - 1 indolinone - 2 par la méthoxy - 6 méthyl - 1 indolinone - 2, on obtient le produit attendu.

Rendement : 76 %

Point de fusion : 135 - 136 °C

| Analyse : | | | | | | | | |
|---|---|---|---|---|---|---|---|---|
| Calculée | C | 62,15 | H | 5,74 | N | 7,25 | S | 16,59 |
| Trouvée | C | 61,99 | H | 5,91 | N | 7,55 | S | 16,31 |

**STADE B : ACIDE (METHYL - 1 METHOXY - 6 INDOLE - 2 THIO) - 3 PROPANOÏOUE**

En procédant comme dans l'exemple 3, stade B, mais en remplaçant la méthyl - 1 méthoxy - 5 indoline thione - 2 par la méthyl - 1 méthoxy - 6 indoline thione - 2 obtenue au stade précédent, on obtient le produit attendu.

Rendement : 72 %

Point de fusion : 120 - 122 °C

| Analyse : | | | | | | | | |
|---|---|---|---|---|---|---|---|---|
| Calculée | C | 58,85 | H | 5,70 | N | 5,28 | S | 12,08 |
| Trouvée | C | 59,01 | H | 5,78 | N | 5,41 | S | 12,06 |

**STADE C : OXO - 4 METHOXY - 7 METHYL - 9 TETRAHYDRO - 2,3,4,9 THIOPYRANO [2,3-b] INDOLE**

En procédant comme dans l'exemple 3, stade C, mais en remplaçant l'acide (méthyl - 1 méthoxy - 5 indolyl - 2 thio) - 3 propanoïque par l'acide (méthyl - 1 méthoxy - 6 indolyl - 2 thio) - 3 propanoïque obtenu au stade précédent, on obtient le produit attendu.

Rendement : 62 %

Point de fusion : 204 - 206 °C

| Analyse : | | | | | | | | |
|---|---|---|---|---|---|---|---|---|
| Calculée | C | 63,14 | H | 5,30 | N | 5,66 | S | 12,96 |
| Trouvée | C | 63,17 | H | 5,56 | N | 5,53 | S | 12,87 |

**STADE D : (DIMETHYLAMINO) METHYL - 3 OXO - 4 METHOXY - 7 METHYL - 9 TETRAHYDRO - 2,3,4,9 THIOPYRANO [2,3-b] INDOLE (CHLOHYDRATE)**

En procédant comme dans l'exemple 3, stade D, mais en remplaçant l'oxo - 4 méthoxy - 6 méthyl - 9 tétrahydro - 2,3,4,9 thiopyrano [2,3-b] indole par l'oxo - 4 méthoxy - 7 méthyl - 9 tétrahydro - 2,3,4,9 thiopyrano [2,3-b] indole obtenu au stade précédent, on obtient le produit attendu.

Rendement : 48 %
Point de fusion : 211 - 212 °C

| Analyse : | | | | | | | | | | |
|---|---|---|---|---|---|---|---|---|---|---|
| Calculée | C | 56,38 | H | 6,21 | N | 8,22 | S | 9,41 | Cl | 10,40 |
| Trouvée | C | 56,71 | H | 5,85 | N | 7,80 | S | 9,75 | Cl | 10,39 |

**STADE E : [(METHYL - 2 IMIDAZOLYL - 1 METHYL] - 3 OXO - 4 METHOXY - 7 METHYL - 9 TETRAHYDRO - 2,3,4,9 THIOPYRANO [2,3-b] INDOLE (CHLORHYDRATE)**

En procédant comme dans l'exemple 3, stade E, mais en remplaçant le (diméthylamino) méthyl - 3 oxo - 4 méthoxy - 6 méthyl - 9 tétrahydro - 2,3,4,9 thiopyrano [2,3-b] indole (chlorhydrate) par le (diméthylamino) méthyl - 3 oxo - 4 méthoxy - 7 méthyl - 9 tétrahydro - 2,3,4,9 thiopyrano [2,3-b] indole (chlorhydrate), on obtient le produit attendu.
Rendement : 32 %
Point de fusion : 154 - 156 °C

| Analyse élémentaire : | | | | | | | | | | |
|---|---|---|---|---|---|---|---|---|---|---|
| Calculée | C | 57,21 | H | 5,33 | N | 11,12 | S | 8,48 | Cl | 9,38 |
| Trouvée | C | 56,67 | H | 5,32 | N | 11,03 | S | 8,53 | Cl | 9,42 |

**EXEMPLE 6 : [(METHYL - 2 IMIDAZOLYL - 1) METHYL] - 3 OXO - 4 METHOXY - 8 METHYL - 9 TETRAHYDRO - 2,3,4,9 THIOPYRANO [2,3-B] INDOLE (MALEATE)**

**STADE A : METHYL - 1 METHOXY - 7 INDOLINE THIONE - 2**

En procédant comme dans l'exemple 3, stade A, mais en remplaçant la méthoxy - 5 méthyl - 1 indolinone - 2 par la méthoxy - 7 méthyl - 1 indolinone - 2, on obtient le produit attendu.
Rendement : 78 %
Point de fusion : 114 - 116 °C

| Analyse : | | | | | | | | |
|---|---|---|---|---|---|---|---|---|
| Calculée | C | 62,15 | H | 5,74 | N | 7,25 | S | 16,59 |
| Trouvée | C | 62,00 | H | 5,78 | N | 7,13 | S | 16,32 |

**STADE B : ACIDE (METHYL - 1 METHOXY - 7 INDOLYL - 2 THIO) - 3 PROPANOÏQUE**

En procédant comme dans l'exemple 3, stade B, mais en remplaçant la méthyl - 1 méthoxy - 5 indoline thione - 2 par la méthyl - 1 méthoxy - 7 indoline thione - 2 obtenue au stade précédent, on obtient le produit attendu.
Rendement : 75 %
Point de fusion : 120 - 122 °C

| Analyse : | | | | | | | | |
|---|---|---|---|---|---|---|---|---|
| Calculée | C | 58,85 | H | 5,70 | N | 5,28 | S | 12,08 |
| Trouvée | C | 59,02 | H | 5,54 | N | 5,28 | S | 11,96 |

**STADE C : OXO - 4 METHOXY - 8 METHYL - 9 TETRAHYDRO - 2,3,4,9 THIOPYRANO [2,3-b] INDOLE**

En procédant comme dans l'exemple 3, stade C, mais en remplaçant l'acide (méthyl - 1 méthoxy - 5 indolyl - 2 thio) - 3 propanoïque par l'acide (méthyl - 1 méthoxy - 7 indolyl - 2 thio) - 3 propanoïque obtenu au stade précédent, on obtient le produit attendu.
Rendement : 71 %
Point de fusion : 106 - 108 °C

| Analyse : | | | | | | | | |
|---|---|---|---|---|---|---|---|---|
| Calculée | C | 63,14 | H | 5,30 | N | 5,66 | S | 12,96 |
| Trouvée | C | 62,93 | H | 5,37 | N | 5,60 | S | 12,97 |

**STADE D : (DIMETHYLAMINO) METHYL - 3 OXO - 4 METHOXY - 8 METHYL - 9 TETRAHYDRO - 2,3,4,9 THIOPYRANO [2,3-b] INDOLE (CHLORHYDRATE)**

En procédant comme dans l'exemple 3, stade D, mais en remplaçant l'oxo - 4 méthoxy - 6 méthyl - 9 tétrahydro - 2,3,4,9 thiopyrano [2,3-b] indole par l'oxo - 4 méthoxy - 8 méthyl - 9 tétrahydro - 2,3,4,9 thiopyrano [2,3-b] indole, on obtient le produit attendu.
Rendement : 53 %
Point de fusion : 190 - 192 °C

**STADE E : [(METHYL - 2 IMIDAZOLYL - 1) METHYL] - 3 OXO - 4 METHOXY - 8 METHYL - 9 TETRAHYDRO - 2,3,4,9 THIOPYRANO [2,3-b] INDOLE (MALEATE)**

En procédant comme dans l'exemple 4, stade E, mais en remplaçant le (diméthylamino) méthyl - 3 oxo - 4 méthoxy - 5 méthyl - 9 tétrahydro - 2,3,4,9 thiopyrano [2,3-b] indole (chlorhydrate) par le (diméthylamino) méthyl - 3 oxo - 4 méthoxy - 8 méthyl - 9 tétrahydro - 2,3,4,9 thiopyrano [2,3-b] indole (chlorhydrate), on obtient le produit attendu.
Rendement : 36 %
Point de fusion : 178 - 180 °C

| Analyse : | | | | | | | | |
|---|---|---|---|---|---|---|---|---|
| Calculée | C | 57,76 | H | 5,07 | N | 9,18 | S | 7,01 |
| Trouvée | C | 57,65 | H | 5,01 | N | 8,72 | S | 6,99 |

**EXEMPLE 7 : [(METHYL - 2 IMIDAZOLYL - 1) METHYL] - 3 OXO - 4 CHLORO - 6 METHYL - 9 TETRAHYDRO - 2,3,4,9 THIOPYRANO [2,3-b] INDOLE (CHLORHYDRATE)**

En procédant comme dans l'exemple 1, mais en remplaçant au stade A la méthyl - 1 indolinone - 2 par la méthyl - 1 chloro - 5 indolinone - 2, on obtient le produit du titre.
Rendement : 42 %
Point de fusion : 252 - 254 °C

| Analyse : | | | | | | | | | | |
|---|---|---|---|---|---|---|---|---|---|---|
| Calculée | C | 53,41 | H | 4,61 | N | 10,99 | S | 8,39 | Cl | 18,55 |
| Trouvée | C | 52,76 | H | 4,36 | N | 11,31 | S | 8,44 | Cl | 18,59 |

**EXEMPLE 8 : [(METHYL - 2 IMIDAZOLYL - 1) METHYL] - 3 OXO - 4 FLUORO - 7 METHYL - 9 TETRAHYDRO - 2,3,4,9 THIOPYRANO [2,3-b] INDOLE (CHLORHYDRATE)**

En procédant comme dans l'exemple 1, mais en remplaçant au stade A la méthyl - 1 indolinone - 2 par la méthyl - 1 fluoro - 6 indolinone - 2, on obtient le produit du titre.

**EXEMPLE 9 : [(METHYL - 2 IMIDAZOLYL - 1) METHYL] - 3 OXO - 4 TRIFLUOROMETHYL - 8 METHYL - 9 TETRAHYDRO - 2,3,4,9 THIOPYRANO [2,3-b] INDOLE (CHLORHYDRATE)**

En procédant comme dans l'exemple 1, mais en remplaçant au stade A la méthyl - 1 indolinone - 2 par la méthyl - 1 trifluorométhyl - 7 indolinone - 2, on obtient le produit du titre.

**EXEMPLE 10 : (PYRROLIDLNYL - 1 METHYL) - 3 OXO - 4 METHYL - 9 TETRAHYDRO - 2,3,4,9 THIOPYRANO [2,3-b] INDOLE (CHLORHYDRATE)**

Chauffer à reflux pendant 10 heures un mélange de 2,50 g (0,008 mole) chlorhydrate de (diméthylamino) méthyl - 3 oxo - 4 méthyl - 9 tétrahydro - 2,3,4,9 thiopyrano [2,3-b] indole obtenu dans l'exemple 1, stade E, et 1,78 g (0,025 mole) de pyrrolidine dans 40 ml d'eau. Après refroidissement, essorer le précipité formé, laver à l'eau, sécher, dissoudre dans 40 ml de tétrahydrofuranne et traiter par une solution éthérée d'acide chlorhydrique. Essorer le précipité formé, laver à l'éther et recristalliser dans l'éthanol.

Rendement : 61 %

Point de fusion : 222 - 224 °C

| Analyse : | | | | | | | | | | |
|---|---|---|---|---|---|---|---|---|---|---|
| Calculée | C | 60,61 | H | 6,28 | N | 8,32 | S | 9,52 | Cl | 10,52 |
| Trouvée | C | 60,74 | H | 6,47 | N | 8,27 | S | 9,57 | Cl | 10,53 |

**EXEMPLE 11 : [(METHYL - 2 IMIDAZOLYL - 1) METHYL] - 3 DIMETHYL - 3,9 OXO - 4 TETRAHYDRO - 2,3,4,9 THIOPYRANO [2,3-b] INDOLE (CHLORHDRATE)**

**STADES A à E : DIMETHYL - 3,9 (DIMETHYLAMINO)METHYL - 3 OXO - 4 - TETRAHYDRO - 2,3,4,9 THIOPYRANO [2,3-b] INDOLE (CHLORHYDRATE)**

En procédant comme dans l'exemple 1, mais en remplaçant au stade B l'acide acrylique par l'acide méthacrylique, on obtient le produit attendu.

Rendement : 78 %

Point de fusion : 220 - 222 °C

| Analyse : | | | | | | | | | | |
|---|---|---|---|---|---|---|---|---|---|---|
| Calculée | C | 59,15 | H | 6,52 | N | 8,62 | S | 9,87 | Cl | 10.91 |
| Trouvée | C | 59,51 | H | 6,59 | N | 8,36 | S | 9,71 | Cl | 10,92 |

**STADE F : [(METHYL - 2 IMIDAZOLYL - 1) METHYL] - 3 DIMETHYL - 3,9 OXO - 4 TETRAHYDRO - 2,3,4,9 THIOPYRANO [2,3-b] INDOLE (CHLORHYDRATE)**

En procédant comme au stade F de l'exemple 1, mais en remplaçant le chlorhydrate de (diméthylamino) méthyl - 3 oxo - 4 méthyl - 9 tétrahydro - 2,3,4,9 thiopyrano [2,3-b] indole par le chlorhydrate de diméthyl - 3,9 (diméthylamino) méthyl - 3 oxo - 4 -tétrahydro - 2,3,4,9 thiopyrano [2,3-b] indole obtenu au stade précédent, on obtient le produit attendu.

**EXEMPLE 12 : (DIMETHYL - 3,5 PIPERAZINYL - 1 METHYL) - 3 OXO - 4 METHYL - 9 TETRAHYDRO - 2,3,4,9 THIOPYRANO [2,3-b] INDOLE (DICHLORHYDRATE)**

En remplaçant dans l'exemple 10, la pyrrolidine par la diméthyl - 2,6 pipérazine, on obtient le produit du titre.

Rendement : 71 %

Point de fusion : 202 - 204 °C

| Analyse : | | | | | | | | | | |
|---|---|---|---|---|---|---|---|---|---|---|
| Calculée | C | 54,80 | H | 6,54 | N | 10,09 | S | 7,70 | Cl | 17,03 |
| Trouvée | C | 54,74 | H | 6,34 | N | 9,92 | S | 8,05 | Cl | 17,18 |

**EXEMPLE 13 : PIPERIDINOMETHYL - 3 OXO - 4 METHYL - 9 TETRAHYDRO - 2,3,4,9 THIOPYRANO [2,3-b] INDOLE (CHLORHDRATE)**

En remplaçant dans l'exemple 10 la pyrrolidine par la pipéridine, on obtient le produit du titre.

Rendement : 58 %

Point de fusion : 211 - 213 °C

| Analyse : | | | | | | | | | | |
|---|---|---|---|---|---|---|---|---|---|---|
| Calculée | C | 61,61 | H | 6,61 | N | 7,98 | S | 9,14 | Cl | 10,10 |
| Trouvée | C | 61,51 | H | 7,02 | N | 7,97 | S | 9,20 | Cl | 10,21 |

**EXEMPLE 14 : MORPHOLINOMETHYL - 3 OXO - 4 METHYL - 9 TETRAHYDRO - 2,3,4,9 THIOPYRANO [2,3-b] INDOLE (CHLORHYDRATE)**

En remplaçant dans l'exemple 10 la pyrrolidine par la morpholine, on obtient le produit attendu.

**EXEMPLE 15 : [(METHOXY - 2 PHENYL) - 4 PIPERAZINYL - 1 METHYL] - 3 OXO - 4 METHYL - 9 TETRAHYDRO - 2,3,4,9 THIOPYRANO [2,3-b] INDOLE (CHLORHYDRATE)**

En remplaçant dans l'exemple 10 la pyrrolidine par la (méthoxy - 2 phényl) - 1 pipérazine, on obtient le produit attendu.

**EXEMPLES 16 A 18 :**

En remplaçant dans les exemples 10, 12 et 13 le chlorhydrate de (diméthylamino) méthyl - 3 oxo - 4 méthyl - 9 tétrahydro - 2,3,4,9 thiopyrano [2,3-b] indole par le chlorhydrate de (diméthylamino) méthyl - 3 oxo - 4 méthoxy - 5 méthyl - 9 oxo - 4 tétrahydro - 2,3,4,9 thiopyrano [2,3-b] indole obtenu dans l'exemple 4, stade D, on obtient :

**EXEMPLE 16 : (PYRROLIDINYL - 1 METHYL) - 3 OXO - 4 METHOXY - 5 METHYL - 9 TETRAHYDRO - 2,3,4,9 THIOPYRANO [2,3-b] INDOLE (CHLORHYDRATE)**

**EXEMPLE 17 : (DIMETHYL - 3,5 PIPERAZINYL - 1 METHYL) - 3 OXO - 4 METHOXY - 5 METHYL - 9 TETRAHYDRO - 2,3,4,9 THIOPYRANO [2,3-b] INDOLE (CHLORHYDRATE)**

**EXEMPLE 18 : PIPERIDINO METHYL - 3 OXO - 4 METHOXY - 5 METHYL - 9 TETRAHYDRO - 2,3,4,9 THIOPYRANO [2,3-b] INDOLE (CHLORHYDRATE)**

**EXEMPLE 19 : [(METHYL - 2 IMIDAZOLYL - 1) METHYL] - 3 OXO - 4 METHYL - 9 TETRAHYDRO - 2,3,4,9 THIOPYRANO [2,3-b] INDOLE, S-OXYDE (CHLORHYDRATE)**

Additionner lentement une solution de 1,38 g (0,008 mole) d'acide méta chloroperbenzoïque dans 20 ml de chloroforme à une solution refroidie à 0 °C de 1,56 g (0,005 mole) de [(méthyl - 2 imidazolyl - 1) méthyl] - 3 oxo - 4 méthyl - 9 tétrahydro - 2,3,4,9 thiopyrano [2,3-b] indole (obtenu dans l'exemple 1) dans 50 ml de chloroforme. Agiter à 5 °C pendant une heure puis à température ambiante pendant une heure. Après filtration, laver la phase organique par la soude à 5 % puis par l'eau, sécher et évaporer. Purifier le produit par chromatographie sur silice en éluant avec un mélange chlorure de méthylène/méthanol = 95 : 5. Dissoudre le produit obtenu dans 80 ml de tétrahydrofuranne et traiter par 2 ml d'une solution éthérée d'acide chlorhydrique. Essorer le précipité, laver à l'éther sécher et recristalliser dans l'éthanol.

Rendement : 30 %
Point de fusion : 175 - 178 °C

| Analyse : | | | | | | | | | | | | |
|---|---|---|---|---|---|---|---|---|---|---|---|---|
| Calculée | C | 56,12 | H | 4,99 | N | 11,55 | S | 8,81 | Cl | 9,74 |
| Trouvée | C | 56,23 | H | 5,21 | N | 11,46 | S | 8,77 | Cl | 9,86 |

**EXEMPLE 20 : [(METHYL - 2 IMIDAZOLYL - 1) METHYL] - 4 OXO - 5 METHYL - 10 PENTAHYDRO - 2,3,4,5,10 THIEPINO [2,3-b] INDOLE ainsi que le composé libre et d'autres sels pharmaceutiquement acceptables de ce dernier.**

**STADE A : (METHYL - 1 INDOLYL - 2 THIO) - 4 BUTANOATE D'ETHYLE**

Agiter à 20 °C pendant 5 heures une suspension composée de 11,43 g (0,07 mole) de méthyl - 1 indoline thione - 2 obtenue dans l'exemple 1,stade A, et de 13,70 g (0,07 mole) de bromo - 4 butanoate d'éthyle dans 30 ml d'acétone en présence de 11,75 g (0,096 mole) de carbonate de potassium finement broyé. Filtrer et évaporer le solvant, l'huile obtenue est utilisée directement dans le stade suivant.

**STADE B : ACIDE (METHYL - 1 INDOLYL - 2 THIO) - 4 BUTANOÏQUE**

Additionner 70 ml de soude normale à une solution de 18,30 g de (méthyl - 1 indolyl - 2 thio) - 4 butanoate d'éthyle, obtenu au stade précédent, dans 250 ml d'éthanol. Après une heure de chauffage à reflux, évaporer le milieu réactionnel et reprendre le résidu par l'eau. Laver la phase aqueuse à l'acétate d'éthyle et acidifier par une solution aqueuse d'acide chlorhydrique. Essorer le précipité, laver à l'eau, sécher et recristalliser dans l'éthanol.

Rendement : 80 %
Point de fusion : 133 - 135 °C

| Analyse : | | | | | | | | |
|---|---|---|---|---|---|---|---|---|
| Calculée | C | 62,62 | H | 6,06 | N | 5,62 | S | 12,86 |
| Trouvée | C | 61,95 | H | 6,10 | N | 5,31 | S | 12,57 |

**STADE C : OXO - 5 METHYL - 10 PENTAHYDRO - 2,3,4,5,10, THIEPINNO [2,3-b] INDOLE**

Agiter à température ambiante pendant 16 heures et sous atmosphère d'azote un mélange de 15 g (0,060 mole) d'acide (méthyl - 1 indolyl - 2 thio) - 4 butanoïque obtenu au stade précédent et de 80 g de polyphosphate ester dans 600 ml de chloroforme. Après dilution par 1,3 l d'eau glacée, décanter la phase organique et extraire la phase aqueuse au chloroforme. Réunir les phases organiques, laver par une solution aqueuse de bicarbonate de sodium à 10 % puis à l'eau, sécher et concentrer sous vide. Recristalliser le résidu dans l'acétonitrile.

Rendement : 92 %

Point de fusion : 165 - 166 °C

| Analyse : | | | | | | | | |
|---|---|---|---|---|---|---|---|---|
| Calculée | C | 67,50 | H | 5,66 | N | 6,06 | S | 13,86 |
| Trouvée | C | 68,23 | H | 5,89 | N | 6,52 | S | 13,82 |

**STADE D : (DIMETHYLAMINO) METHYL - 4 OXO - 5 METHYL - 10 PENTAHYDRO - 2,3,4,5,10 THIEPINNO [2,3-b] INDOLE (CHLORHYDRATE)**

Agiter à 100 °C pendant 2 heures 30 sous atmosphère d'azote une suspension de 12,6 g (0,055 mole) d'oxo - 5 méthyl - 10 pentahydro - 2,3,4,5,10 thiépinno [2,3-b] indole, de 9,10 g (0,11 mole) de chlorhydrate de diméthylamine et de 3,50 g de paraformaldéhyde dans 200 ml d'acide acétique. Refroidir, évaporer le solvant et dissoudre le résidu dans un mélange eau/acide acétique = 80 : 20. Laver la phase aqueuse par l'acétate d'éthyle et alcaliniser par l'ammoniaque concentrée. Extraire le précipité huileux au chlorure de méthylène et laver la phase organique par une solution aqueuse saturée de chlorure de sodium et évaporer. Dissoudre le résidu dans 80 ml de tétrahydrofuranne et traiter par 15 ml d'une solution éthérée d'acide chlorhydrique à 0 °C pendant 3 heures. Essorer le précipité, laver à l'éther éthylique, sécher et recristalliser dans un mélange éthanol-éther.

Rendement : 54 %

Point de fusion : 232 - 233 °C

| Analyse : | | | | | | | | | | |
|---|---|---|---|---|---|---|---|---|---|---|
| Calculée | C | 59,15 | H | 6,52 | N | 8,62 | S | 9,87 | Cl | 10,91 |
| Trouvée | C | 58,83 | H | 6,44 | N | 8,32 | S | 9,69 | Cl | 10,70 |

**STADE E : [(METHYL - 2 IMIDAZOLYL - 1) METHYL] - 4 OXO - 5 METHYL - 10 PENTAHYDRO - 2,3,4,5,10 THIEPINNO [2,3-b] INDOLE (CHLORHYDRATE)**

Chauffer à 100 °C pendant 20 heures une solution de 2,46 g (0,03 mole) de méthyl - 2 imidazole et de 3,25 g (0,01 mole) de chlorhydrate de (diméthylamino) méthyl - 4 oxo - 5 méthyl - 10 pentahydro - 2,3,4,5,10 thiépinno [2,3-b] indole dans 40 ml d'eau. Après refroidissement le résidu solide est essoré, lavé à l'eau et séché sous vide. Dissoudre le résidu obtenu dans 80 ml d'éthanol chaud et traiter par 4 ml d'une solution éthérée d'acide chlorhydrique pendant 30 minutes à 40 °C. Evaporer le solvant et cristalliser le résidu dans l'éthanol.

Rendement : 75 %

Point de fusion : 222 - 224 °C

| Analyse : | | | | | | | | | | |
|---|---|---|---|---|---|---|---|---|---|---|
| Calculée | C | 59,74 | H | 5,57 | N | 11,61 | S | 8,86 | Cl | 9,80 |
| Trouvée | C | 59,67 | H | 5,65 | N | 11,37 | S | 8,99 | Cl | 9,64 |

**EXEMPLE 21 : [(METHYL - 2 IMIDAZOLYL - 1) METHYL] - 3 OXO - 4 METHYLSULFONYL - 9 TETRAHYDRO - 2,3,4,9 THIOPYRANO [2,3-b] INDOLE (CHLORHYDRATE)**

**STADE A : ACIDE (INDOLYL - 2 THIO) - 3 PROPIONIOUE**

Additioner lentement en 2 heures 30, un litre de triéthylamine à une suspension fortement agitée de 194 g (1,3 mole) d'indoline thione - 2 (préparée selon Y. Nakisumi et Coll. Chem. Pharm. Bull. 1984, 32, (3), 877) dans 108 g (1,5 mole) d'acide acrylique. Porter à reflux pendant 12 heures en maintenant l'agitation puis refroidir et concentrer sous vide. Reprendre le résidu par une solution aqueuse de bicarbonate de sodium à 10% et filtrer. Acidifier par une solution aqueuse d'acide chlorhydrique. Essorer le précipité formé, laver à l'eau et sécher. Recristalliser dans le toluène.

Rendement : 75 %
Point de fusion : 116 - 118 °C

| Analyse : | | | | | | | | |
|---|---|---|---|---|---|---|---|---|
| Calculée | C | 59,71 | H | 5,01 | N | 6,33 | S | 14,49 |
| Trouvée | C | 59,72 | H | 4,89 | N | 6,19 | S | 14,16 |

**STADE B : OXO - 4 TETRAHYDRO - 2,3,4,9 THIOPYRANO [2,3-b] INDOLE**

Agiter pendant 16 heures à température ambiante et sous atmosphère d'azote un mélange de 88,5 g (0,4 mole) d'acide (indolyl - 2 thio) - 3 propionique et de 300 g de polyphosphate ester dans 3,5 litres de chloroforme. Diluer par 3 litres d'eau glacée, décanter la phase organique et extraire la phase aqueuse au chloroforme. Réunir les phases organiques, laver par une solution aqueuse de bicarbonate de sodium à 10 % puis à l'eau, sécher et concentrer au bain-marie sous vide. Recristalliser dans l'éthanol, éliminer les cristaux obtenus, et concentrer le filtrat.

Rendement : 35 %
Point de fusion : 226 - 228 °C

| Analyse : | | | | | | | | |
|---|---|---|---|---|---|---|---|---|
| Calculée | C | 65,00 | H | 4,46 | N | 6,89 | S | 15,77 |
| Trouvée | C | 64,71 | H | 4,60 | N | 6,84 | S | 15,74 |

**STADE C : (DIMETHYLAMINO) METHYL - 3 OXO - 4 TETRAHYDRO - 2,3,4,9 THIOPYRANO [2,3-b] INDOLE (CHLORHYDRATE)**

Agiter fortement à 100 °C sous atmosphère d'azote et durant 45 minutes un mélange de 4,10 g (0,02 mole) d'oxo - 4 tétrahydro - 2,3,4,9 thiopyrano [2,3-b] indole, de 3,26 g (0,04 mole) de chlorhydrate de diméthylamine et de 1 g de paraformaldéhyde dans 50 ml d'acide acétique. Après refroidissement, évaporer le solvant, dissoudre le résidu dans 80 ml d'eau, laver la phase aqueuse à l'acétate d'éthyle et alcaliniser à' l'ammoniaque concentrée. Extraire le précipité au chlorure de méthylène, laver la solution par de l'eau saturée en chlorure de sodium, puis concentrer. Dissoudre le résidu dans 50 ml d'éthanol chaud et traiter à 40 °C par 7 ml d'une solution éthanolique d'acide chlorhydrique. Après dilution par l'éther éthylique, essorer le précipité, laver à l'éther éthylique, sécher, recristalliser dans l'éthanol.

Rendement : 76 %
Point de fusion : 235 - 237 °C

| Analyse : | | | | | | | | | | |
|---|---|---|---|---|---|---|---|---|---|---|
| Calculée | C | 56,65 | H | 5,77 | N | 9,44 | S | 10,80 | Cl | 11,94 |
| Trouvée | C | 56,76 | H | 6,02 | N | 9,43 | S | 10,55 | Cl | 11,64 |

**STADE D : [(METHYL - 2 IMIDAZOLYL - 1) METHYL] - 3 OXO - 4 TETRAHYDRO - 2,3,4,9 THIOPYRANO [2,3-b] INDOLE**

Ajouter 5,60 ml (0,09 mole) d'iodométhane à une solution de 23,43 g (0,09 mole) de (diméthylamino) méthyl - 3 oxo - 4 tétrahydro - 2,3,4,9 thiopyrano [2,3-b] indole (chlorhydrate) dans 250 ml de diméthylformamide. Agiter le mélange à température ambiante pendant une heure puis ajouter une solution de 22,17 g (0,27 mole) de méthyl - 2 imidazole dans 50 ml de diméthyl formamide, chauffer ensuite le milieu réactionnel à 100 °C pendant 36 heures. Après refroidissement, ajouter 500 ml d'une solution aqueuse de carbonate de sodium 2N et extraire à 3 reprises à l'acétate d'éthyle. Réunir les phases organiques, laver à l'eau (200 ml), sécher et concentrer sous vide. Chromatographier sur silice en éluant avec un mélange chlorure de méthylène/éthanol/ammoniaque : 92/8/0,5. Recristalliser le résidu dans l'acétonitrile.

Rendement : 45 %

Point de fusion : 231 - 233 °C

**STADE E : [(METHYL - 2 IMIDAZOLYL - 1) METHYL] - 3 OXO - 4 TETRAHYDRO - 2,3,4,9 THIOPYRANO [2,3-b] INDOLE (CHLORHYDRATE)**

Dissoudre 1 g du produit obtenu au stade précédent dans 20 ml d'éthanol à 50 °C et traiter par 2 ml d'une solution éthanolique d'acide chlorhydrique (5,2 N) pendant 30 minutes. Evaporer le solvant, recristalliser le résidu dans l'éthanol.

Point de fusion : 228 - 230 °C

| Analyse : | | | | | | | | | | |
|---|---|---|---|---|---|---|---|---|---|---|
| Calculée | C | 57,57 | H | 4,83 | N | 12,59 | S | 9,60 | Cl | 10,62 |
| Trouvée | C | 57,49 | H | 5,25 | N | 12,10 | S | 9,47 | Cl | 10,44 |

**STADE F : [(METHYL - 2 IMIDAZOLYL - 1) METHYL] - 3 OXO - 4 METHYLSULFONYL - 9 TETRAHYDRO - 2,3,4,9 THIOPYRANO [2 ,3-b] INDOLE (CHLORHYDRATE)**

A une solution de 1,19 g (0,004 mole) de [méthyl - 2 imidazolyl - 1) méthyl] - 3 oxo - 4 tétrahydro - 2,3,4,9 thiopyrano [2,3-b] indole dans 20 ml de diméthylformamide ajouter 0,20 g (0,0042 mole) d'hydrure de sodium à 50 % dans l'huile, en 30 minutes, sous atmosphère d'azote puis 0,325 ml (0,0042 mole) de chlorure de méthane sulfonyle. Agiter 4 heures à température ambiante, ajouter 150 ml d'une solution 2N de carbonate de sodium et extraire à trois reprises à l'acétate d'éthyle. Réunir les phases organiques, sécher et évaporer. Chromatographier le résidu (1,35 g) sur gel de silice en éluant avec un mélange chlorure de méthylène/éthanol:92/8. Dissoudre le résidu obtenu dans 20 ml d'éthanol, traiter par 3 ml d'une solution éthérée d'acide chlorhydrique à 10 °C pendant 1 heure. Essorer le précipité, laver à l'éther éthylique et recristalliser dans l'acétonitrile.

Rendement : 30 %

Point de fusion : 228 - 230 °C

| Analyse : | | | | | | | | | | |
|---|---|---|---|---|---|---|---|---|---|---|
| Calculée | C | 49,57 | H | 4,40 | N | 10,20 | S | 15,57 | Cl | 8,61 |
| Trouvée | C | 50,48 | H | 4,76 | N | 10,50 | S | 15,26 | Cl | 8,55 |

**EXEMPLE 22 : [(METHYL - 2 IMIDAZOLYL - 1) METHYL] - 3 OXO - 4 PARATOLYLSULFONYL - 9 TETRAHYDRO - 2,3,4,9 THIOPYRANO [2,3-b] INDOLE (CHLORHYDRATE)**

En procédant comme dans l'exemple 21, mais en remplaçant dans le stade F le chlorure de méthane sulfonyle par le chlorure de paratolyl sulfonyle, on obtient le produit attendu.

**EXEMPLE 23 : CHLORHYDRATE DE (TRIMETHYL - 1,1,2 GUANIDINO METHYL) - 3 OXO - 4 METHYL - 9 TETRAHYDRO - 2,3,4,9 THIOPYRANO [2,3-b] INDOLE**

**STADE A : CHLORHYDRATE DE [(DIHYDRO - 10,11 [5H] DIBENZO [a,d] CYCLOHEPTENYL - 5) AMINO METHYL] - 3 OXO - 4 METHYL - 9 TETRAHYDRO - 2,3,4,9 THIOPYRANO [2,3-b] INDOLE**

Agiter fortement à une température voisine de 90 °C sous atmosphère d'azote et pendant 20 heures, un mélange de 22 g de chlorhydrate de (diméthylamino) méthyl - 3 oxo - 4 méthyl - 9 tétrahydro - 2,3,4,9 thiopyrano [2,3-b] indole obtenu dans l'exemple 1, stade E et de 29,62 g (0,14 mole) de (dihydro - 10,11 [5H] dibenzo [a,d] cycloheptenyl - 5) amine dans 300 ml d'eau. Après refroidissement, essorer, sécher le résidu obtenu et chromatographier sur silice en éluant par un mélange chlorure de méthylène/méthanol:97/3. Dissoudre le solide obtenu dans 150 ml d'éthanol et traiter par une solution d'éther chlorhydrique. Essorer le précipité formé, laver à l'éther éthylique et recristalliser dans l'éthanol.

Rendement : 82 %

Point de fusion : 217 - 218 °C

| Analyse : | | | | | | | | | | |
|---|---|---|---|---|---|---|---|---|---|---|
| Calculée | C | 70,79 | H | 5,73 | N | 5,90 | S | 6,75 | Cl | 7,46 |
| Trouvée | C | 70,73 | H | 5,51 | N | 6,00 | S | 6,81 | Cl | 7,45 |

**STADE B : CHLORHYDRATE D'AMINOMETHYL - 3 OXO - 4 METHYL - 9 TETRAHYDRO - 2,3,4,9 THIOPYRANO [2,3-b] INDOLE**

Chauffer à reflux pendant 6 heures une solution de 27,10 g (0,057 mole) de chlorhydrate de [(dihydro - 10,11 [5H] dibenzo [a,d] cycloheptenyl - 5) aminométhyl] - 3 oxo - 4 tétrahydro - 2,3,4,9 thiopyrano [2,3-b] indole obtenu au stade précédent dans 600 ml d'acide acétique aqueux à 60%. Après refroidissement et filtration, évaporer le filtrat sous vide, reprendre le résidu dans 200 ml d'éthanol et traiter par 30 ml d'une solution d'éther chlorhydrique. Diluer à l'éther, essorer, laver à l'éther et recristalliser dans le méthanol.

Rendement : 91 %

Point de fusion : 255 - 257 °C

| Analyse : | | | | | | | | | | |
|---|---|---|---|---|---|---|---|---|---|---|
| Calculée | C | 55,22 | H | 5,35 | N | 9,91 | S | 11,34 | Cl | 12,54 |
| Trouvée | C | 55,45 | H | 5,41 | N | 9,94 | S | 11,48 | Cl | 12,47 |

**STADE C : CHLORHYDRATE DE (TRIMETHYL - 1,1,2 GUANIDINOMETHYL) - 3 OXO - 4 METHYL - 9 TETRAHYDRO - 2,3,4,9 THIOPYRANO [2,3-b] INDOLE**

Dissoudre le chlorhydrate (d'amino méthyl) - 3 oxo - 4 méthyl - 9 tétrahydro - 2,3,4,9 thiopyrano [2,3-b] indole obtenu au stade précédent dans l'eau, neutraliser par du bicarbonate de potassium, extraire au chlorure de méthylène, sécher et évaporer. Dissoudre 4,90 g (0,02 mole) du produit ainsi obtenu dans 80 ml de pyridine. Ajouter 5,20 g (0,02 mole) d'iodhydrate de tétraméthyl N,N,N′,S isothiouronium et chauffer cette solution à 90 °C pendant 2 heures 30. Evaporer le solvant et chromatographier le résidu huileux sur silice en éluant avec un mélange chloroforme/méthanol : 92/8. Dissoudre le produit obtenu dans un mélange de 70 ml d'éthanol et de de 20 ml d'eau et traiter par 3 g de carbonate d'argent sous vive agitation pendant 1 heure. Filtrer sur célite, évaporer, reprendre le résidu dans 30 ml d'éthanol et traiter par une solution d'éther chlorhydrique. Diluer à l'éther, essorer, laver le précipité à l'éther et recristalliser dans l'acétonitrile.

Rendement : 42 %

Point de fusion : 204 - 206 °C

| Analyse : | | | | | | | | | | |
|---|---|---|---|---|---|---|---|---|---|---|
| Calculée | C | 55,65 | H | 6,32 | N | 15,27 | S | 8,74 | Cl | 9,66 |
| Trouvée | C | 55,80 | H | 6,44 | N | 15,20 | S | 8,82 | Cl | 9,58 |

**EXEMPLE 24 : CHLORHYDRATE DE [(DIHYDRO - 4,5 IMIDAZOLYL - 2) AMINOMETHYL] - 3 OXO - 4 METHYL - 9 TETRAHYDRO - 2,3,4,9 THIOPYRANO [2,3-b] INDOLE**

En procédant comme dans l'exemple 23, mais en remplaçant au stade C L'iodhydrate de tétraméthyl N,N,N',S isothiouronium par l'iodhydrate de méthyl mercapto - 2 dihydro - 4,5 imidazole, on obtient le produit attendu.

Rendement : 66 %

Point de fusion : 245 - 247 °C

| Analyse : | | | | | | | | | | |
|---|---|---|---|---|---|---|---|---|---|---|
| Calculée | C | 54,77 | H | 5,46 | N | 15,97 | S | 9,14 | Cl | 10,10 |
| Trouvée | C | 54,46 | H | 5,55 | N | 15,93 | S | 9,17 | Cl | 9,99 |

**EXEMPLE 25 : [(METHYL - 2 IMIDAZOLYL - 1) METHYL] - 3 PHENYL - 2 OXO - 4 METHYL - 9 TETRAHYDRO - 2,3,4,9 THIOPYRANO [2,3-b] INDOLE (CHLORHYDRATE)**

En procédant comme dans l'exemple 1, mais en remplaçant l'acide acrylique au stade B par l'acide cinnamique, on obtient le produit du titre.

Rendement : 65 %

Point de fusion : 195 - 197 °C

| Analyse : | | | | | | | | | | |
|---|---|---|---|---|---|---|---|---|---|---|
| Calculée | C | 65,16 | H | 5,23 | N | 9,91 | S | 7,56 | Cl | 8,36 |
| Trouvée | C | 64,80 | H | 5,45 | N | 9,65 | S | 7,45 | Cl | 8,39 |

**EXEMPLE 26 : [(METHYL - 2 IMIDAZOLYL - 1) METHYL] - 3 (METHYL - 4 PHENYL) - 2 OXO - 4 METHYL - 9 TETRAHYDRO - 2,3,4,9 THIOPYRANO [2,3-b] INDOLE (CHLORHYDRATE)**

En procédant comme dans l'exemple 1, mais en remplaçant l'acide acrylique au stade B par l'acide méthyl - 4 cinnamique, on obtient le produit du titre.

**EXEMPLE 27 : [(PHENYL - 2 IMIDAZOLYL - 1) METHYL] - 3 OXO - 4 METHYL - 9 TETRAHYDRO - 2,3,4,9 THIOPYRANO [2,3-b] INDOLE (CHLORHYDRATE)**

En procédant comme dans l'exemple 1, mais en remplaçant au stade F le méthyl - 2 imidazole par le phényl - 2 imidazole, on obtient le produit du titre.

**EXEMPLE 28 : (BENZIMIDAZOLYL - 1 METHYL) - 3 OXO - 4 METHYL - 9 TETRAHYDRO - 2,3,4,9 THIOPYRANO [2,3-b] INDOLE (CHLORHYDRATE)**

En procédant comme dans l'exemple 1, mais en remplaçant au stade F le méthyl - 2 imidazole par le benzimidazole, on obtient le produit du titre.

**EXEMPLE 29 : (THIOMORPHOLINOMETHYL) - 3 OXO - 4 METHYL - 9 TETRAHYDRO - 2,3,4,9 THIOPYRANO [2,3-b] INDOLE (CHLORHYDRATE)**

En procédant comme dans l'exemple 14, mais en remplaçant la morpholine par la thiomorpholine, on obtient le produit du titre.

**EXEMPLE 30 : ETUDE PHARMACOLOGIOUE DES COMPOSES DE L'INVENTION : INHIBITION DE L'EFFET BEZOLD-JARISCH**

Cette étude est réalisée sur des rats Charles River d'un poids compris entre 350 et 400 g anesthésiés par une dose de 1,25 $g.kg^{-1}$ d'uréthanne administré intrapéritonéalement. La pression artérielle est enregistrée au niveau d'une artère carotide au moyen d'une cellule de pression Statham P 23 dB. La fréquence cardiaque est enregistrée à partir de la pression artérielle en utilisant un cardiotachymètre. Les enregistrements sont réalisés sur un enregistreur GOULD 2400. Un cathéter est placé dans la veine jugulaire et atteint l'oreillette droite de l'animal. L'effet Bezold-Jarisch est provoqué par une injection rapide de sérotonine à la dose de 40 µg/kg par voie intraveineuse dans l'oreillette droite. Le ralentissement cardiaque est associé à une chute de la pression artérielle qui provient de la stimulation des fibres afférentes vagales principalement localisées dans le ventricule droit.

Les produits à tester sont administrés par voie intraveineuse aux doses de 10 µg/kg ou 50 µg/kg. Un effet Bezold-Jarisch est provoqué à l'un ou plusieurs des temps suivants : 5, 15, 30, 45 et 60 minutes après injection des produits testés.

Le pourcentage d'inhibition de la bradycardie réflexe est évalué à partir du tracé de fréquence cardiaque.

Les composés de l'invention ont montré un puissant effet inhibiteur de l'effet Bezold-Jarisch.

Ainsi, administrés à une dose de 50 µg/kg, les composés des exemples 1 et 20 provoquent cinq minutes après leur administration un antagonisme de 95% dont l'effet se prolonge dans le temps. En effet, cet antagonisme demeure voisin de 95%, quinze minutes après l'administration de 50 µg/kg de l'un ou de l'autre de ces deux composés. Une heure après administration de 50 µg/kg du composé de l'exemple 20, on observe encore un antagonisme de l'ordre de 60%.

A titre de comparaison, le composé de référence GR 38032 administré à une dose identique provoque un antagonisme de 90% cinq minutes après son administration. Ce taux n'est plus que de 55%, 15 minutes après l'administration et chute à 25%, une heure après l'administration.

Ces résultats indiquent de façon flagrante la supériorité des composés de l'invention.

**EXEMPLE 31 : COMPOSITION PHARMACEUTIOUE**

Comprimé dosé à 2,5 mg de chlorhydrate de [méthyl - 2 imidazolyl - 1) méthyl] - 3 oxo - 4 méthyl - 9 tétrahydro - 2,3,4,9 thiopyrano [2,3-b] indole

| Formule de préparation pour 1000 comprimés. | |
|---|---|
| (méthyl - 2 imidazolyl - 1) méthyl - 3 oxo - 4 méthyl - 9 tétrahydro - 2,3,4,9 thiopyrano [2,3-b] indole (chlorhydrate) | 2,5 g |
| Hydroxypropylcellulose | 1,5 g |
| Amidon de blé | 12 g |
| Lactose | 120 g |
| Stéarate de magnésium | 2 g |
| Talc | 2 g |

## Revendications

**Revendications pour les Etats contractants suivants : AT, BE, CH, DE, FR, GB, IT, LI, LU, NL, SE**

**1.** Dérivés de formule (I) :

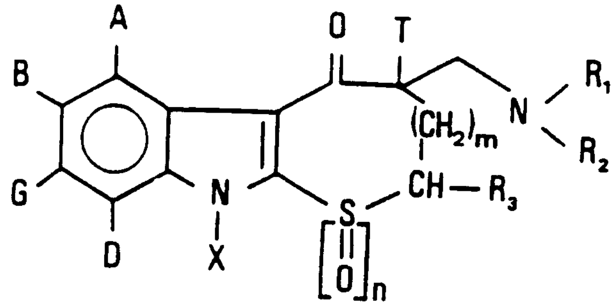

(I)

dans laquelle :

A, B, G, D identiques ou différents représentent chacun un atome d'hydrogène ou un atome d'halogène, un groupement alkoxy inférieur ou encore un groupement alkyle inférieur linéaire ou ramifié éventuellement substitué par un ou plusieurs atomes d'halogène,

X représente un atome d'hydrogène, un groupement alkyle inférieur linéaire ou ramifié ou un groupement $SO_2E$ dans laquelle E représente un groupement alkyle inférieur linéaire ou ramifié ou phényle éventuellement substitué par un groupement alkyle inférieur linéaire ou ramifié,

T représente un atome d'hydrogène ou un groupement alkyle inférieur,

$R_3$ représente un atome d'hydrogène ou un groupement alkyle inférieur linéaire ou ramifié ou un groupement phényle éventuellement substitué par un ou plusieurs groupements alkyle inférieurs linéaires ou ramifiés,

n et m identiques ou différents représentent chacun 0 ou 1,

$R_1$ et $R_2$ identiques ou différents représentent chacun un atome d'hydrogène ou un groupement alkyle inférieur linéaire ou ramifié,

ou bien,

$R_1$ et $R_2$ forment ensemble avec l'atome d'azote auxquels ils sont liés un système hétérocyclique azoté, mono ou bicyclique, saturé ou non, chaque cycle comprenant cinq ou six sommets et comprenant éventuellement un ou deux autres hétéroatomes choisis parmi azote, oxygène ou soufre, et étant éventuellement substitué par un ou plusieurs groupements alkyle ou alkoxy inférieurs, linéaires ou ramifiés ou par un groupement aryle éventuellement lui-même substitué par un ou plusieurs groupements alkyle inférieurs, alkoxy inférieurs, trifluorométhyle ou bien encore un ou plusieurs atome d'halogène,

ou bien,

$R_1$ représente un groupement

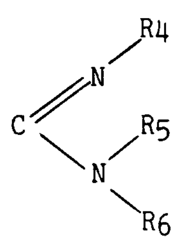

dans laquelle $R_4$, $R_5$, $R_6$ identiques ou différents représentent chacun un atome d'hydrogène ou un groupement alkyle inférieur linéaire ou ramifié ou bien $R_4$ forme avec $R_5$ un pont $(CH_2)p$, p étant compris entre 2 et 4,

ou bien encore $R_1$ représente un ensemble dihydro - 10,11 [5H] dibenzo [a,d] cycloheptenyl - 5 et $R_2$

représente un atome d'hydrogène,
le cas échéant, leurs isomères ainsi que leurs sels d'addition à un acide pharmaceutiquement acceptable,
étant entendu que par groupement alkyle inférieur ou alkoxy inférieur, on entend des groupements comprenant de 1 à 6 atomes de carbone.

**2.** Composés selon la revendication 1 pour lesquelles n représente 0, X représente un groupement alkyle inférieur linéaire ou ramifié et $R_1$ et $R_2$ forment ensemble avec l'atome d'azote auxquel ils sont attachés un noyau imidazole éventuellement substitué par un ou plusieurs groupements alkyle ou alkoxy inférieurs linéaires ou ramifiés, ou par un groupement aryle éventuellement lui-même substitué par un groupement alkyle inférieur ou alkoxy inférieur trifluorométhyle ou bien encore un atome d'halogène, leurs isomères ainsi que leurs sels d'addtion à un acide pharmaceutiquement acceptable.

**3.** Composés selon l'une des revendications 1 ou 2 pour lesquels $R_1$ et $R_2$ forment ensemble avec l'atome d'azote auquel ils sont attachés un groupement méthyl - 2 imidazole.

**4.** Composé selon l'une des revendications 1 ou 3 qui est le [(méthyl - 2 imidazolyl - 1) méthyl] - 3 oxo - 4 méthyl - 9 tétrahydro - 2,3,4,9 thiopyrano [2,3-b] indole ainsi que ses sels d'addition à un acide pharmaceutiquement acceptable.

**5.** Composé selon l'une des revendications 1 ou 3 qui est le [(méthyl - 2 imidazolyl - 1) méthyl] - 4 oxo - 5 méthyl - 10 pentahydro - 2,3,4,5,10 thiépinno [2,3-b] indole ainsi que ses sels d'addition à un acide pharmaceutiquement acceptable.

**6.** Procédé de synthèse des dérivés de formule (I) caractérisé en ce que l'on condense un dérivé de formule (II) :

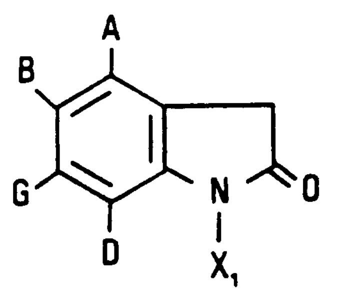

(II)

dans laquelle A, B, G, D ont la même signification que dans la formule (I),
et $X_1$ représente un atome d'hydrogène ou un groupement alkyle inférieur,
que l'on traite en présence d'un agent de thionation tel que le pentasulfure de phosphore pour obtenir un dérivé de formule (III) :

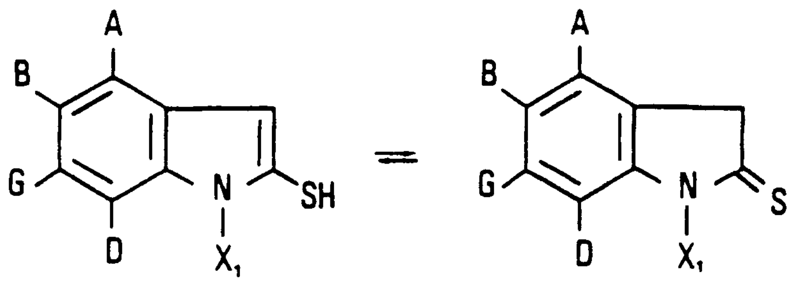

(III)

dans laquelle A, B, G, D et $X_1$ ont la même signification que précédemment,
que l'on traite par un dérivé de formule (IV) :

$JCHR_3-(CH_2)_m-CTK-COOR$ (IV)

dans laquelle :
ou bien
m représente 0 et dans ce cas J et K représentent ensemble une liaison $\pi$,
ou bien
m représente 1 et dans ce cas J représente un atome d'halogène, et K représente un atome d'hydrogène,
R représente un atome d'hydrogène ou un groupement alkyle inférieur linéaire ou ramifié et T et $R_3$ ont la même signification que dans la formule (I),
pour obtenir un dérivé de formule (V) :

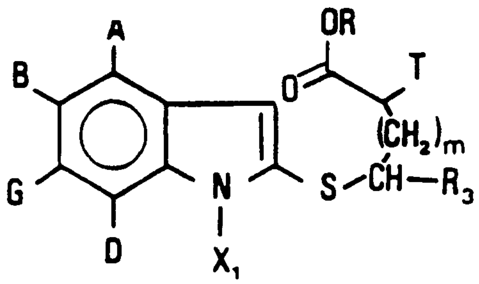

(V)

dans laquelle A, B, G, D, T, R, $R_3$, $X_1$ et m ont la même signification que précédemment,
qui, lorsque R est différent de H est soumis à l'action d'un agent alcalin pour conduire à un dérivé de formule (Va), cas particulier des dérivés de formule (V) pour lesquels R représente H,

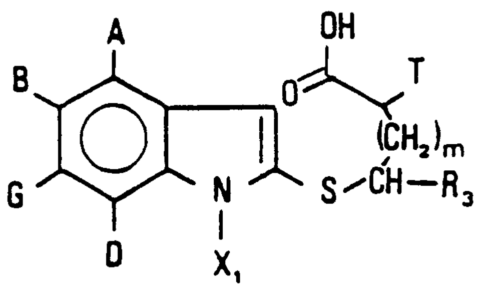

(Va)

que l'on soumet à l'action de polyphosphate ester préparé préférentiellement sous atmosphère d'azote pour conduire à un dérivé de formule (VI) :

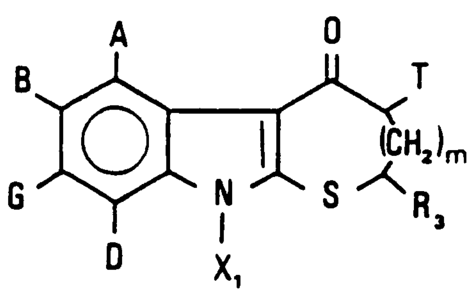

(VI)

dans lequel A, B, G, D, T, m $X_1$ et $R_3$ ont la même signification que précédemment,
que l'on traite ensuite par la paraformaldéhyde préférentiellement sous atmosphère d'azote, en présence d'une dialkylamine,

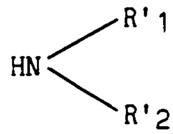

ou d'un de ses sels d'acide fort,
dans laquelle $R'_1$ et $R'_2$ signifient alkyle inférieur,
pour conduire à un dérivé de formule (I/A), cas particulier des dérivés de formule (I) :

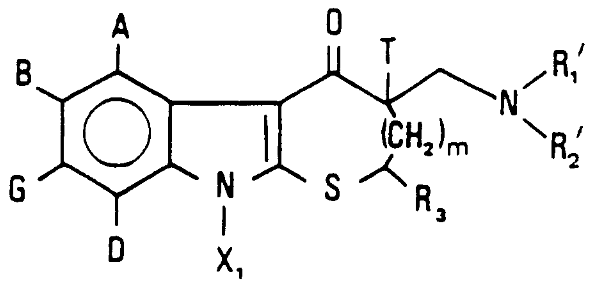

(I/A)

dans laquelle A, B, G, D, $R_3$, T, m et $X_1$ ont la même signification que précédemment et $R'_1$ et $R'_2$ signifient un groupement alcoyle inférieur, que l'on purifie si nécessaire par chromatographie ou/et cristallisation qui,

. lorsque, dans le dérivé de formule (I) que l'on souhaite obtenir, $R_1$ et $R_2$ forment avec l'atome d'azote auquel ils sont attachés un système hétérocyclique tel que défini dans la formule (I), est traité ensuite par un composé hétérocyclique azoté, l'atome d'azote étant lié à un atome d'hydrogène, mono ou bicyclique, saturé ou non, chaque cycle comprenant cinq ou six sommets et éventuellement un ou deux autres hétéroatomes choisis parmi azote, oxygène ou soufre, et éventuellement substitué par un ou plusieurs groupements alkyle ou alkoxy inférieurs linéaires ou ramifiés, ou par un groupement aryle éventuellement lui-même substitué par un ou plusieurs groupements alkyle inférieurs, alkoxy inférieurs, un ou plusieurs atome d'halogène ou groupements trifluorométhyle pour donner un composé de formule (I/B) :

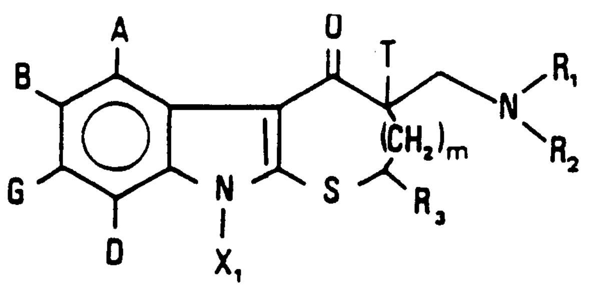

(I/B)

cas particulier des dérivés de formule (I) dans lesquels A, B, G, D, $R_3$, T, m et $X_1$ ont la même signification que précédemment et $R_1$, $R_2$ forment avec l'atome d'azote auquel ils sont attachés un système hétérocyclique tel que défini précédemment,

. lorsque $R_1$ et $R_2$ représentent dans le dérivé de formule (I) que l'on souhaite obtenir chacun un atome d'hydrogène est traité par la dihydro - 10,11 - [5H] dibenzo [a,d] cycloheptenyl - 5 amine pour conduire après purification par chromatographie et cristallisation, à un dérivé de formule (I/C1) :

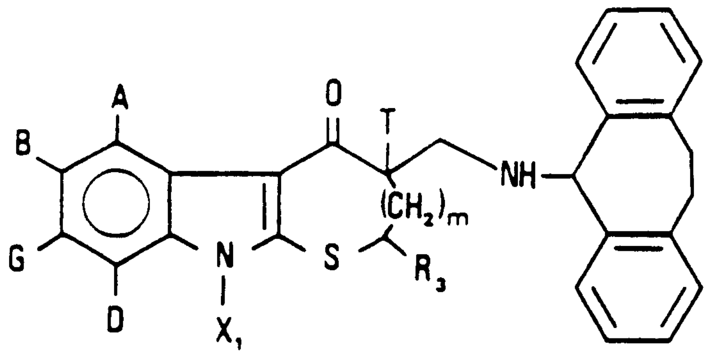

(I/C1)

cas particulier de la formule (I) dans laquelle A, B, G, D, $R_3$, T, m et $X_1$ ont la même signification que précédemment, que l'on traite par chauffage dans l'acide acétique, éventuellement dilué, pour conduire après éventuelle cristallisation à un dérivé de formule (I/C) :

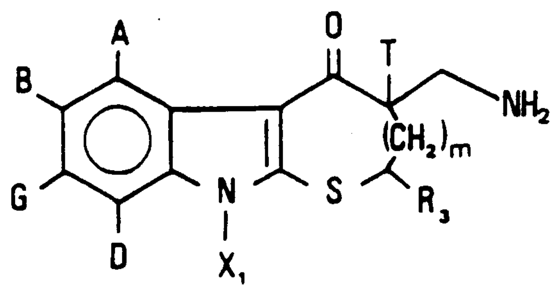

(I/C)

cas particulier des dérivés de formule (I) dans laquelle A, B, G, D, $R_3$, T, m et $X_1$ ont la même signification que précédemment,
que l'on purifie si nécessaire par chromatographie et/ou cristallisation et qui lorsque dans le dérivé de formule (I) que l'on souhaite obtenir $R_1$ représente un groupement :

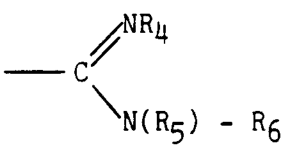

dans lequel $R_4$, $R_5$ et $R_6$ ont la même définition que dans la formule (I),
est traité par un sel d'acide fort d'un dérivé de formule (VII) :

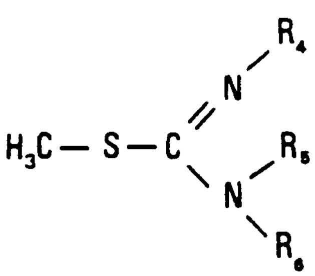

(VII)

dans laquelle $R_4$, $R_5$ et $R_6$ ont la même définition que dans la formule (I),
pour conduire, après éventuelle purification par chromatographie, traitement par un sel de métal lourd, et éventuelle cristallisation, à un dérivé de formule (I/D) :

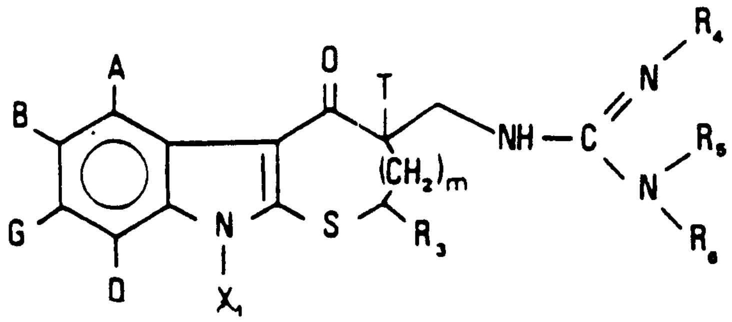

(I/D)

cas particulier de dérivé de formule (I) dans laquelle $R_1$ représente un groupement :

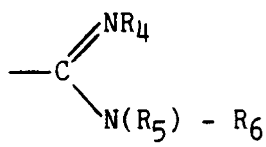

et $R_2$ représente un atome d'hydrogène,
et où A, B, G, D, $R_3$, T, m et $X_1$ ont la même signification que précédemment,
qui, dans le cas où $X_1$ représente un atome d'hydrogène peut être soumis, en fonction du composé de formule (I) que l'on désire obtenir à l'action d'un composé de formule (VIII) :

Hal—$SO_2E$ (VIII)

dans laquelle E a la même signification que dans la formule (I) et Hal représente un atome d'halogène, en présence d'hydrure de sodium, pour conduire après extraction et éventuelle purification par chromatographie sur colonne à un dérivé de formule (I/E) :

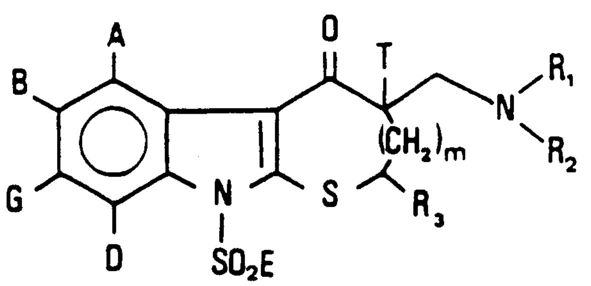

(I/E)

cas particulier des dérivés de formule (I) dans lequel X représente $SO_2E$ et où A, B, G, D, $R_1$, $R_2$, $R_3$, T, m et E ont la même signification que dans la formule (I),
dérivé de formule (I/A), (I/B), (I/C1), (I/C), (I/D) ou (I/E) qui,dans le cas où dans le composé de formule (I) que l'on souhaite obtenir n représente 1, est traité par un agent oxydant, préférentiellement l'acide métachloroperbenzoïque, pour conduire après éventuelle purification par chromatographie et/ou cristallisation au dérivé de formule (I/F):

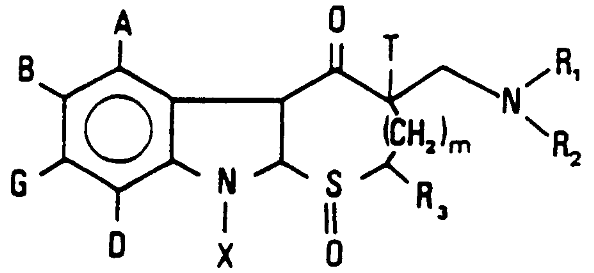

(I/F)

cas particulier des dérivés de formule (I) où A, B, G, D, X, T, $R_1$, $R_2$, $R_3$ et m ont la même signification que dans la formule (I), n représentant ici 1,
dérivé de formule (I/A), (I/B), (I/C1), (I/C), (I/D), (I/E) ou (I/F) qui, si on le désire peut être le cas échéant séparé en ses isomères et/ou salifié soit pour en faciliter la purification pour les éventuelles étapes ultérieures, soit salifié par un acide pharmaceutiquement acceptable et éventuellement cristallisé pour usage à des fins thérapeutiques.

**7.** Procédé selon la revendication 6 de préparation des composés selon l'une des revendications 2 ou 3, caractérisé en ce que l'on traite un composé de formule (I/a) :
cas particulier des dérivés de formule (I) :

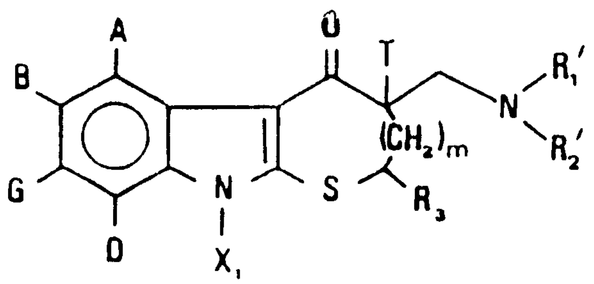

(I/A)

dans laquelle A, B, G, D, $R_3$, T, m et $X_1$ ont la même signification que dans la revendication 6 et $R'_1$ et $R'_2$ signifient un groupement alcoyle inférieur, que l'on purifie si nécessaire par chromatographie ou/et cristallisation par un dérivé de formule (M) :

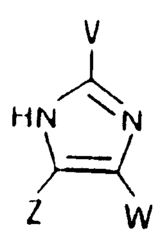

(M)

dans laquelle V, W, Z identiques ou différents représentent chacun indépendamment l'un de l'autre un groupement alkyle ou alkoxy inférieur linéaire ou ramifié ou un groupement phényle éventuellement lui-même substitué par un groupement alkyle inférieur, alkoxy inférieur, un atome d'halogène ou un groupemnt trifluorométhyle pour donner un composé de formule (I/B1) :

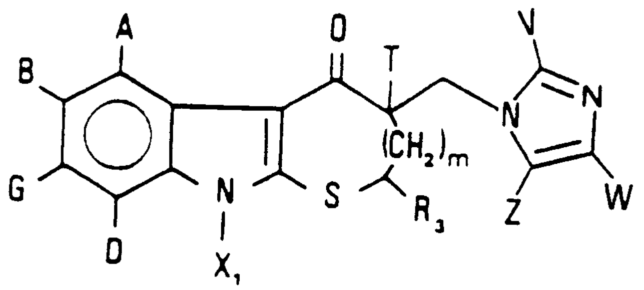

(I/B1)

dans laquelle A, B, G, D, T, $R_3$, m ont la même signification que dans la formule (I) et $X_1$, V, W et Z la même signification que précédemment, qui si on le désire peut être séparé en ses isomères et ou salifiés par un acide pharmaceutiquement acceptable et éventuellement cristallisé.

**8.** Composition pharmaceutique contenant comme principe actif au moins un composé selon l'une des revendications 1 à 5 en combinaison avec un ou plusieurs excipients ou véhicules inertes, non toxiques, pharmaceutiquement acceptables.

**9.** Composition pharmaceutique selon la revendication 8 utile dans le traitement des affections liées à un dysfonctionnement du système sérotoninergique et notamment les épisodes douloureux, l'anxiété, la schizophrénie, les vomissements et notamment ceux consécutifs à l'administration de traitement anticancéreux, les troubles du rythme cardiaque, certains désordres gastrointestinaux, ainsi que les troubles cognitifs et particulièrement les états démentiels et les troubles de la mémoire.

**Revendications pour l'Etat contractant suivant : ES**

**1.** Procédé de préparation de composés de formule générale (I) :

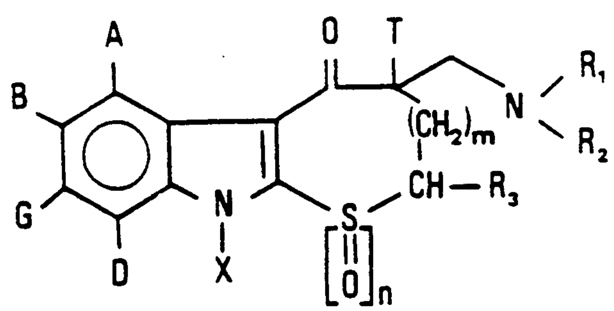

(I)

dans laquelle :

| | |
|---|---|
| A, B, G, D | identiques ou différents représentent chacun un atome d'hydrogène ou un atome d'halogène, un groupement alkoxy inférieur ou encore un groupement alkyle inférieur linéaire ou ramifié éventuellement substitué par un ou plusieurs atomes d'halogène, |
| X | représente un atome d'hydrogène, un groupement alkyle inférieur linéaire ou ramifié ou un groupement $SO_2E$ dans laquelle E représente un groupement alkyle inférieur linéaire ou ramifié ou phényle éventuellement substitué par un groupement alkyle inférieur linéaire ou ramifié, |
| T | représente un atome d'hydrogène ou un groupement alkyle inférieur, |
| $R_3$ | représente un atome d'hydrogène ou un groupement alkyle inférieur linéaire ou ramifié ou un groupement phényle éventuellement substitué par un ou plusieurs groupements alkyle inférieurs linéaires ou ramifiés, |
| n et m | identiques ou différents représentent chacun 0 ou 1, |
| $R_1$ et $R_2$ | identiques ou différents représentent chacun un atome d'hydrogène ou un groupement alkyle inférieur linéaire ou ramifié, |

ou bien,

$R_1$ et $R_2$ forment ensemble avec l'atome d'azote auxquels ils sont liés un système hétérocyclique azoté, mono ou bicyclique, saturé ou non, chaque cycle comprenant cinq ou six sommets et comprenant éventuellement un ou deux autres hétéroatomes choisis parmi azote, oxygène ou soufre, et étant éventuellement substitué par un ou plusieurs groupements alkyle ou alkoxy inférieurs, linéaires ou ramifiés ou par un groupement aryle éventuellement lui-même substitué par un ou plusieurs groupements alkyle inférieurs, alkoxy inférieurs, trifluorométhyle ou bien encore un ou plusieurs atome d'halogène,

ou bien,

$R_1$ représente un groupement

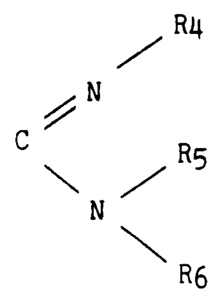

dans laquelle $R_4$, $R_5$, $R_6$ identiques ou différents représentent chacun un atome d'hydrogène ou un groupement alkyle inférieur linéaire ou ramifié ou bien $R_4$ forme avec $R_5$ un pont $(CH_2)p$, p étant compris entre 2 et 4,

ou bien encore $R_1$ représente un ensemble dihydro - 10,11 [5H] dibenzo [a,d] cycloheptenyl - 5 et $R_2$ représente un atome d'hydrogène,

le cas échéant, leurs isomères ainsi que leurs sels d'addition à un acide pharmaceutiquement acceptable,

étant entendu que par groupement alkyle inférieur ou alkoxy inférieur, on entend des groupements comprenant de 1 à 6 atomes de carbone,

caractérisé en ce que l'on condense un dérivé de formule (II) :

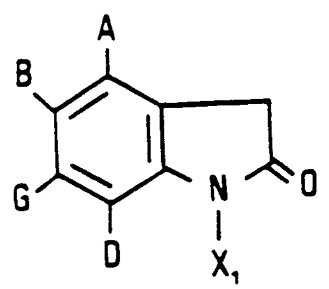

(II)

dans laquelle A, B, G, D ont la même signification que dans la formule (I),

et $X_1$ représente un atome d'hydrogène ou un groupement alkyle inférieur,

que l'on traite en présence d'un agent de thionation tel que le pentasulfure de phosphore pour obtenir un dérivé de formule (III) :

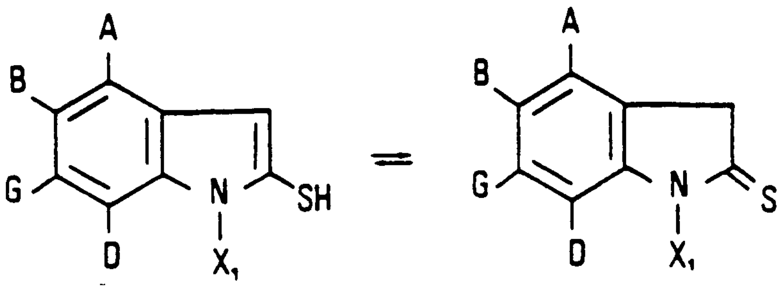

(III)

dans laquelle A, B, G, D et $X_1$ ont la même signification que précédemment, que l'on traite par un dérivé de formule (IV) :

$JCHR_3-(CH_2)_m-CTK-COOR$ (IV)

dans laquelle :

ou bien

- m représente 0 et dans ce cas J et K représentent ensemble une liaison $\pi$,

ou bien

- m représente 1 et dans ce cas J représente un atome d'halogène, et K représente un atome d'hydrogène,
- R représente un atome d'hydrogène ou un groupement alkyle inférieur linéaire ou ramifié et T et $R_3$ ont la même signification que dans la formule (I),

pour obtenir un dérivé de formule (V) :

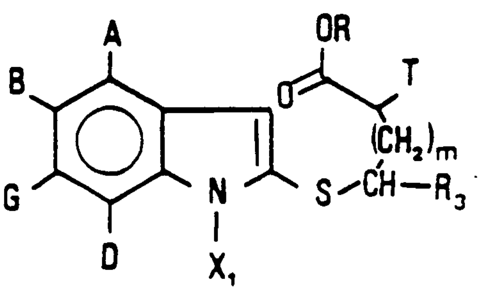

(V)

dans laquelle A, B, G, D, T, R, $R_3$, $X_1$ et m ont la même signification que précédemment, qui, lorsque R est différent de H est soumis à l'action d'un agent alcalin pour conduire à un dérivé de formule (Va), cas particulier des dérivés de formule (V) pour lesquels R représente H,

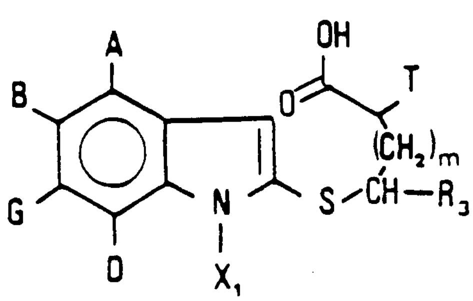

(Va)

que l'on soumet à l'action de polyphosphate ester préparé préférentiellement sous atmosphère d'azote pour conduire à un dérivé de formule (VI) :

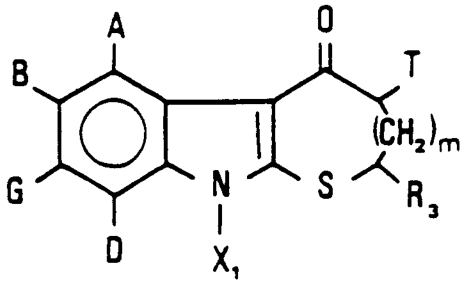

(VI)

dans lequel A, B, G, D, T, m $X_1$ et $R_3$ ont la même signification que précédemment, que l'on traite ensuite par la paraformaldéhyde préférentiellement sous atmosphère d'azote, en présence d'une dialkylamine,

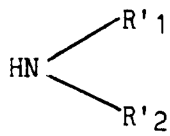

ou d'un de ses sels d'acide fort,
dans laquelle $R'_1$ et $R'_2$ signifient alkyle inférieur,
pour conduire à un dérivé de formule (I/A), cas particulier des dérivés de formule (I) :

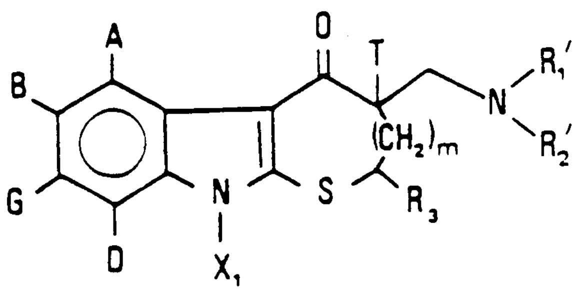

(I/A)

dans laquelle A, B, G, D, $R_3$, T, m et $X_1$ ont la même signification que précédemment et $R'_1$ et $R'_2$ signifient un groupement alcoyle inférieur, que l'on purifie si nécessaire par chromatographie ou/et cristallisation qui,

. lorsque, dans le dérivé de formule (I) que l'on souhaite obtenir, $R_1$ et $R_2$ forment avec l'atome d'azote auquel ils sont attachés un système hétérocyclique tel que défini dans la formule (I), est traité ensuite par un composé hétérocyclique azoté, l'atome d'azote étant lié à un atome d'hydrogène, mono ou bicyclique, saturé ou non, chaque cycle comprenant cinq ou six sommets et éventuellement un ou deux autres hétéroatomes choisis parmi azote, oxygène ou soufre, et éventuellement substitué par un ou plusieurs groupements alkyle ou alkoxy inférieurs linéaires ou ramifiés, ou par un groupement aryle éventuellement lui-même substitué par un ou plusieurs groupements alkyle inférieurs, alkoxy inférieurs, un ou plusieurs atome d'halogène ou groupements trifluorométhyle pour donner un composé de formule (I/B) :

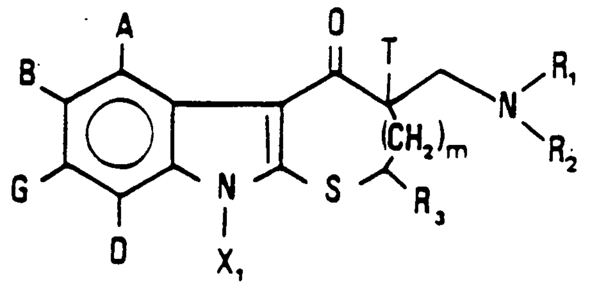

(I/B)

cas particulier des dérivés de formule (I) dans lesquels A, B, G, D, $R_3$, T, m et $X_1$ ont la même signification que précédemment et $R_1$, $R_2$ forment avec l'atome d'azote auquel ils sont attachés un système hétérocyclique tel que défini précédemment,

. lorsque $R_1$ et $R_2$ représentent dans le dérivé de formule (I) que l'on souhaite obtenir chacun un atome d'hydrogène est traité par la dihydro - 10,11 - [5H] dibenzo [a,d] cycloheptenyl - 5 amine pour conduire après purification par chromatographie et cristallisation, à un dérivé de formule (I/C1) :

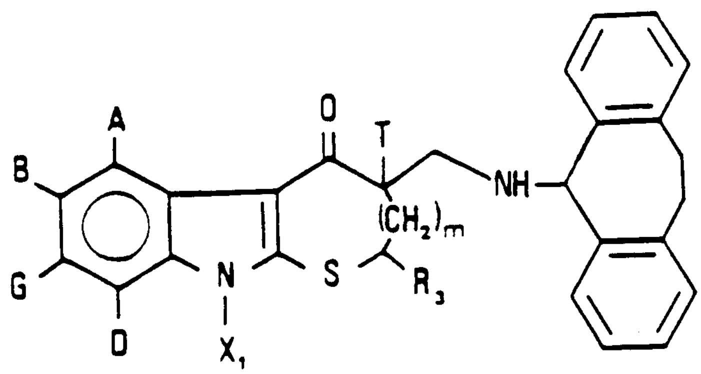

(I/C1)

cas particulier de la formule (I) dans laquelle A, B, G, D, $R_3$, T, m et $X_1$ ont la même signification que précédemment, que l'on traite par chauffage dans l'acide acétique, éventuellement dilué, pour conduire après éventuelle cristallisation à un dérivé de formule (I/C) :

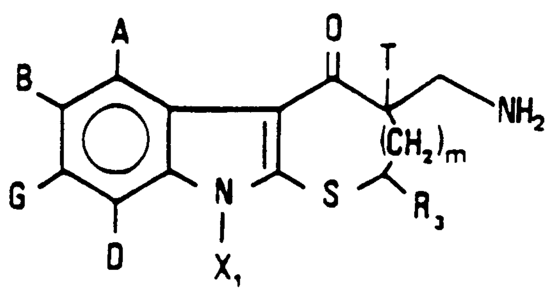

(I/C)

cas particulier des dérivés de formule (I) dans laquelle A, B, G, D, $R_3$, T, m et $X_1$ ont la même signification que précédemment,

que l'on purifie si nécessaire par chromatographie et/ou cristallisation et qui, et qui lorsque dans le dérivé de formule (I) que l'on souhaite obtenir $R_1$ représente un groupement :

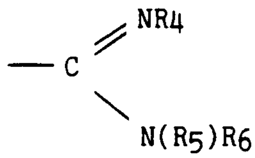

dans lequel $R_4$, $R_5$ et $R_6$ ont la même définition que dans la formule (I), est traité par un sel d'acide fort d'un dérivé de formule (VII) :

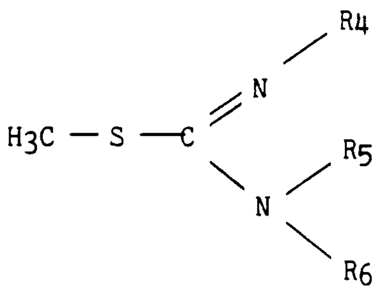

(VII)

dans laquelle $R_4$, $R_5$ et $R_6$ ont la même définition dans la formule (I), pour conduire, après éventuelle purification par chromatographie, traitement par un sel de métal lourd, et éventuelle cristallisation, à un dérivé de formule (I/D) :

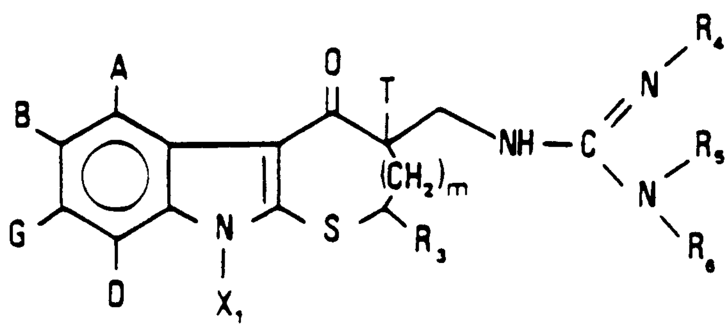

(I/D)

cas particulier de dérivé de formule (I) dans laquelle $R_1$ représente un groupement :

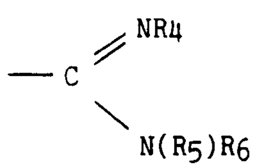

et $R_2$ représente un atome d'hydrogène,
et ou A, B, G, D, $R_3$, T, m et $X_1$ ont la même signification que précédemment, qui, dans le cas où $X_1$ représente un atome d'hydrogène peut être soumis, en fonction du composé de formule (I) que l'on désire obtenir à l'action d'un composé de formule (VIII) :

Hal—$SO_2$E (VIII)

dans laquelle E a la même signification que dans la formule (I) et Hal représente un atome d'halogène, en présence d'hydrure de sodium, pour conduire après extraction et éventuelle purification par chromatographie sur colonne à un dérivé de formule (I/E) :

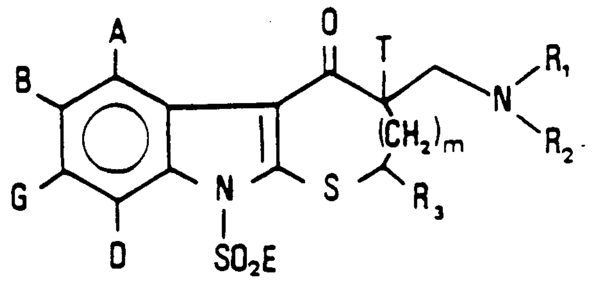

(I/E)

cas particulier des dérivés de formule (I) dans lequel X représente $SO_2E$ et où A, B, G, D, $R_1$, $R_2$, $R_3$, T, m et E ont la même signification que dans la formule (I),
dérivé de formule (I/A), (I/B), (I/C1), (I/C), (I/D) ou (I/E) qui,dans le cas où le composé de formule (I) que l'on souhaite obtenir n représente 1, est traité par un agent oxydant, préférentiellement l'acide métachloroperbenzoïque, pour conduire après éventuelle purification par chromatogaphie et/ou cristallisation au dérivé de formule (I/F):

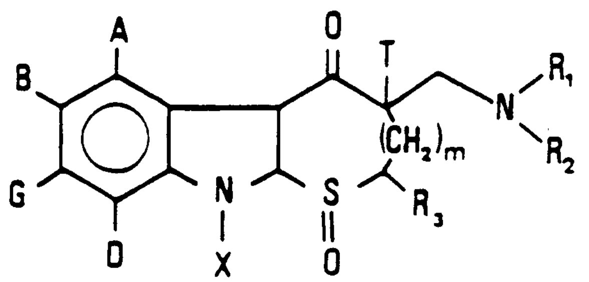

(I/F)

cas particulier des dérivés de formule (I) où A, B, G, D, X, T, $R_1$, $R_2$, $R_3$ et m ont la même signification que dans la formule (I), n représentant ici 1,
dérivé de formule (I/A), (I/B), (I/C1), (I/C), (I/D), (I/E) ou (I/F) qui, si on le désire peut être le cas échéant séparé en ses isomères et/ou salifié soit pour en faciliter la purification pour les éventuelles étapes ultérieures, soit salifié par un acide pharmaceutiquement acceptable et éventuellement cristallisé pour usage à des fins thérapeutiques.

**2.** Procédé de préparation selon la revendication 1 des composés de formule générale (I/B1) :

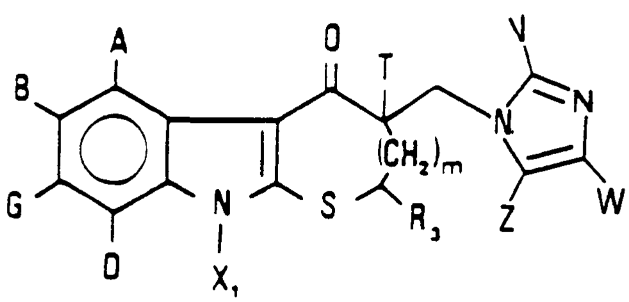

(I/B1)

dans laquelle :

A, B, G, D, T, $R_3$, m ont la même signification que dans la formule (I) selon la revendication 1,

$X_1$ représente un atome d'hydrogène ou un groupement alkyle inférieur

V, W, Z identiques ou différents représentent chacun indépendamment l'un de l'autre un groupement alkyle ou alkoxy inférieur linéaire ou ramifié ou un groupement phényle éventuellement lui-même substitué par un groupement alkyle inférieur, alkoxy inférieur, un atome d'halogène ou un groupement trifluorométhyle,

caractérisé en ce que l'on traite un composé de formule (I/a) :
cas particulier des dérivés de formule (I) :

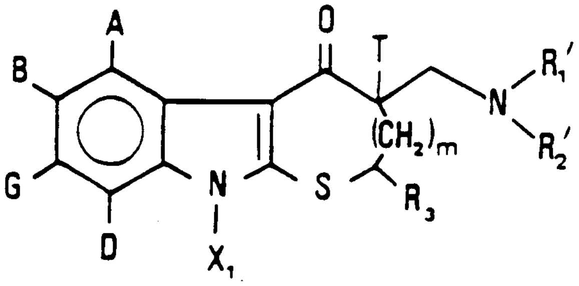

(I/A)

dans laquelle A, B, G, D, $R_3$, T, m et $X_1$ ont la même signification que dans la revendication 1 et $R'_1$ et $R'_2$ signifient un groupement alcoyle inférieur, que l'on purifie si nécessaire par chromatographie ou/et cristallisation par un dérivé de formule (M) :

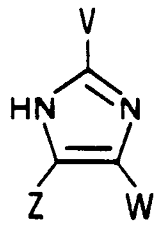

(M)

dans laquelle V, W, Z ont la même signification que précédemment pour donner un composé de formule (I/B1) qui si on le désire peut être séparé en ses isomères et ou salifiés par un acide pharmaceutiquement acceptable et éventuellement cristallisé.

**Revendications pour l'Etat contractant suivant : GR**

**1.** Procédé de préparation de composés de formule générale (I) :

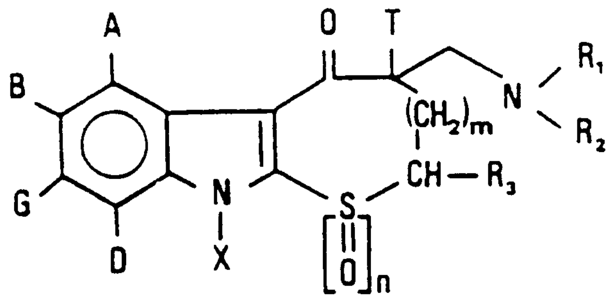

(I)

dans laquelle :

| | |
|---|---|
| A, B, G, D | identiques ou différents représentent chacun un atome d'hydrogène ou un atome d'halogène, un groupement alkoxy inférieur ou encore un groupement alkyle inférieur linéaire ou ramifié éventuellement substitué par un ou plusieurs atomes d'halogène, |
| X | représente un atome d'hydrogène, un groupement alkyle inférieur linéaire ou ramifié ou un groupement $SO_2E$ dans laquelle E représente un groupement alkyle inférieur linéaire ou ramifié ou phényle éventuellement substitué par un groupement alkyle inférieur linéaire ou ramifié, |
| T | représente un atome d'hydrogène ou un groupement alkyle inférieur, |
| $R_3$ | représente un atome d'hydrogène ou un groupement alkyle inférieur linéaire ou ramifié ou un groupement phényle éventuellement substitué par un ou plusieurs groupements alkyle inférieurs linéaires ou ramifiés, |
| n et m | identiques ou différents représentent chacun 0 ou 1, |
| $R_1$ et $R_2$ | identiques ou différents représentent chacun un atome d'hydrogène ou un groupe- |

ment alkyle inférieur linéaire ou ramifié,

ou bien,

$R_1$ et $R_2$ forment ensemble avec l'atome d'azote auxquels ils sont liés un système hétérocyclique azoté, mono ou bicyclique, saturé ou non, chaque cycle comprenant cinq ou six sommets et comprenant éventuellement un ou deux autres hétéroatomes choisis parmi azote, oxygène ou soufre, et étant éventuellement substitué par un ou plusieurs groupements alkyle ou alkoxy inférieurs, linéaires ou ramifiés ou par un groupement aryle éventuellement lui-même substitué par un ou plusieurs groupements alkyle inférieurs, alkoxy inférieurs, trifluorométhyle ou bien encore un ou plusieurs atome d'halogène,

ou bien,

$R_1$ représente un groupement

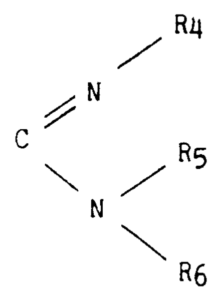

dans laquelle $R_4$, $R_5$, $R_6$ identiques ou différents représentent chacun un atome d'hydrogène ou un groupement alkyle inférieur linéaire ou ramifié ou bien $R_4$ forme avec $R_5$ un pont $(CH_2)p$, p étant compris entre 2 et 4,

ou bien encore $R_1$ représente un ensemble dihydro - 10,11 [5H] dibenzo [a,d] cycloheptenyl - 5 et $R_2$ représente un atome d'hydrogène,

le cas échéant, leurs isomères ainsi que leurs sels d'addition à un acide pharmaceutiquement acceptable,

étant entendu que par groupement alkyle inférieur ou alkoxy inférieur, on entend des groupements comprenant de 1 à 6 atomes de carbone,

caractérisé en ce que l'on condense un dérivé de formule (II) :

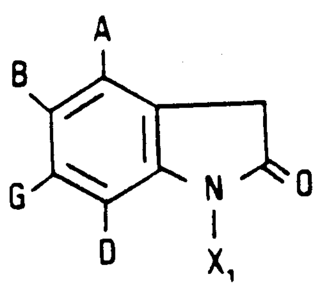

(II)

dans laquelle A, B, G, D ont la même signification que dans la formule (I),

et $X_1$ représente un atome d'hydrogène ou un groupement alkyle inférieur,

que l'on traite en présence d'un agent de thionation tel que le pentasulfure de phosphore pour obtenir un dérivé de formule (III) :

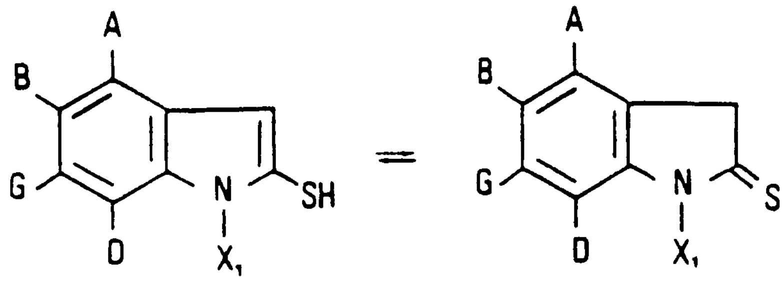

(III)

dans laquelle A, B, G, D et $X_1$ ont la même signification que précédemment, que l'on traite par un dérive de formule (IV) :

$JCHR_3-(CH_2)_m-CTK-COOR$ **(IV)**

dans laquelle :

ou bien

m représente 0 et dans ce cas J et K représentent ensemble une liaison $\pi$,

ou bien

m représente 1 et dans ce cas J représente un atome d'halogène, et K représente un atome d'hydrogène,

R représente un atome d'hydrogène ou un groupement alkyle inférieur linéaire ou ramifié et T et $R_3$ ont la même signification que dans la formule (I),

pour obtenir un dérivé de formule (V) :

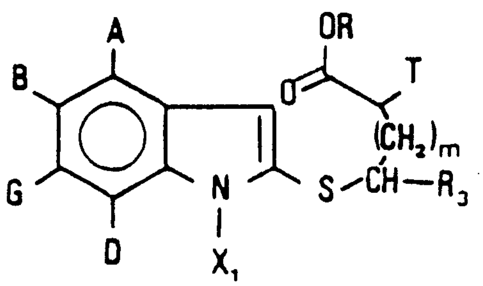

(V)

dans laquelle A, B, G, D, T, R, $R_3$, $X_1$ et m ont la même signification que précédemment, qui, lorsque R est différent de H est soumis à l'action d'un agent alcalin pour conduire à un dérivé de formule (Va), cas particulier des dérivés de formule (V) pour lesquels R représente H,

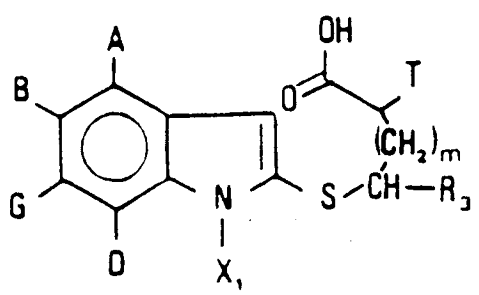

(Va)

que l'on soumet à l'action de polyphosphate ester préparé préférentiellement sous atmosphère d'azote pour conduire à un dérivé de formule (VI) :

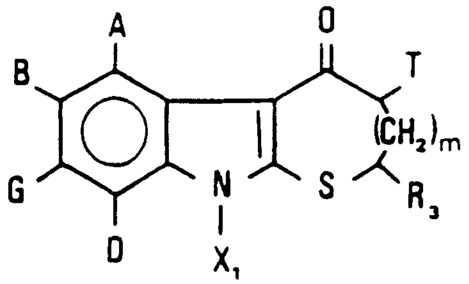

(VI)

dans lequel A, B, G, D, T, m $X_1$ et $R_3$ ont la même signification que précédemment, que l'on traite ensuite par la paraformaldéhyde préférentiellement sous atmosphère d'azote, en présence d'une dialkylamine,

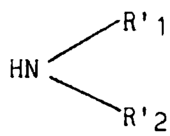

ou d'un de ses sels d'acide fort, dans laquelle $R'_1$ et $R'_2$ signifient alkyle inférieur, pour conduire à un dérivé de formule (I/A), cas particulier des dérivés de formule (I) :

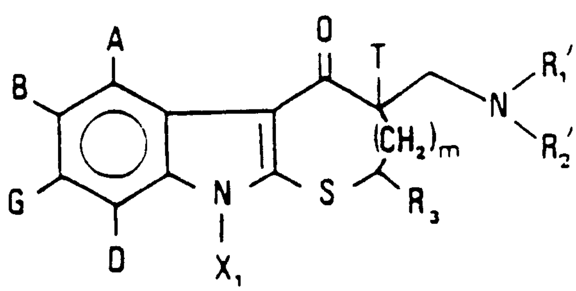

(I/A)

dans laquelle A, B, G, D, $R_3$, T, m et $X_1$ ont la même signification que précédemment et $R'_1$ et $R'_2$ signifient un groupement alcoyle inférieur, que l'on purifie si nécessaire par chromatographie ou/et cristallisation qui,

- lorsque, dans le dérivé de formule (I) que l'on souhaite obtenir, $R_1$ et $R_2$ forment avec l'atome d'azote auquel ils sont attachés un système hétérocyclique tel que défini dans la formule (I), est traité ensuite par un composé hétérocyclique azoté, l'atome d'azote étant lié à un atome d'hydrogène, mono ou bicyclique, saturé ou non, chaque cycle comprenant cinq ou six sommets et éventuellement un ou deux autres hétéroatomes choisis parmi azote, oxygène ou soufre, et éventuellement substitué par un ou plusieurs groupements alkyle ou alkoxy inférieurs linéaires ou ramifiés, ou par un groupement aryle éventuellement lui-même substitué par un ou plusieurs groupements alkyle inférieurs, alkoxy inférieurs, un ou plusieurs atome d'halogène ou groupements trifluorométhyle pour donner un composé de formule (I/B) :

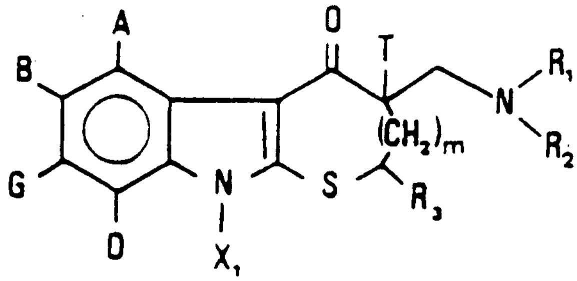

(I/B)

cas particulier des dérivés de formule (I) dans lesquels A, B, G, D, $R_3$, T, m et $X_1$ ont la même signification que précédemment et $R_1$, $R_2$ forment avec l'atome d'azote auquel ils sont attachés un système hétérocyclique tel que défini précédemment,

. lorsque $R_1$ et $R_2$ représentent dans le dérivé de formule (I) que l'on souhaite obtenir chacun un atome d'hydrogène est traité par la dihydro - 10,11 - [5H] dibenzo [a,d] cycloheptenyl - 5 amine pour conduire après purification par chromatographie et cristallisation, à un dérivé de formule (I/C1) :

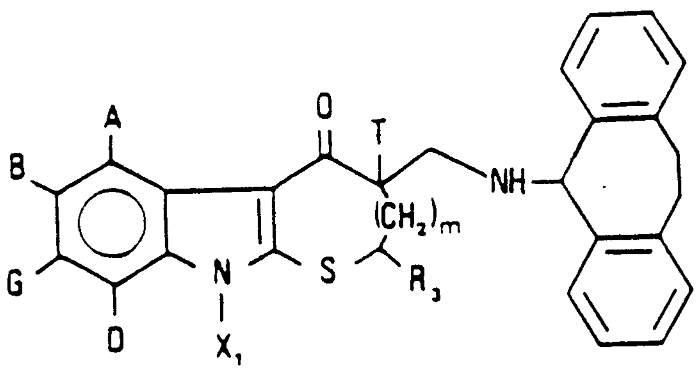

(I/C1)

cas particulier ce la formule (I) dans laquelle A, B, G, D, $R_3$, T, m et $X_1$ ont la même signification que précédemment, que l'on traite par chauffage dans l'acide acétique, éventuellement dilué, pour conduire après éventuelle cristallisation à un dérivé de formule (I/C) :

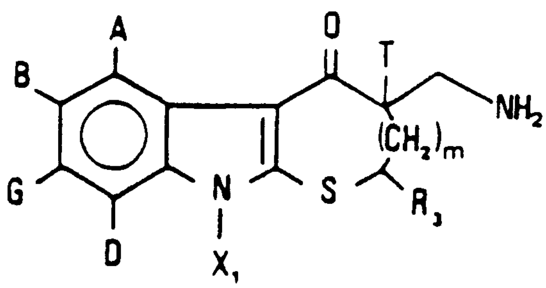

(I/C)

cas particulier des dérivés de formule (I) dans laquelle A, B, G, D, $R_3$, T, m et $X_1$ ont la même signification que précédemment,

que l'on purifie si nécessaire par chromatographie et/ou cristallisation et qui, et qui lorsque dans le dérivé de formule (I) que l'on souhaite obtenir $R_1$ représente un groupement :

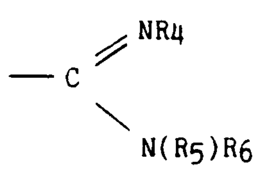

dans lequel $R_4$, $R_5$ et $R_6$ ont la même définition que dans la formule (I), est traité par un sel d'acide fort d'un dérivé de formule (VII) :

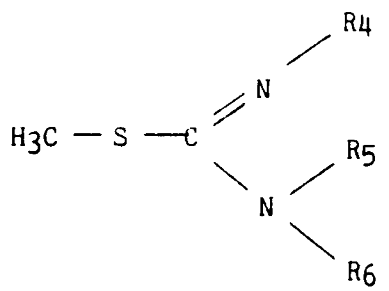

**(VII)**

dans laquelle $R_4$, $R_5$ et $R_6$ ont la même définition dans la formule (I), pour conduire, après éventuelle purification par chromatographie, traitement par un sel de métal lourd, et éventuelle cristallisation, à un dérivé de formule (I/D) :

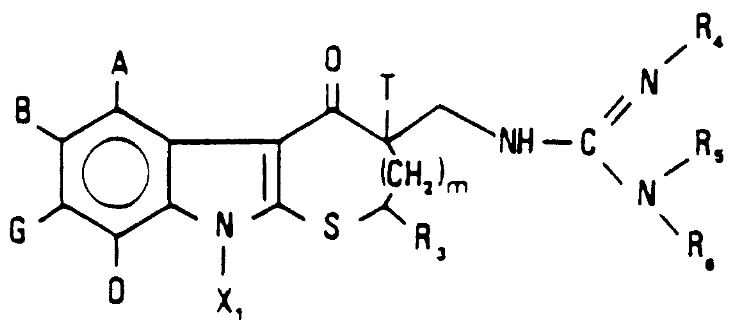

**(I/D)**

cas particulier de dérivé de formule (I) dans laquelle $R_1$ représente un groupement :

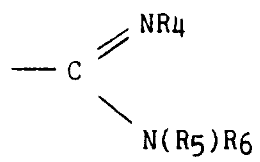

et $R_2$ représente un atome d'hydrogène, et ou A, B, G, D, $R_3$, T, m et $X_1$ ont la même signification que précédemment, qui, dans le cas où $X_1$ représente un atome d'hydrogène peut être soumis, en fonction du composé de formule (I) que l'on désire obtenir à l'action d'un composé de formule (VIII) :

$Hal—SO_2E$ **(VIII)**

dans laquelle E a la même signification que dans la formule (I) et Hal représente un atome d'halogène, en présence d'hydrure de sodium, pour conduire après extraction et éventuelle purification par chromatographie sur colonne à un dérivé de formule (I/E) :

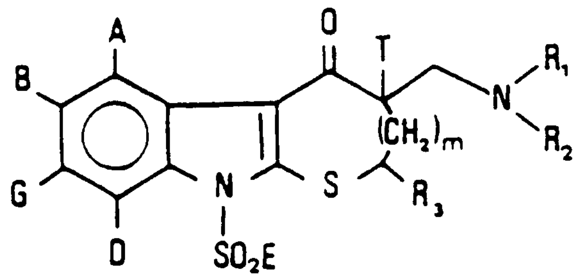

(I/E)

cas particulier des dérivés de formule (I) dans lequel X représente $SO_2E$ et où A, B, G, D, $R_1$, $R_2$, $R_3$, T, m et E ont la même signification que dans la formule (I),
dérivé de formule (I/A), (I/B), (I/C1), (I/C), (I/D) ou (I/E) qui,dans le cas où le composé de formule (I) que l'on souhaite obtenir n représente 1, est traité par un agent oxydant, préférentiellement l'acide métachloroperbenzoïque, pour conduire après éventuelle purification par chromatogaphie et/ou cristallisation au dérivé de formule (I/F):

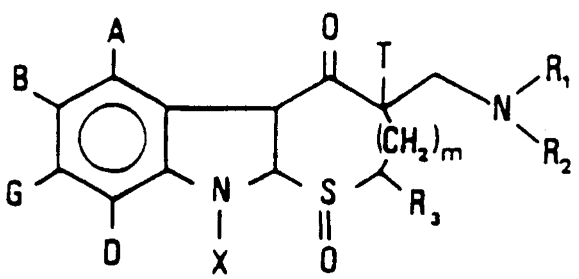

(I/F)

cas particulier des dérivés de formule (I) où A, B, G, D, X, T, $R_1$, $R_2$, $R_3$ et m ont la même signification que dans la formule (I), n représentant ici 1,
dérivé de formule (I/A), (I/B), (I/C1), (I/C), (I/D), (I/E) ou (I/F) qui, si on le désire peut être le cas échéant séparé en ses isomères et/ou salifié soit pour en faciliter la purification pour les éventuelles étapes ultérieures, soit salifié par un acide pharmaceutiquement acceptable et éventuellement cristallisé pour usage à des fins thérapeutiques.

2. Procédé de préparation selon la revendication 1 des composés de formule générale (I/B1) :

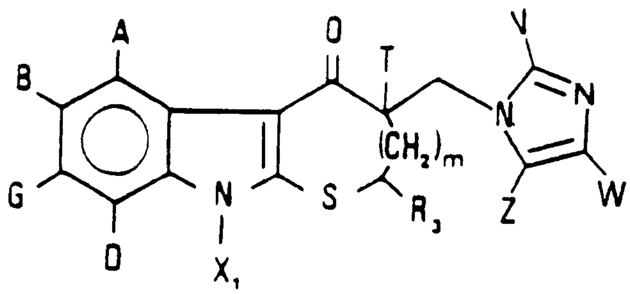

(I/B1)

dans laquelle :

A, B, G, D, T, $R_3$, m ont la même signification que dans la formule (I) selon la revendication 1,

$X_1$ représente un atome d'hydrogène ou un groupement alkyle inférieur

Y, W, Z identiques ou différents représentent chacun indépendamment l'un de l'autre un groupement alkyle ou alkoxy inférieur linéaire ou ramifie ou un groupement phényle éventuellement lui-même substitué par un groupement alkyle inférieur, alkoxy inférieur, un atome d'halogène ou un groupement trifluorométhyle,

caractérisé en ce que l'on traite un composé de formule (I/a) :
cas particulier des dérivés de formule (I) :

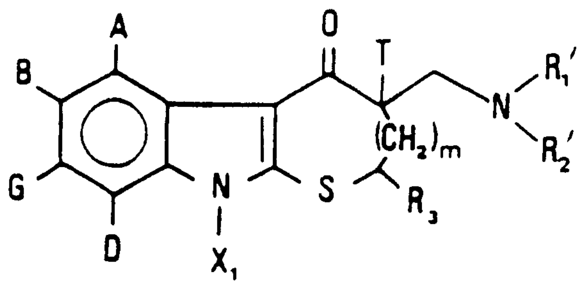

(I/A)

dans laquelle A, B, G, D, $R_3$, T, m et $X_1$ ont la même signification que dans la revendication 1 et $R'_1$ et $R'_2$ signifient un groupement alcoyle inférieur, que l'on purifie si nécessaire par chromatographie ou/et cristallisation par un dérivé de formule (M) :

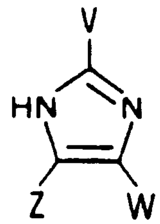

(M)

dans laquelle Y, W, Z ont la même signification que précédemment pour donner un composé de formule (I/B1) qui si on le désire peut être séparé en ses isomères et ou salifiés par un acide pharmaceutiquement acceptable et éventuellement cristallisé.

## Claims

**Claims for the following Contracting States : AT, BE, CH, DE, FR, GB, IT, LI, LU, NL, SE**

**1.** Compounds of formula (I):

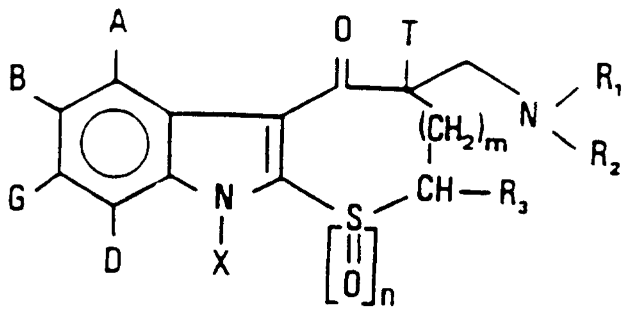

(I)

in which:

each of

A, B, G and D, which may be identical or different, represents a hydrogen atom or a halogen atom, a lower alkoxy grouping or a linear or branched lower alkyl grouping optionally substituted by one or more halogen atoms,

X represents a hydrogen atom, a linear or branched lower alkyl grouping or a $SO_2E$ grouping in which E represents a linear or branched lower alkyl grouping or a phenyl grouping optionally substituted by a linear or branched lower alkyl grouping,

T represents a hydrogen atom or a lower alkyl grouping,

$R_3$ represents a hydrogen atom or a linear or branched lower alkyl grouping or a

phenyl grouping optionally substituted by one or more linear or branched lower alkyl groupings,

each of
n and m, which may be identical or different, represents 0 or 1,

each of
$R_1$ and $R_2$, which may be identical or different, represents a hydrogen atom or a linear or branched lower alkyl grouping,

or
$R_1$ and $R_2$ form together with the nitrogen atom to which they are bonded a mono- or bi-cyclic, saturated or unsaturated nitrogen-containing heterocyclic system, each ring comprising 5 or 6 ring members and optionally containing one or two further hetero atoms selected from nitrogen, oxygen and sulphur, and being optionally substituted by one or more linear or branched lower alkyl or alkoxy groupings, or by an aryl grouping which is itself optionally substituted by one or more lower alkyl, lower alkoxy or trifluoromethyl groupings or one or more halogen atoms,

or
$R_1$ represents a grouping

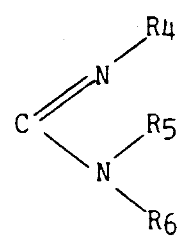

in which each of $R_4$, $R_5$ and $R_6$, which may be identical or different, represents a hydrogen atom or a linear or branched lower alkyl grouping or $R_4$ forms with $R_5$ a $(CH_2)_p$ bridge, p being an integer from 2 to 4,
or $R_1$ represents a 10,11-dihydro-[5H]-dibenzo[a,d]5-cycloheptenyl group and $R_2$ represents a hydrogen atom,
where appropriate, their isomers and also their addition salts with a pharmaceutically acceptable acid,
it being understood that lower alkyl and lower alkoxy groupings are to be understood as being groupings comprising from 1 to 6 carbon atoms.

2. Compounds according to claim 1 in which n represents 0, X represents a linear or branched lower alkyl grouping and $R_1$ and $R_2$ form together with the nitrogen atom to which they are attached an imidazole ring optionally substituted by one or more linear or branched lower alkyl or alkoxy groupings, or by an aryl grouping which is itself optionally substituted by a lower alkyl or lower alkoxy grouping, trifluoromethyl or a halogen atom, their isomers and also their addition salts with a pharmaceutically acceptable acid.

3. Compounds according to either claim 1 or claim 2, in which $R_1$ and $R_2$ form together with the nitrogen atom to which they are attached a 2-methylimidazole grouping.

4. Compound according to either claim 1 or claim 3 which is 3-[(2-methyl-1-imidazolyl)methyl]-4-oxo-9-methyl-2,3,4,9-tetrahydrothiopyrano[2,3-b]indole, and also its addition salts with a pharmaceutically acceptable acid.

5. Compound according to either claim 1 or claim 3 which is 4-[(2-methyl-1-imidazolyl)methyl]5-oxo-10-methyl-2,3,4,5,10-pentahydrothiepino[2,3-b]indole and also its addition salts with a pharmaceutically acceptable acid.

6. Process for the synthesis of the compounds of formula (I), characterised in that one condenses a compound of formula (II):

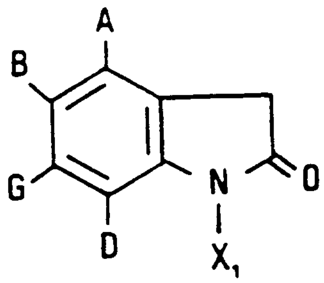

(II)

in which A, B, G and D are as defined in formula (I),
and $X_1$ represents a hydrogen atom or a lower alkyl grouping,
which is treated in the presence of a thionising agent, such as phosphorus pentasulphide, to obtain a compound of formula (III):

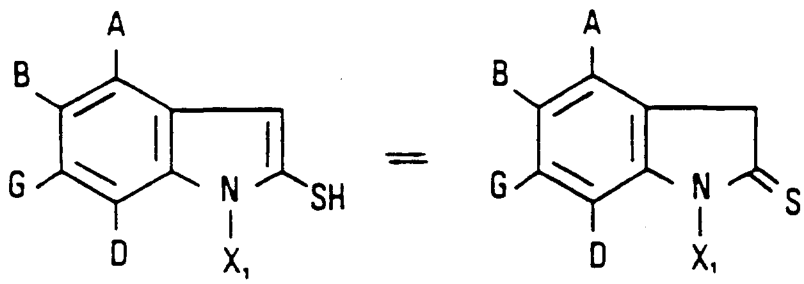

(III)

in which A, B, G, D and $X_1$ are as defined above,
which is treated with a compound of formula (IV):

$JCHR_3$-$(CH_2)_m$-CTK-COOR (IV)

in which:
either
m represents 0 and in that case J and K together represent a $\pi$ bond,
or
m represents 1 and in that case J represents a halogen atom and K represents a hydrogen atom,
R represents a hydrogen atom or a linear or branched lower alkyl grouping and T and $R_3$ are as defined in formula (I),
to obtain a compound of formula (V):

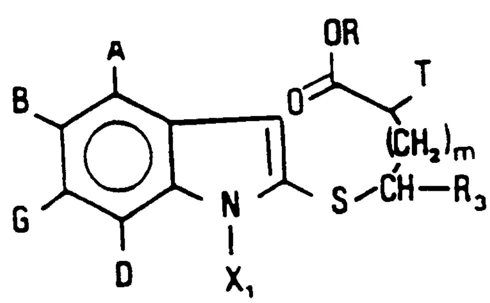

(V)

in which A, B, G, D, T, R, $R_3$, $X_1$ and m are as defined above,
which, when R is other than H, is subjected to the action of an alkaline agent to yield a compound of formula (Va), a particular case of the compounds of formula (V) in which R represents H,

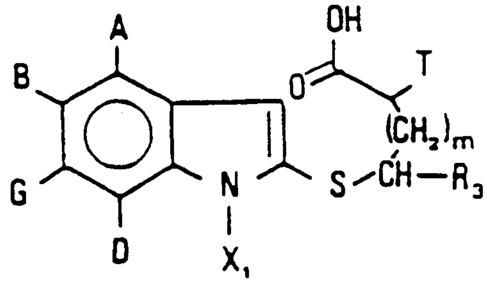

(Va)

which is subjected to the action of polyphosphate ester prepared preferably under a nitrogen atmosphere to yield a compound of formula (VI):

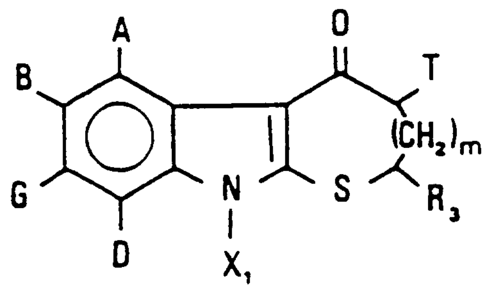

(VI)

in which A, B, G, D, T, m, $X_1$ and $R_3$ are as defined above,
which is then treated with paraformaldehyde, preferably under a nitrogen atmosphere,
in the presence of a dialkylamine,

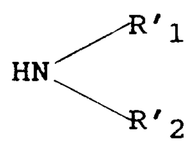

,

in which
$R'_1$ and $R'_2$ represent lower alkyl, or one of its salts with a strong acid,
to yield a compound of formula (I/A), a particular case of the compounds of formula (I):

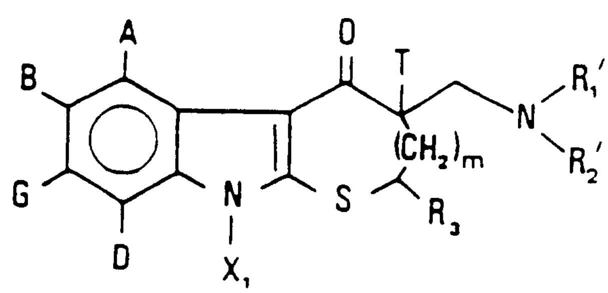

(I/A)

in which A, B, G, D, $R_3$, T, m and $X_1$ are as defined above and $R'_1$ and $R'_2$ represent a lower alkyl grouping, which is purified, if necessary, by chromatography and/or crystallisation and which

. when, in the compound of formula (I) that it is desired to obtain, $R_1$ and $R_2$ form with the nitrogen atom to which they are attached a heterocyclic system such as defined in formula (I), is then treated with a nitrogen-containing mono- or bi-cyclic, saturated or unsaturated heterocyclic compound, the nitrogen atom being bonded to a hydrogen atom, each ring comprising 5 or 6 ring members and optionally one or two further hetero atoms selected from nitrogen, oxygen and sulphur, and being optionally substituted by one or more linear or branched lower alkyl or alkoxy

groupings or by an aryl grouping which is itself optionally substituted by one or more lower alkyl or lower alkoxy groupings, one or more halogen atoms or trifluoromethyl groupings to give a compound of formula (I/B):

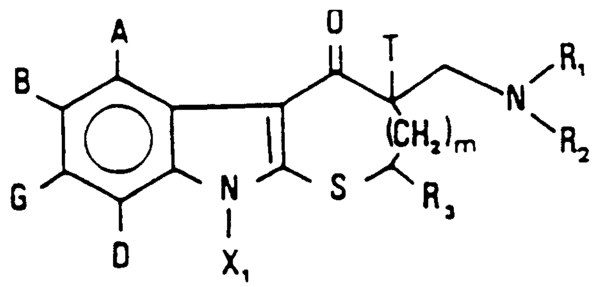

(I/B)

a particular case of the compounds of formula (I) in which A, B, G, D, $R_3$, T m and $X_1$ are as defined above and $R_1$ and $R_2$ form with the nitrogen atom to which they are attached a heterocyclic system such as defined above,

. when each of $R_1$ and $R_2$ represents in the compound of formula (I) that it is desired to obtain a hydrogen atom, is treated with 10,11-dihydro-[5H]-dibenzo[a,d]5-cycloheptenylamineto yield, after purification by chromatography and crystallisation, a compound of formula (I/C1):

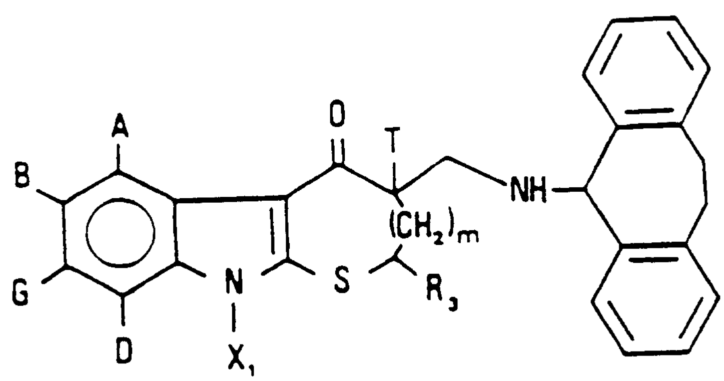

(I/C1)

a particular case of the formula (I) in which A, B, G, D, $R_3$, T, m and $X_1$ are as defined above, which is treated by heating in optionally diluted acetic acid to yield, after optional crystallisation, a compound of formula (I/C):

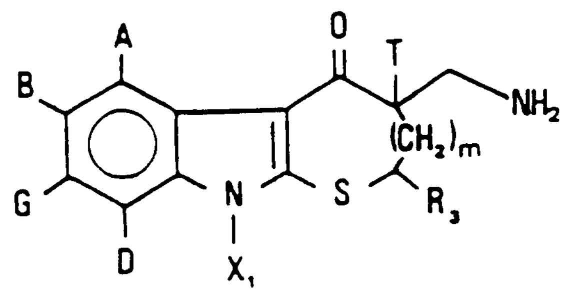

(I/C)

a particular case of the compounds of formula (I) in which A, B, G, D, $R_3$, T m and $X_1$ are as defined above,

which is purified, if necessary, by chromatography and/or crystallisation and which, when in the compound of formula (I) that it is desired to obtain $R_1$ represents a grouping:

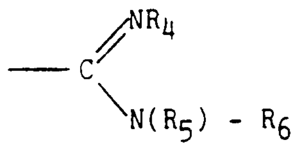

in which $R_4$, $R_5$ and $R_6$ are as defined in formula (I), is treated with a salt of a strong acid of a compound of formula (VII):

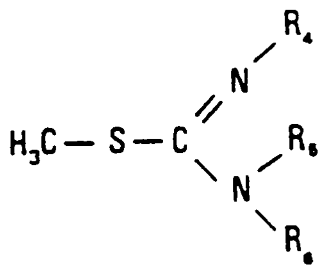

(VII)

in which $R_4$, $R_5$ and $R_6$ are as defined in formula (I), to yield, after optional purification by chromatography, treatment with a heavy metal salt, and optional crystallisation, a compound of formula (I/D):

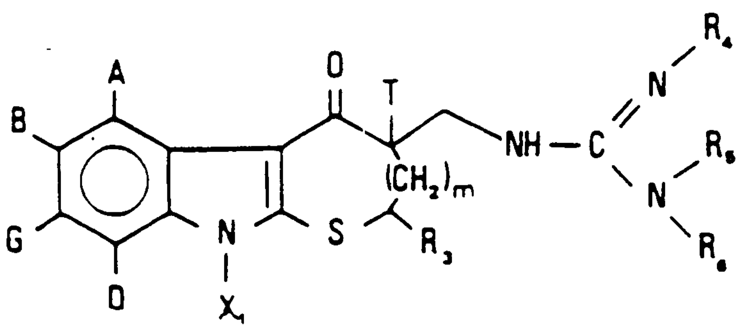

(I/D)

a particular case of the compound of formula (I) in which $R_1$ represents a grouping:

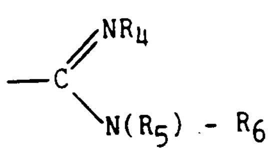

and $R_2$ represents a hydrogen atom, and in which A, B, G, D, $R_3$, T, m and $X_1$ are as defined above, which, in the case where $X_1$ represents a hydrogen atom may be subjected, according to the compound of formula (I) that it is desired to obtain, to the action of a compound of formula (VIII):

$Hal\text{-}SO_2E$ (VIII)

in which E is as defined in formula (I) and Hal represents a halogen atom, in the presence of sodium hydride, to yield, after extraction and optional purification by column chromatography, a compound of formula (I/E):

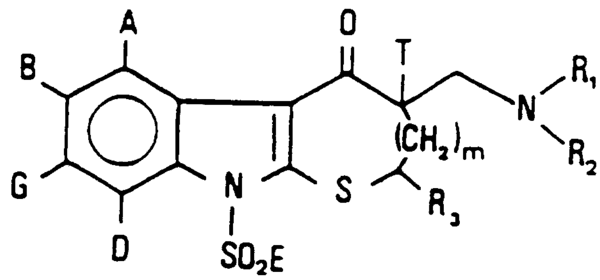

(I/E)

a particular case of the compounds of formula (I) in which X represents $SO_2E$ and in which A, B, G, D, $R_1$, $R_2$, $R_3$, T, m and E are as defined in formula (I), which compound of formula (I/A), (I/B), (I/C1), (I/C), (I/D) or (I/E), in the case where, in the compound of formula (I) that it is desired to obtain, n represents 1, is treated with an oxidising agent, preferably metachloroperbenzoic acid, to yield, after optional purification by chromatography and/or crystallisation, the compound of formula (I/F):

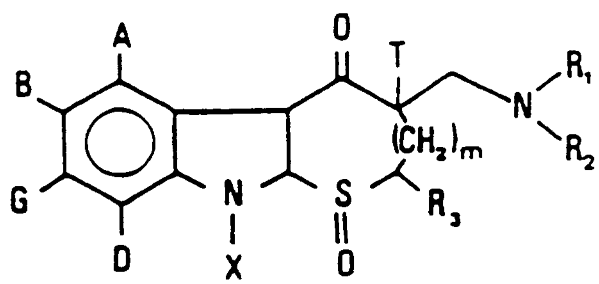

(I/F).

a particular case of the compounds of formula (I) in which A, B, G, D, X, T, $R_1$, $R_2$, $R_3$ and m are as defined in formula (I), n in this case representing 1, which compound of formula (I/A), (I/B), (I/C1), (I/C), (I/D), (I/E) or (I/F) may, if desired, be separated, where appropriate, into its isomers and/or either converted into a salt to facilitate its purification for any later stages, or converted into a salt by means of a pharmaceutically acceptable acid and optionally crystallised for use for therapeutic purposes.

**7.** Process according to claim 6 for the preparation of the compounds according to either claim 2 or claim 3, characterised in that a compound of formula (I/a): a particular case of the compounds of formula (I):

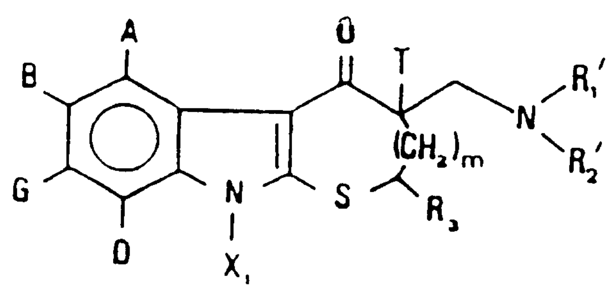

(I/A)

in which A, B, G, D, $R_3$, T, m and $X_1$ are as defined in claim 6 and $R'_1$ and $R'_2$ represent a lower alkyl grouping, which is purified, if necessary, by chromatography and/or crystallisation, is treated by a compound of formula (M):

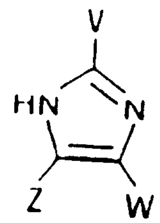

(M)

in which each of V, W and Z, which may be identical or different, represents, independently of the others, a linear or branched lower alkyl or alkoxy grouping or a phenyl grouping which is itself optionally substituted by a lower alkyl or lower alkoxy grouping, a halogen atom or a trifluoromethyl grouping to give a compound of formula (I/B1):

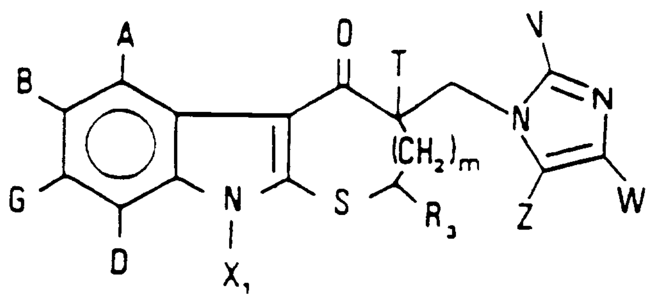

(I/B1)

in which A, B, G, D, T, $R_3$ and m are as defined in formula (I) and $X_1$, V, W and Z are as defined above, which, if desired, can be separated into its isomers and/or converted into a salt by means of a pharmaceutically acceptable acid and optionally crystallised.

**8.** Pharmaceutical composition containing as active ingredient at least one compound according to any one of claims 1 to 5 in combination with one or more pharmaceutically acceptable, inert, non-toxic excipients or carriers.

**9.** Pharmaceutical composition according to claim 8 which can be used in the treatment of disorders associated with a dysfunction of the serotoninergic system and especially painful episodes, anxiety, schizophrenia, vomiting and especially vomiting following the administration of anti-cancer treatment, cardiac rhythm disorders, certain gastrointestinal disorders, and also cognitive disorders and especially states of dementia and memory disorders.

**Claims for the following Contracting State : ES**

**1.** Process for the preparation of compounds of the general formula (I):

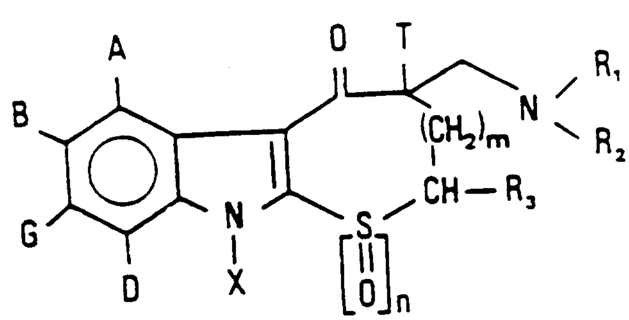

(I)

in which:

each of

A, B, G and D, which may be identical or different, represents a hydrogen atom or a halogen atom, a lower alkoxy grouping or a linear or branched lower alkyl grouping optionally substituted by one or more halogen atoms,

X represents a hydrogen atom, a linear or branched lower alkyl grouping or a $SO_2E$ grouping in which E represents a linear or branched lower alkyl grouping or a phenyl grouping optionally substituted by a linear or branched lower alkyl grouping,

T represents a hydrogen atom or a lower alkyl grouping,

$R_3$ represents a hydrogen atom or a linear or branched lower alkyl grouping or a phenyl grouping optionally substituted by one or more linear or branched lower alkyl groupings,

each of

$\underline{n}$ and $\underline{m}$, which may be identical or different, represents 0 or 1,

each of

$R_1$ and $R_2$, which may be identical or different, represents a hydrogen atom or a linear or branched lower alkyl grouping,

or

$R_1$ and $R_2$ form together with the nitrogen atom to which they are bonded a mono- or bi-cyclic, saturated or unsaturated nitrogen-containing heterocyclic system, each ring comprising 5 or 6 ring members and optionally containing one or two further hetero atoms selected from nitrogen, oxygen and sulphur, and being optionally substituted by one or more linear or branched lower alkyl or alkoxy groupings, or by an aryl grouping which is itself optionally substituted by one or more lower alkyl, lower alkoxy or trifluoromethyl groupings or one or more halogen atoms,

or

$R_1$ represents a grouping

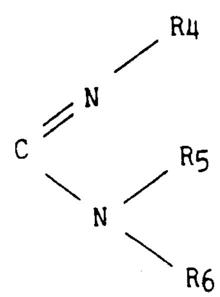

in which each of $R_4$, $R_5$ and $R_6$, which may be identical or different, represents a hydrogen atom or a linear or branched lower alkyl grouping or $R_4$ forms with $R_5$ a $(CH_2)_p$ bridge, $\underline{p}$ being an integer from 2 to 4,

or $R_1$ represents a 10,11-dihydro-[5H]-dibenzo[a,d]5-cycloheptenyl group and $R_2$ represents a hydrogen atom,

where appropriate, their isomers and also their addition salts with a pharmaceutically acceptable acid,

it being understood that lower alkyl and lower alkoxy groupings are to be understood as being groupings comprising from 1 to 6 carbon atoms, characterised in that a compound of formula (II):

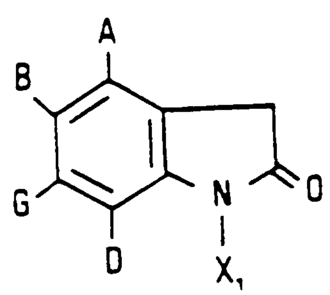

(II)

in which A, B, G and D are as defined in formula (I),
and $X_1$ represents a hydrogen atom or a lower alkyl grouping,
treated in the presence of a thionising agent, such as phosphorus pentasulphide, to obtain a compound of formula (III):

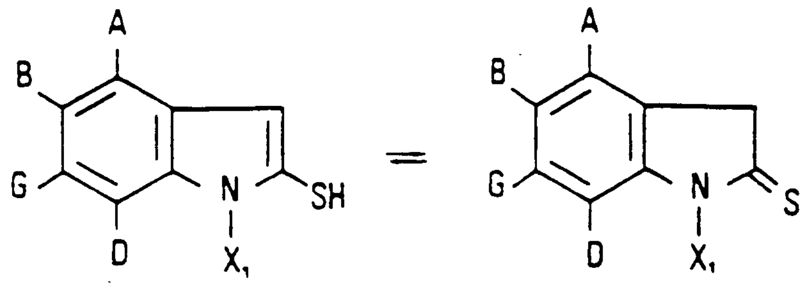

(III)

in which A, B, G, D and $X_1$ are as defined above, is condensed,
treated with a compound of formula (IV):

$JCHR_3\text{-}(CH_2)_m\text{-}CTK\text{-}COOR$ (IV)

in which:
either
- m represents 0 and in that case J and K together represent a $\pi$ bond,

or
- m represents 1 and in that case J represents a halogen atom and K represents a hydrogen atom,
- R represents a hydrogen atom or a linear or branched lower alkyl grouping and T and $R_3$ are as defined in formula (I),

to obtain a compound of formula (V):

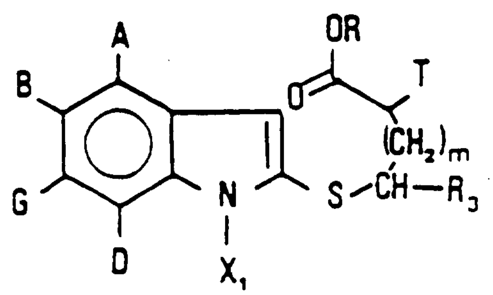

(V)

in which A, B, G, D, T, R, $R_3$, $X_1$ and m are as defined above,
which, when R is other than H, is subjected to the action of an alkaline agent to yield a compound of formula (Va), a particular case of the compounds of formula (V) in which R represents H,

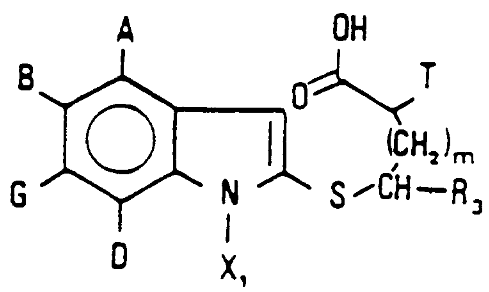

(Va)

which is subjected to the action of polyphosphate ester prepared preferably under a nitrogen

atmosphere to yield a compound of formula (VI):

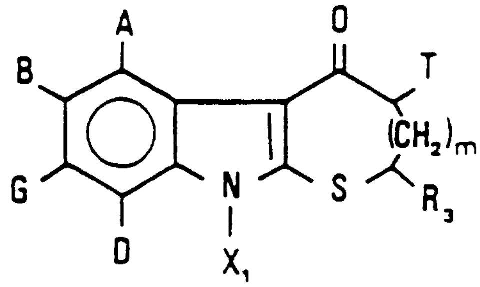

(VI)

in which A, B, G, D, T, m, $X_1$ and $R_3$ are as defined above,
which is then treated with paraformaldehyde, preferably under a nitrogen atmosphere,
in the presence of a dialkylamine,

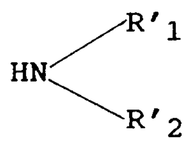

,

in which
$R'_1$ and $R'_2$ represent lower alkyl, or one of its salts with a strong acid,
to yield a compound of formula (I/A), a particular case of the compounds of formula (I):

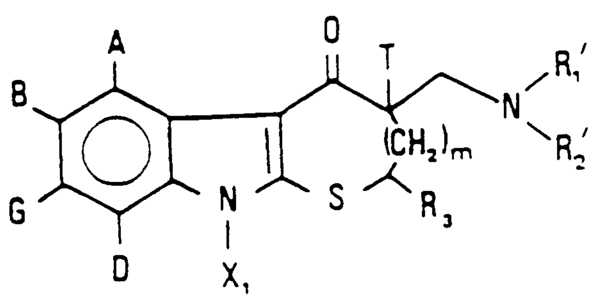

(I/A)

in which A, B, G, D, $R_3$, T, m and $X_1$ are as defined above and $R'_1$ and $R'_2$ represent a lower alkyl grouping, which is purified, if necessary, by chromatography and/or crystallisation and which

. when, in the compound of formula (I) that it is desired to obtain, $R_1$ and $R_2$ form with the nitrogen atom to which they are attached a heterocyclic system such as defined in formula (I), is then treated with a nitrogen-containing mono- or bi-cyclic, saturated or unsaturated heterocyclic compound, the nitrogen atom being bonded to a hydrogen atom, each ring comprising 5 or 6 ring members and optionally one or two further hetero atoms selected from nitrogen, oxygen and sulphur, and being optionally substituted by one or more linear or branched lower alkyl or alkoxy groupings or by an aryl grouping which is itself optionally substituted by one or more lower alkyl or lower alkoxy groupings, one or more halogen atoms or trifluoromethyl groupings to give a compound of formula (I/B):

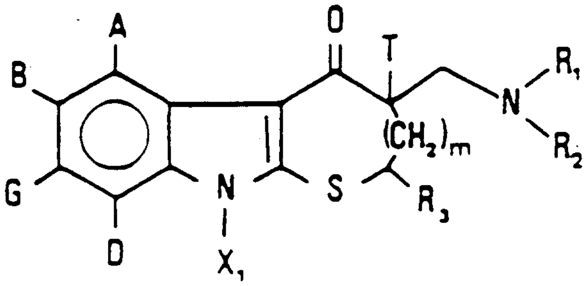

(I/B)

a particular case of the compounds of formula (I) in which A, B, G, D, $R_3$, T, m and $X_1$ are as defined above and $R_1$ and $R_2$ form with the nitrogen atom to which they are attached a heterocyclic system such as defined above,

. when each of $R_1$ and $R_2$ represents in the compound of formula (I) that it is desired to obtain a hydrogen atom, is treated with 10,11-dihydro-[5H]-dibenzo[a,d]5-cycloheptenylamineto yield, after purification by chromatography and crystallisation, a compound of formula (I/C1):

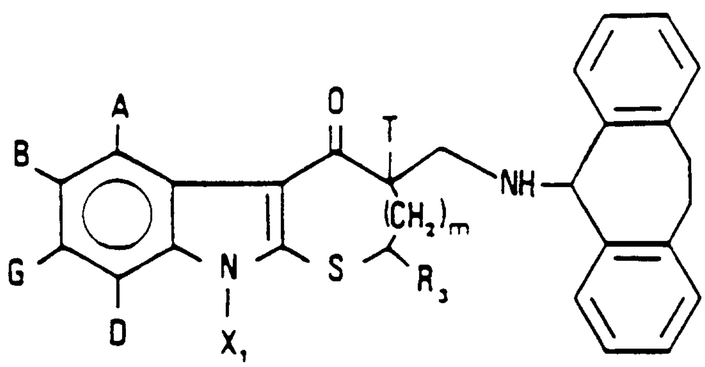

(I/C1)

a particular case of the formula (I) in which A, B, G, D, $R_3$, T, m and $X_1$ are as defined above, which is treated by heating in optionally diluted acetic acid to yield, after optional crystallisation, a compound of formula (I/C):

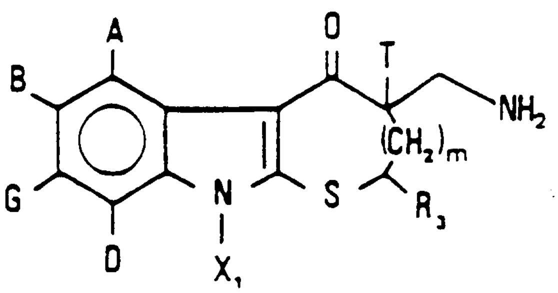

(I/C)

a particular case of the compounds of formula (I) in which A, B, G, D, $R_3$, T, m and $X_1$ are as defined above,

which is purified, if necessary, by chromatography and/or crystallisation and which, when in the compound of formula (I) that it is desired to obtain $R_1$ represents a grouping:

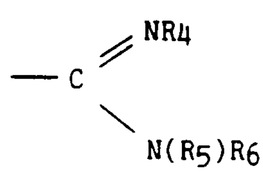

in which $R_4$, $R_5$ and $R_6$ are as defined in formula (I),
is treated with a salt of a strong acid of a compound of formula (VII):

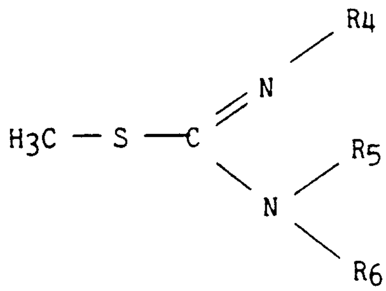

(VII)

in which $R_4$, $R_5$ and $R_6$ are as defined in formula (I),
to yield, after optional purification by chromatography, treatment with a heavy metal salt, and optional crystallisation, a compound of formula (I/D):

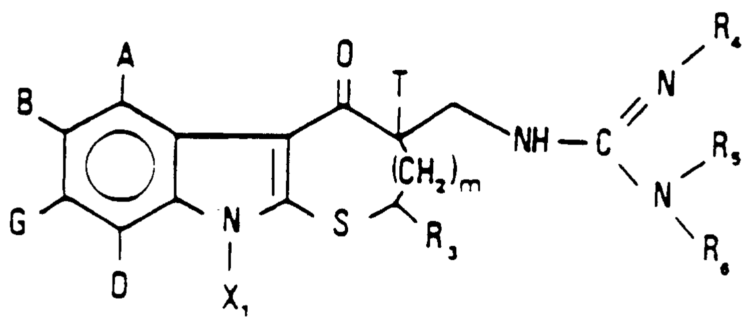

(I/D)

a particular case of the compound of formula (I) in which
$R_1$ represents a grouping:

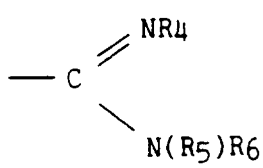

and $R_2$ represents a hydrogen atom,
and in which A, B, G, D, $R_3$, T, m and $X_1$ are as defined above,
which, in the case where $X_1$ represents a hydrogen atom may be subjected, according to the compound of formula (I) that it is desired to obtain, to the action of a compound of formula (VIII):

$Hal\text{-}SO_2E$ (VIII)

in which E is as defined in formula (I) and Hal represents a halogen atom, in the presence of sodium hydride, to yield, after extraction and optional purification by column chromatography, a compound of formula (I/E):

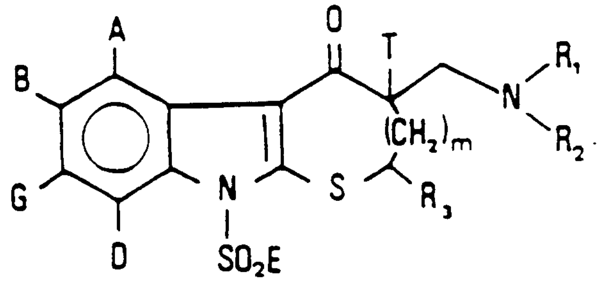

(I/E)

a particular case of the compounds of formula (I) in which X represents $SO_2E$ and in which A, B, G, D, $R_1$, $R_2$, $R_3$, T, m and E are as defined in formula (I),

which compound of formula (I/A), (I/B), (I/C1), (I/C), (I/D) or (I/E), in the case where, in the compound of formula (I) that it is desired to obtain, n represents 1, is treated with an oxidising agent, preferably metachloroperbenzoic acid, to yield, after optional purification by chromatography and/or crystallisation, the compound of formula (I/F):

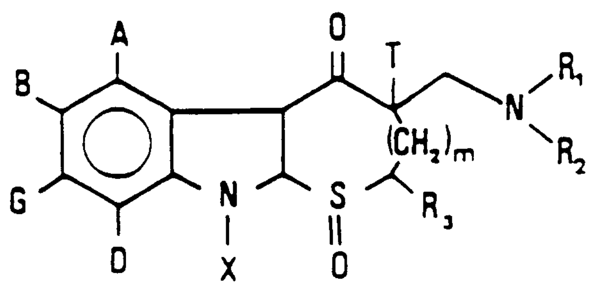

(I/F)

a particular case of the compounds of formula (I) in which A, B, G, D, X, T, $R_1$, $R_2$, $R_3$ and m are as defined in formula (I), n in this case representing 1,

which compound of formula (I/A), (I/B), (I/C1), (I/C), (I/D), (I/E) or (I/F) may, if desired, be separated, where appropriate, into its isomers and/or either converted into a salt to facilitate its purification for any later stages, or converted into a salt by means of a pharmaceutically acceptable acid and optionally crystallised for use for therapeutic purposes.

**2.** Process for the preparation according to claim 1 of the compounds of the general formula (1/B1):

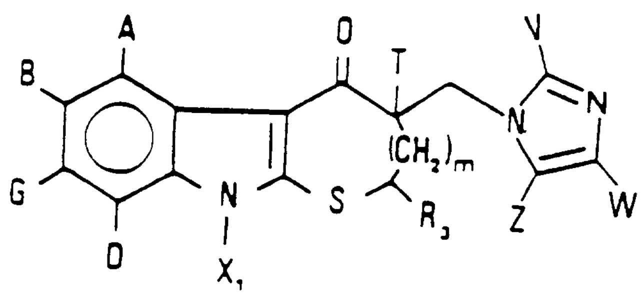

(I/B1)

in which:

A, B, G, D, T, $R_3$ and m are as defined in formula (I) according to claim 1,

$X_1$ represents a hydrogen atom or a lower alkyl grouping, and

each of V, W and Z, which may be identical or different, represents, independently of the others, a linear or branched lower alkyl or alkoxy grouping or a phenyl grouping which is itself optionally substituted by a lower alkyl or lower alkoxy grouping, a halogen atom or a trifluoromethyl grouping,

characterised in that a compound of formula (I/a):

a particular case of the compounds of formula (I):

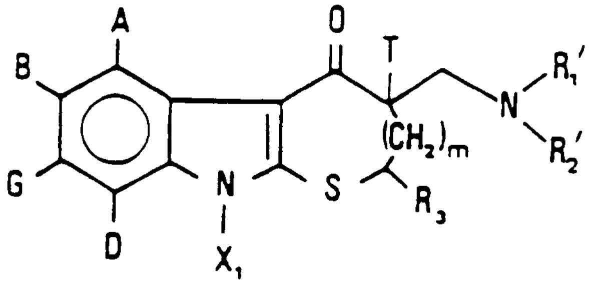

(I/A)

in which A, B, G, D, $R_3$, T, m and $X_1$ are as defined in claim 1 and $R'_1$ and $R'_2$ represent a lower alkyl grouping, which is purified, if necessary, by chromatography and/or crystallisation, is treated by a compound of formula (M):

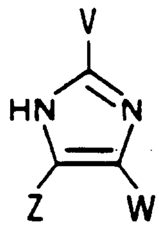

(M)

in which V, W and Z are as defined above, to give a compound of formula (I/B1) which, if desired, can be separated into its isomers and/or converted into a salt by means of a pharmaceutically acceptable acid and optionally crystallised.

**Claims for the following Contracting State : GR**

**1.** Process for the preparation of compounds of the general formula (I):

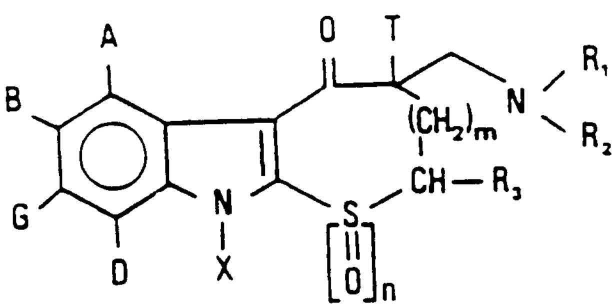

(I)

in which:
each of

| | |
|---|---|
| A, B, G and D, | which may be identical or different, represents a hydrogen atom or a halogen atom, a lower alkoxy grouping or a linear or branched lower alkyl grouping optionally substituted by one or more halogen atoms, |
| X | represents a hydrogen atom, a linear or branched lower alkyl grouping or a $SO_2E$ grouping in which E represents a linear or branched lower alkyl grouping or a phenyl grouping optionally substituted by a linear or branched lower alkyl grouping, |
| T | represents a hydrogen atom or a lower alkyl grouping, |
| $R_3$ | represents a hydrogen atom or a linear or branched lower alkyl grouping or a phenyl grouping optionally substituted by one or more linear or branched lower alkyl groupings, |

each of

$\underline{n}$ and $\underline{m}$ which may be identical or different, represents 0 or 1,

each of

$R_1$ and $R_2$, which may be identical or different, represents a hydrogen atom or a linear or branched lower alkyl grouping,

or

$R_1$ and $R_2$ form together with the nitrogen atom to which they are bonded a mono- or bi-cyclic, saturated or unsaturated nitrogen-containing heterocyclic system, each ring comprising 5 or 6 ring members and optionally containing one or two further hetero atoms selected from nitrogen, oxygen and sulphur, and being optionally substituted by one or more linear or branched lower alkyl or alkoxy groupings, or by an aryl grouping which is itself optionally substituted by one or more lower alkyl, lower alkoxy or trifluoromethyl groupings or one or more halogen atoms,

or

$R_1$ represents a grouping

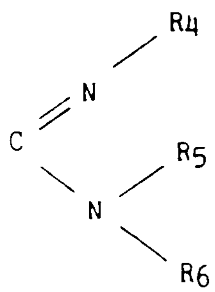

in which each of $R_4$, $R_5$ and $R_6$, which may be identical or different, represents a hydrogen atom or a linear or branched lower alkyl grouping or $R_4$ forms with $R_5$ a $(CH_2)_p$ bridge, $\underline{p}$ being an integer from 2 to 4,

or $R_1$ represents a 10,11-dihydro-[5H]-dibenzo[a,d]5-cycloheptenyl group and $R_2$ represents a hydrogen atom,

where appropriate, their isomers and also their addition salts with a pharmaceutically acceptable acid,

it being understood that lower alkyl and lower alkoxy groupings are to be understood as being groupings comprising from 1 to 6 carbon atoms, characterised in that a compound of formula (II):

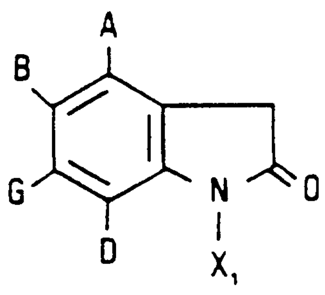

(II)

in which A, B, G and D are as defined in formula (I),

and $X_1$ represents a hydrogen atom or a lower alkyl grouping,

treated in the presence of a thionising agent, such as phosphorus pentasulphide, to obtain a compound of formula (III):

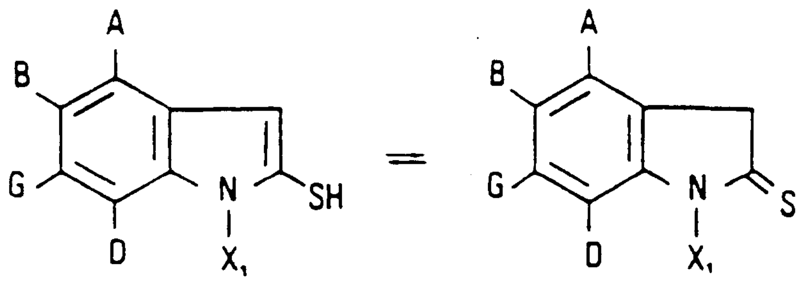

(III)

in which A, B, G, D and $X_1$ are as defined above, is condensed, treated with a compound of formula (IV):

$JCHR_3$-$(CH_2)_m$-CTK-COOR (IV)

in which:
either
m represents 0 and in that case J and K together represent a $\pi$ bond,
or
m represents 1 and in that case J represents a halogen atom and K represents a hydrogen atom,
R represents a hydrogen atom or a linear or branched lower alkyl grouping and T and $R_3$ are as defined in formula (I),

to obtain a compound of formula (V):

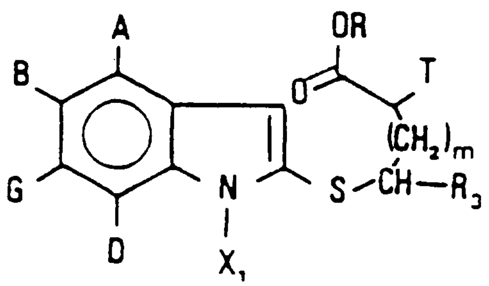

(V)

in which A, B, G, D, T, R, $R_3$, $X_1$ and m are as defined above,
which, when R is other than H, is subjected to the action of an alkaline agent to yield a compound of formula (Va), a particular case of the compounds of formula (V) in which R represents H,

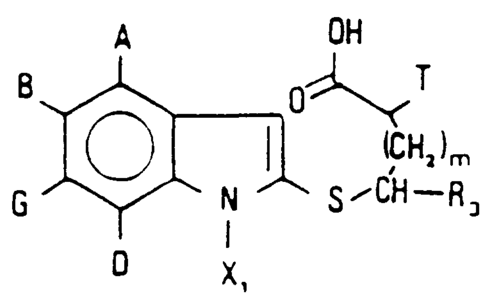

(Va)

which is subjected to the action of polyphosphate ester prepared preferably under a nitrogen atmosphere to yield a compound of formula (VI):

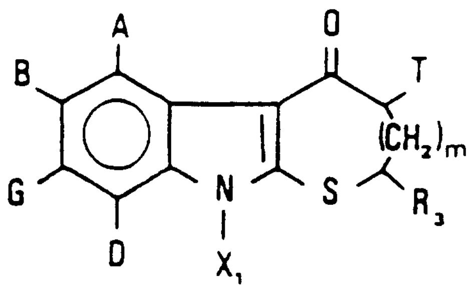

(VI)

in which A, B, G, D, T, m, $X_1$ and $R_3$ are as defined above,
which is then treated with paraformaldehyde, preferably under a nitrogen atmosphere,
in the presence of a dialkylamine,

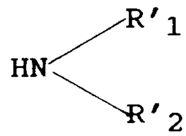

,

in which
$R'_1$ and $R'_2$ represent lower alkyl, or one of its salts with a strong acid,
to yield a compound of formula (I/A), a particular case of the compounds of formula (I):

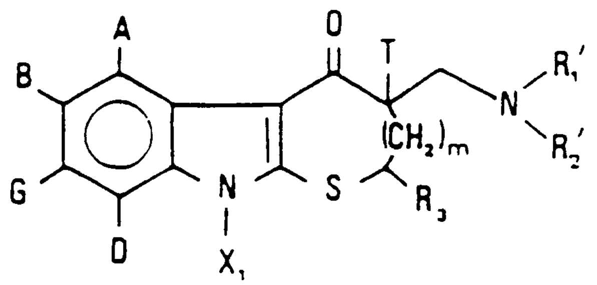

(I/A)

in which A, B, G, D, $R_3$, T, m and $X_1$ are as defined above and $R'_1$ and $R'_2$ represent a lower alkyl grouping, which is purified, if necessary, by chromatography and/or crystallisation and which

. when, in the compound of formula (I) that it is desired to obtain, $R_1$ and $R_2$ form with the nitrogen atom to which they are attached a heterocyclic system such as defined in formula (I), is then treated with a nitrogen-containing mono- or bi-cyclic, saturated or unsaturated heterocyclic compound, the nitrogen atom being bonded to a hydrogen atom, each ring comprising 5 or 6 ring members and optionally one or two further hetero atoms selected from nitrogen, oxygen and sulphur, and being optionally substituted by one or more linear or branched lower alkyl or alkoxy groupings or by an aryl grouping which is itself optionally substituted by one or more lower alkyl or lower alkoxy groupings, one or more halogen atoms or trifluoromethyl groupings to give a compound of formula (I/B):

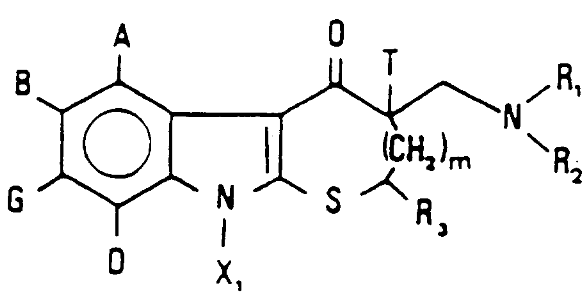

(I/B)

a particular case of the compounds of formula (I) in which A, B, G, D, $R_3$, T, m and $X_1$ are as defined above and $R_1$ and $R_2$ form with the nitrogen atom to which they are attached a heterocyclic system such as defined above,

. when each of $R_1$ and $R_2$ represents in the compound of formula (I) that it is desired to obtain a hydrogen atom, is treated with 10,11-dihydro-[5H]-dibenzo[a,d]5-cycloheptenylamineto yield, after purification by chromatography and crystallisation, a compound of formula (I/C1):

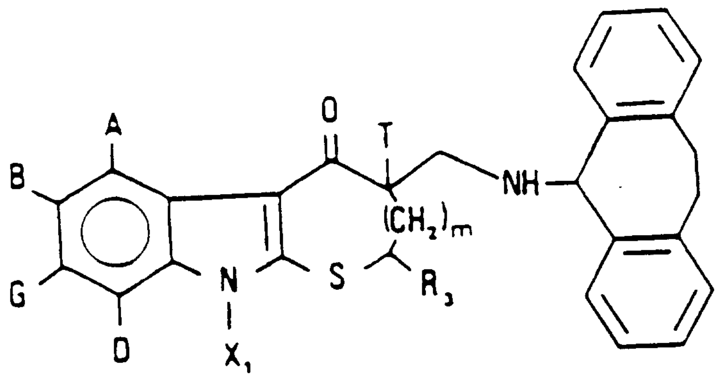

(I/C1)

a particular case of the formula (I) in which A, B, G, D, $R_3$, T, m and $X_1$ are as defined above, which is treated by heating in optionally diluted acetic acid to yield, after optional crystallisation, a compound of formula (I/C):

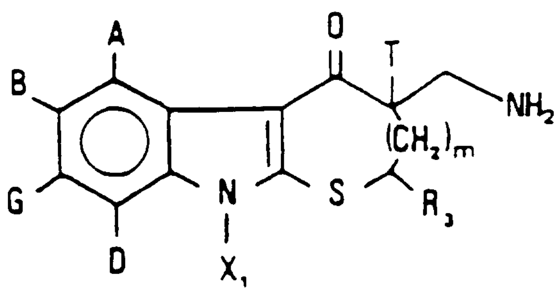

(I/C)

a particular case of the compounds of formula (I) in which A, B, G, D, $R_3$, T, m and $X_1$ are as defined above,

which is purified, if necessary, by chromatography and/or crystallisation and which, when in the compound of formula (I) that it is desired to obtain $R_1$ represents a grouping:

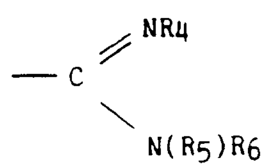

in which $R_4$, $R_5$ and $R_6$ are as defined in formula (I),

is treated with a salt of a strong acid of a compound of formula (VII):

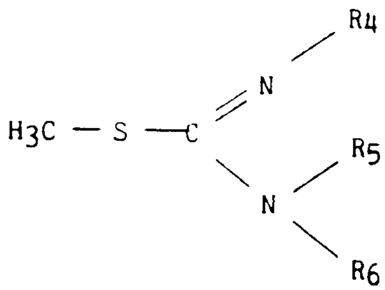

(VII)

in which $R_4$, $R_5$ and $R_6$ are as defined in formula (I), to yield, after optional purification by chromatography, treatment with a heavy metal salt, and optional crystallisation, a compound of formula (I/D):

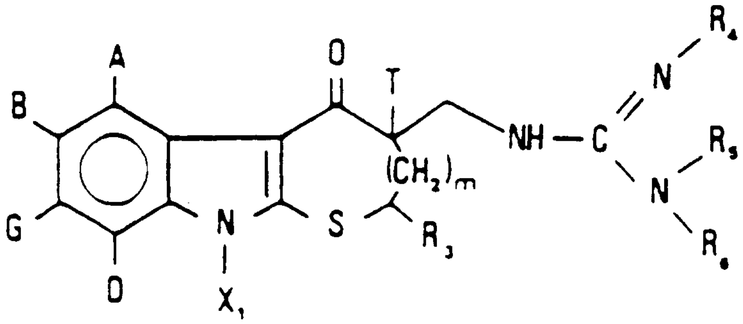

(I/D)

a particular case of the compound of formula (I) in which
$R_1$ represents a grouping:

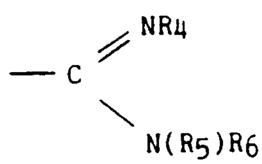

and $R_2$ represents a hydrogen atom,
and in which A, B, G, D, $R_3$, T, <u>m</u> and $X_1$ are as defined above,
which, in the case where $X_1$ represents a hydrogen atom may be subjected, according to the compound of formula (I) that it is desired to obtain, to the action of a compound of formula (VIII):

Hal-$SO_2$E (VIII)

in which E is as defined in formula (I) and Hal represents a halogen atom, in the presence of sodium hydride, to yield, after extraction and optional purification by column chromatography, a compound of formula (I/E):

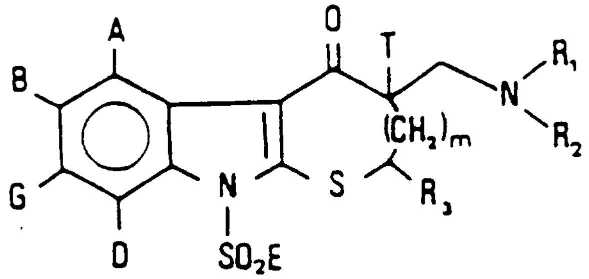

(I/E)

a particular case of the compounds of formula (I) in which X represents $SO_2E$ and in which A, B, G, D, $R_1$, $R_2$, $R_3$, T, m and E are as defined in formula (I),
which compound of formula (I/A), (I/B), (I/C1), (I/C), (I/D) or (I/E), in the case where, in the compound of formula (I) that it is desired to obtain, n represents 1, is treated with an oxidising agent, preferably metachloroperbenzoic acid, to yield, after optional purification by chromatography and/or crystallisation, the compound of formula (I/F):

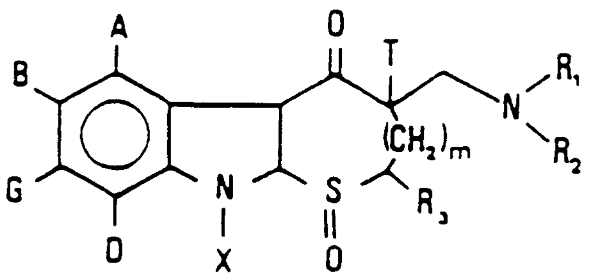

(I/F)

a particular case of the compounds of formula (I) in which A, B, G, D, X, T, $R_1$, $R_2$, $R_3$ and m are as defined in formula (I), n in this case representing 1,
which compound of formula (I/A), (I/B), (I/C1), (I/C), (I/D), (I/E) or (I/F) may, if desired, be separated, where appropriate, into its isomers and/or either converted into a salt to facilitate its purification for any later stages, or converted into a salt by means of a pharmaceutically acceptable acid and optionally crystallised for use for therapeutic purposes.

**2.** Process for the preparation according to claim 1 of the compounds of the general formula (1/B1):

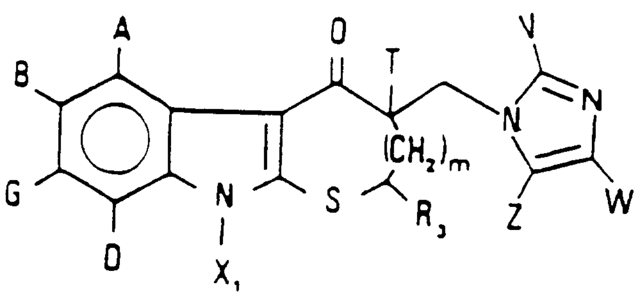

(I/B1)

in which:

A, B, G, D, T, $R_3$ and m are as defined in formula (I) according to claim 1,

$X_1$ represents a hydrogen atom or a lower alkyl grouping, and

each of V, W and Z, which may be identical or different, represents, independently of the others, a linear or branched lower alkyl or alkoxy grouping or a phenyl grouping which is itself optionally substituted by a lower alkyl or lower alkoxy grouping, a halogen atom or a trifluoromethyl grouping,
characterised in that a compound of formula (I/a):

a particular case of the compounds of formula (I):

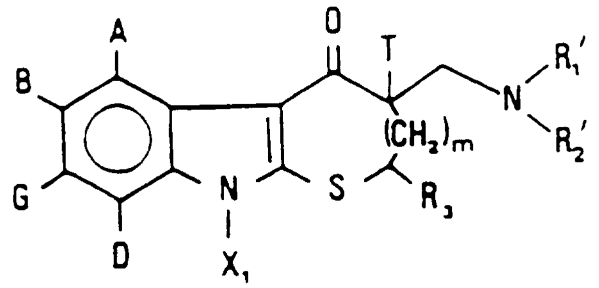

(I/A)

in which A, B, G, D, $R_3$, T, m and $X_1$ are as defined in claim 1 and $R'_1$ and $R'_2$ represent a lower alkyl grouping, which is purified, if necessary, by chromatography and/or crystallisation, is treated by a compound of formula (M):

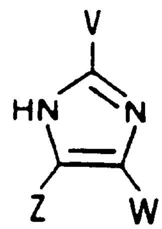

(M)

in which V, W and Z are as defined above, to give a compound of formula (I/B1) which, if desired, can be separated into its isomers and/or converted into a salt by means of a pharmaceutically acceptable acid and optionally crystallised.

**Patentansprüche**
**Patentansprüche für folgende Vertragsstaaten : AT, BE, CH, DE, FR, GB, IT, LI, LU, NL, SE**

**1.** Derivate der Formel (I):

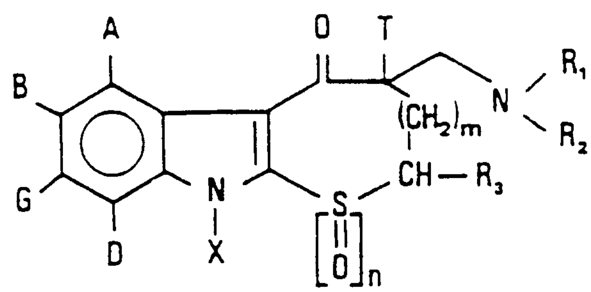

(I)

in der

| | |
|---|---|
| A, B, G, D, | die gleichartig oder verschieden sein können, jeweils ein Wasserstoffatom oder ein Halogenatom, eine Niedrigalkoxygruppe oder eine geradkettige oder verzweigte Niedrigalkylgruppe, die gegebenenfalls durch eines oder mehrere Halogenatome substituiert ist, |
| X | ein Wasserstoffatom, eine geradkettige oder verzweigte Niedrigalkylgruppe oder eine Gruppe $SO_2E$, worin E eine geradkettige oder verzweigte Niedrigalkylgruppe oder Phenylgruppe, die gegebenenfalls durch eine geradkettige oder verzweigte Niedrigalkylgruppe substituiert ist, darstellt, |
| T | ein Wasserstoffatom oder eine Niedrigalkylgruppe, |
| $R_3$ | ein Wasserstoffatom oder eine geradkettige oder verzweigte Niedrigalkylgruppe oder |

eine Phenylgruppe, die gegebenenfalls durch eine oder mehrere geradkettige oder verzweigte Niedrigalkylgruppen substituiert ist,

n und m, die gleichartig oder verschieden sein können, jeweils 0 oder 1,

$R_1$ und $R_2$, die gleichartig oder verschieden sein können, jeweils ein Wasserstoffatom oder eine geradkettige oder verzweigte Niedrigalkylgruppe

oder

$R_1$ und $R_2$ gemeinsam mit dem Stickstoffatom, an das sie gebunden sein, ein mono- oder bicyclisches, gesättigtes oder ungesättigtes stickstoffhaltiges heterocyclisches System, wobei jeder Ring sechs oder sieben Ringglieder aufweist und gegebenenfalls eines oder zwei weitere Heteroatome, ausgewählt aus Stickstoff-, Sauerstoff- und Schwefelatomen, umfaßt und gegebenenfalls durch eine oder mehrere geradkettige oder verzweigte Niedrigalkyl- oder Niedrigalkoxgruppen oder durch eine ihrerseits gegebenenfalls durch eine oder mehrere Niedrigalkylgruppen, Niedrigalkoxygruppen, Trifluormethylgrupppen oder eines oder mehrere Halogenatome substituierte Arylgruppe substituiert ist,

oder

$R_1$ eine Gruppe

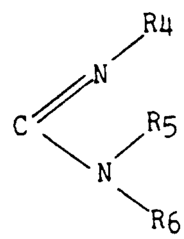

in der $R_4$, $R_5$ und $R_6$, die gleichartig oder verschieden sein können, jeweils ein Wasserstoffatom oder eine geradkettige oder verzweigte Niedrigalkylgruppe darstellen oder $R_4$ zusammen mit $R_5$ eine Brücke $(CH_2)_p$ darstellt, worin p zwischen 2 und 4 liegt,

oder $R_1$ eine 10,11-Dihydro[5H]dibenzo[a,b]cyclohepten-5-yl-gruppe und $R_2$ ein Wasserstoffatom bedeuten,

sowie gegebenenfalls deren Isomeren und deren Additionssalze mit einer pharmazeutisch annehmbaren Säure,

wobei unter Niedrigalkylgruppen oder Niedrigalkoxygruppen Gruppen mit 1 bis 6 Kohlenstoffatomen zu verstehen sind.

**2.** Verbindungen nach Anspruch 1, bei denen n 0, X eine geradkettige oder verzweigte Niedrigalkylgruppe und $R_1$ und $R_2$ gemeinsam mit dem Stickstoffatom, an das sie gebunden sind, einen Imidazolring bedeuten, der gegebenenfalls durch eine oder mehrere geradkettige oder verzweigte Niedrigalkyl- oder Niedrigalkoxygruppen oder durch eine ihrerseits gegebenenfalls durch eine Niedrigalkylgruppe oder Niedrigalkoxygruppe, Trifluormethylgruppe oder ein Halogenatom substituierte Arylgruppe substituiert ist, sowie deren Isomeren und deren Additionssalze mit einer pharmazeutisch annehmbaren Säure.

**3.** Verbindungen nach einem der Ansprüche 1 oder 2, worin $R_1$ und $R_2$ zusammen mit dem Stickstoffatom, an das sie gebunden sind, eine 2-Methyl-imidazolgruppe bedeuten.

**4.** Verbindungen nach einem der Ansprüche 1 oder 3, nämlich 3-[(2-Methyl-imidazol-1-yl)-methyl]-4-oxo-9-methyl-2,3,4,9-tetrahydro-thiopyrano[2,3-b]in dol sowie dessen Additionssalze mit einer pharmazeutisch annehmbaren Säure.

**5.** Verbindungen nach einem der Ansprüche 1 oder 3, nämlich 4-[(2-Methyl-imidazol-1-yl)-methyl]-5-oxo-10-methyl-2,3,4,5,10-pentahydro-thiepino[2,3-b]indol sowie dessen Additionssalze mit einer pharmazeutisch annehmbaren Säure.

**6.** Verfahren zur Synthese der Derivate der Formel (I), **dadurch gekennzeichnet**, daß man ein Derivat der Formel (II):

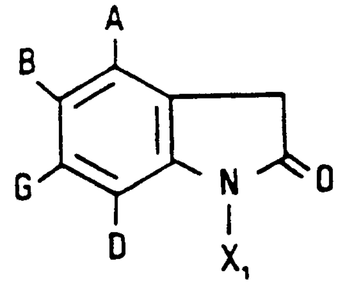

(II)

in der A, B, G und D die bezuglich der Formel (I) angegebenen Bedeutungen besitzen und $X_1$ ein Wasserstoffatom oder eine Niedrigalkylgruppe bedeutet, in Gegenwart eines Thionierungsmittels, wie Phosphorpentasulfid, kondensiert, so daß man ein Derivat der Formel (III):

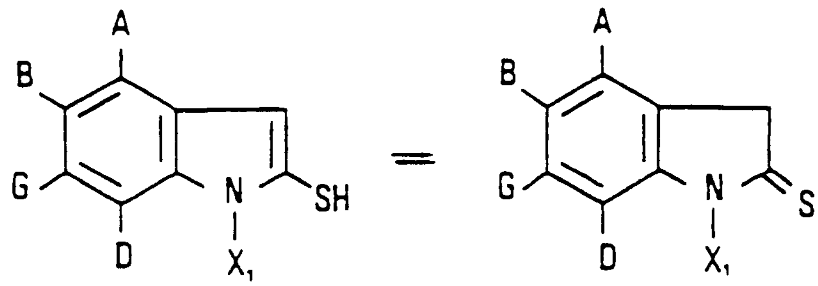

(III)

erhält, in der A, B, G, D und $X_1$ die oben angegebenen Bedeutungen besitzen, welches man mit einem Derivat der Formel (IV):

$JCHR_3$-$(CH_2)_m$-CTK-COOR (IV)

in der
entweder m 0 und in diesem Fall J und K gemeinsam eine $\pi$-Bindung,
oder

- m 1 und in diesem Fall J ein Halogenatom und K ein Wasserstoffatom und
- R ein Wasserstoffatom oder eine geradkettige oder verzweigte Niedrigalkylgruppe bedeuten und T und $R_3$ die bezuglich der Formel (I) angegebenen Bedeutungen besitzen,

behandelt, so daß man ein Derivat der Formel (V):

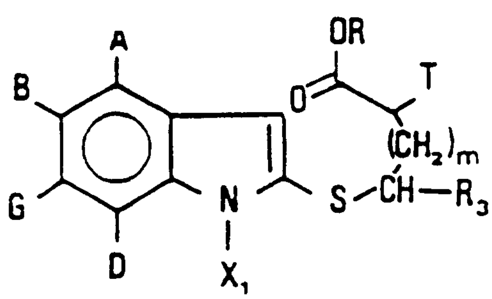

(V)

erhält, in der A, B, G, D, T, R, $R_3$, $X_1$ und m die oben angegebenen Bedeutungen besitzen,
welches, wenn R von H verschieden ist, der Einwirkung eines alkalischen Mittels unterworfen wird, so daß man ein Derivat der Formel (Va) erhält, einem Sonderfall der Derivate der Formel (V), worin R H bedeutet,

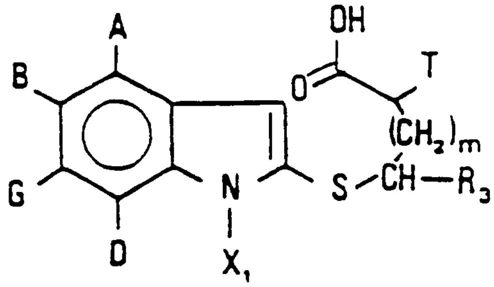

(Va)

welches man der Einwirkung eines vorzugsweise unter einer Stickstoffatmosphäre hergestellten Polyphosphatesters unterwirft, so daß man ein Derivat der Formel (VI) erhält:

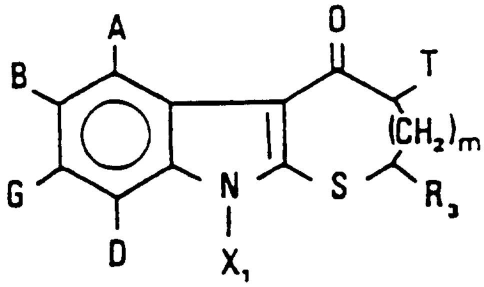

(VI)

in der A, B, G, D, T, m, $X_1$ und $R_3$ die oben angegebenen Bedeutungen besitzen, welches man anschließend vorzugsweise unter einer Stickstoffatomosphäre in Gegenwart eines Dialkylamins der Formel

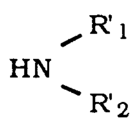

in der $R'_1$ und $R'_2$ Niedrigalkylgruppen bedeuten, oder eines seiner Salze mit einer starken Säure mit Paraformaldehyd behandelt,
so daß man ein Derivat der Formel (I/A) erhält, einem Sonderfall der Derivate der Formel (I):

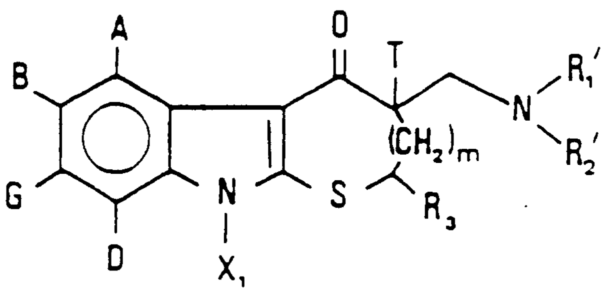

(I/A)

in der A, B, G, D, $R_3$, T, m und $X_1$ die oben angegebenen Bedeutungen besitzen und $R'_1$ und $R'_2$ Niedrigalkylgruppen bedeuten, welches man erforderlichenfalls durch Chromatographie und/oder Kristallisation reinigt, welches man

- wenn in dem Derivat der Formel (I), welches hergestellt werden soll, $R_1$ und $R_2$ zusammen mit dem Stickstoffatm, an das sie gebunden sind, ein heterocyclisches System bilden, wie es bezüglich der Formel (I) definiert ist, anschließend mit einer mono- oder bicyclischen, gesättigten oder ungesättigten stickstoffhaltigen heterocyclischen Verbindung, an dessen Stickstoffatom ein Wasserstoffatom gebunden ist, wobei jeder Ring fünf oder sechs Ringglieder und gegebenenfalls

eines oder zwei weitere Heteroatome, die aus Stickstoff-, Sauerstoff- oder Schwefelatomen ausgewählt sind, aufweist und gegebenenfalls durch eine oder mehrere geradkettige oder verzweigte Niedrigalkyl- oder Niedrigalkoxygruppen oder eine ihrerseits gegebenenfalls durch eine oder mehrere Niedrigalkylgruppen, Niedrigalkoxygruppen oder einem oder mehrere Halogenatome oder Trifluormethylgruppen substituierte Arylgruppe substituiert ist, behandelt, so daß man eine Verbindung der Formel (I/B) erhält:

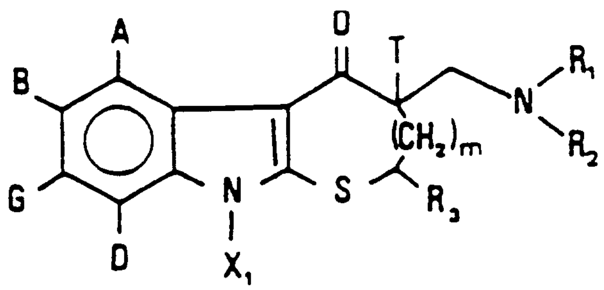

(I/B)

einem Sonderfall der Derivate der Formel (I), in der A, B, G, D, $R_3$, T, m und $X_1$ die oben angegebenen Bedeutungen besitzen und $R_1$ und $R_2$ zusammen mit dem Stickstoffatom, an das sie gebunden sind, ein heterocyclisches System bilden, wie es oben definiert ist,

- dann, wenn $R_1$ und $R_2$ in dem Derivat der Formel (I), welches hergestellt werden soll, jeweils ein Wasserstoffatom bedeuten, mit 10,11-Dihydro-[5H]-dibenzo[a,b]cyclohepten-5-yl-amin behandelt, so daß man nach der Reinigung durch Chromatographie und Kristallisation ein Derivat der Formel (I/C1) erhält:

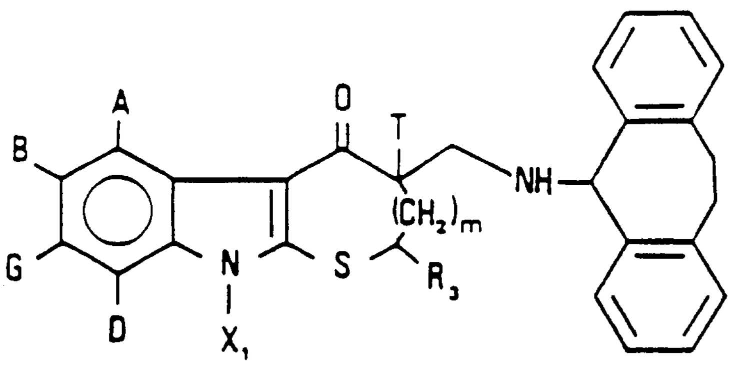

(I/C1)

einem Sonderfall der Formel (I), in der A, B, G, D, $R_3$, T, m und $X_1$ die oben angegebenen Bedeutungen besitzen, welches man in gegebenenfalls verdünnter Essigsäure erhitzt, so daß man nach der eventuellen Kristallisation ein Derivat der Formel (I/C) erhält:

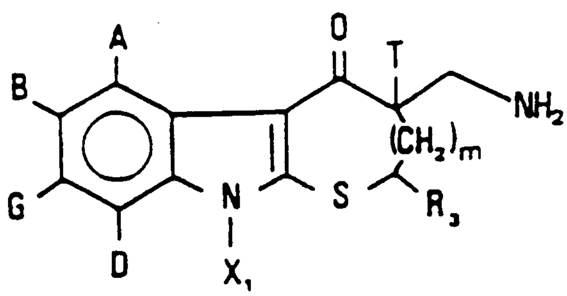

(I/C)

einem Sonderfall der Derivate der Formel (I), in der A, B, G, D, $R_3$, T, m und $X_1$ die oben angegebenen Bedeutungen besitzen,

welches man erforderlichenfalls durch Chromatographie und/oder Kristallisation reinigt und welches dann, wenn $R_1$ in dem Derivat der Formel (I), welches hergestellt werden soll, eine Gruppe der Formel:

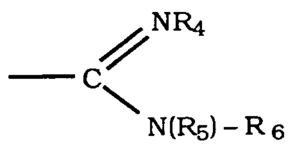

darstellt, in der $R_4$, $R_5$ und $R_6$ die bezuglich der Formel (I) angegebenen Bedeutungen besitzen, mit einem Salz eines Derivats der Formel (VII):

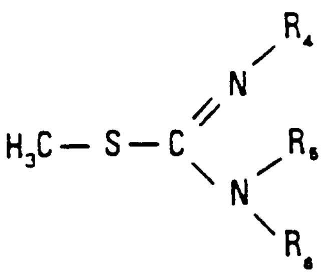

(VII)

in der $R_4$, $R_5$ und $R_6$ die bezuglich der Formel (I) angegebenen Bedeutungen besitzen, mit einer starken Säure behandelt,
so daß man nach der eventuellen Reinigung durch Chromatographie, der Behandlung mit einem Schwermetallsalz und gegebenenfalls der Kristallisation ein Derivat der Formel (I/D) erhält:

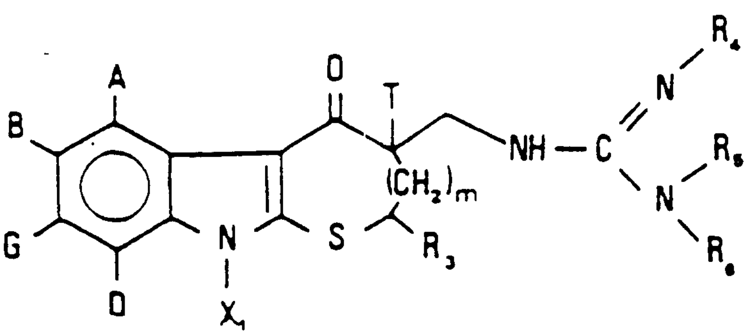

(I/D)

einem Sonderfall der Derivate der Formel (I), in der $R_1$ eine Gruppe der Formel:

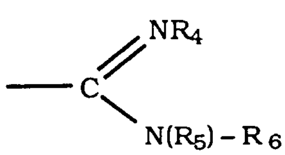

und $R_2$ ein Wasserstoffatom bedeuten,
und worin A, B, G, D, $R_3$, T, m und $X_1$ die oben angegebenen Bedeutungen besit zen, welches dann, wenn $X_1$ ein Wasserstoffatom darstellt, in Abhängigkeit von der Verbindung der Formel (I), die hergestellt werden soll, der Einwirkung einer Verbindung der Formel (VIII):

Hal-$SO_2$E (VIII)

in der E die bezüglich der Formel (I) angegebenen Bedeutungen besitzt und Hal ein Halogenatom darstellt, in Gegenwart von Natriumhydrid unterworfen werden kann, so daß man nach der Extraktion und der eventuellen Reinigung durch Säulenchromatographie ein Derivat der Formel (I/E) erhält:

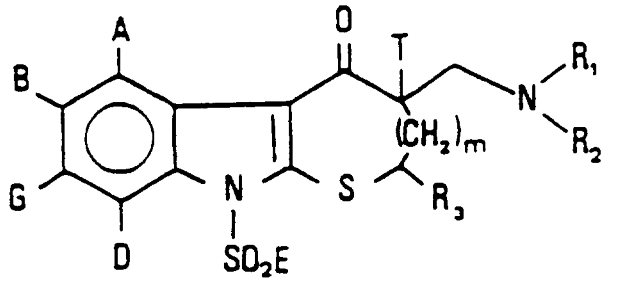

(I/E)

einem Sonderfall der Derivate der Formel (I), in der X eine Gruppe der Formel $SO_2E$ darstellt und A, B, G, D, $R_1$, $R_2$, $R_3$, T, m und E die bezüglich der Formel (I) angegebenen Bedeutungen besitzen,
welches Derivat der Formel (I/A), (I/B), (I/C1), (I/C), (I/D) oder (I/E) dann, wenn n in der Verbindung der Formel (I), die hergestellt werden soll, 1 bedeutet, mit einem Oxidationsmittel, vorzugsweise m-Chlorperbenzoesäure, behandelt wird, so daß man nach der eventuellen Reinigung durch Chromatographie und/oder Kristallisation ein Derivat der Formel (I/F) erhält:

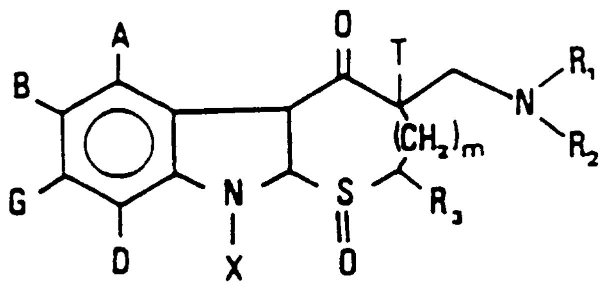

(I/F)

einem Sonderfall der Derivate der Formel (I) in der A, B, G, D, X, T, $R_1$, $R_2$, $R_3$ und m die bezüglich der Formel (I) angegebenen Bedeutungen besitzen und n 1 bedeutet,
welches Derivat der Formel (I/A), (I/B), (I/C1), (I/C), (I/D), (I/E) oder (I/F) gewünschtenfalls in seine Isomeren aufgetrennt und/oder in ein Salz überführt werden kann zur Erleichterung der Reinigung bei den eventuellen späteren Stufen, oder auch mit einer pharmazeutisch annehmbaren Säure in Salz überführt werden und gegebenenfalls für die therapeutische Anwendung kristallisiert werden kann.

7. Verfahren nach Anspruch 6 zur Herstellung der Verbindungen nach einem der Ansprüche 2 oder 3, **dadurch gekennzeichnet**, daß man eine Verbindung der Formel (I/A):

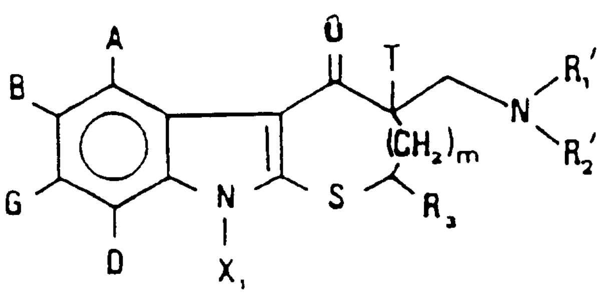

(I/A)

einem Sonderfall der Derivate der Formel (I), in der A, B, G, D, $R_3$, T, m und $X_1$ die in Anspruch 6 angegebenen Bedeutungen besitzen und $R'_1$ und $R'_2$ Niedrigalkylgruppen bedeuten, welches man

erforderlichenfalls durch Chromatographie und/oder Kristallisation gereinigt hat, mit einem Derivat der Formel (M):

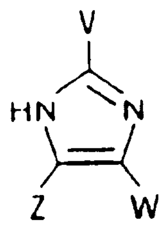

(M)

in der V, W, Z, die gleichartig oder verschieden sein können, jeweils unabhängig voneinander geradkettige oder verzweigte Niedrigalkyl- oder Niedrigalkoxygruppen oder eine ihrerseits gegebenenfalls durch eine Niedrigalkylgruppe, eine Niedrigalkoxygruppe, ein Halogenatom oder eine Trifluormethylgruppe substituierte Phenylgruppe bedeuten, behandelt, so daß man eine Verbindung der Formel (I/B1) erhält:

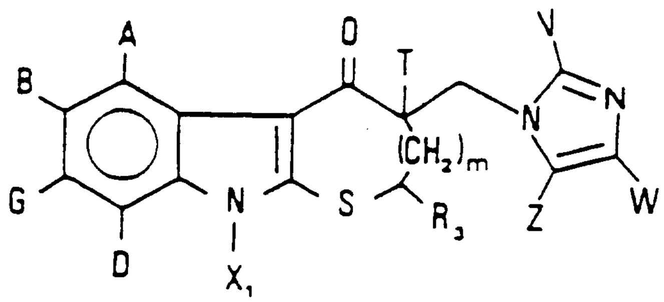

(I/B1)

in der A, B, G, D, T, $R_3$ und m die bezüglich der Formel (I) angegebenen Bedeutungen besitzen und $X_1$, V, W und Z die oben angegebenen Bedeutungen besitzen, welches man gewünschtenfalls in seine Isomeren auftrennen oder mit einer pharmazeutisch annehmbaren Säure in ein Salz überführen und gegebenenfalls kristallisieren kann.

**8.** Pharmazeutische Zubereitung enthaltend als Wirkstoff mindestens eine Verbindung nach einem der Ansprüche 1 bis 5 in Kombination mit einem oder mehreren inerten, nichttoxischen, pharmazeutisch annehmbaren Bindemitteln oder Trägermaterialien.

**9.** Pharmazeutische Zubereitung nach Anspruch 8 zur Behandlung von Störungen, die mit einer Fehlfunktion des serotoninergischen Systems verbunden sind und insbesondere von Schmerzzuständen, Angstzuständen, der Schizophrenie, des Erbrechens und insbesondere von Störungen, die die Folge einer Antikrebs-Behandlung sind, Störungen des Herzrhythmus, bestimmten Magen-Darm-Störungen sowie von Bewußtseinsstörungen und insbesondere Wahnzuständen und Gedächtnisstörungen.

**Patentansprüche für folgenden Vertragsstaat : ES**

1. Verfahren zur Herstellung von Verbindungen der allgemeinen Formel (I):

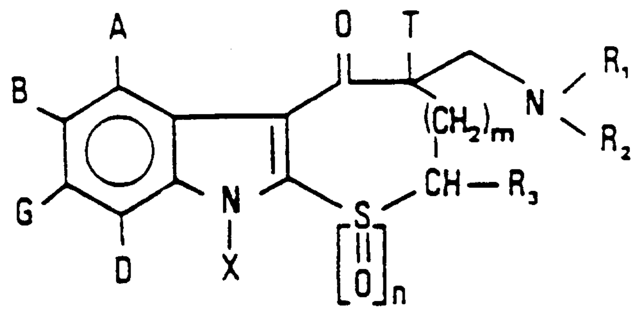

(I)

in der

A, B, G, D, die gleichartig oder verschieden sein können, jeweils ein Wasserstoffatom oder ein Halogenatom, eine Niedrigalkoxygruppe oder eine geradkettige oder verzweigte Niedrigalkylgruppe, die gegebenenfalls durch eines oder mehrere Halogenatome substituiert ist,

X ein Wasserstoffatom, eine geradkettige oder verzweigte Niedrigalkylgruppe oder eine Gruppe $SO_2E$, worin E eine geradkettige oder verzweigte Niedrigalkylgruppe oder Phenylgruppe, die gegebenenfalls durch eine geradkettige oder verzweigte Niedrigalkylgruppe substituiert ist, darstellt,

T ein Wasserstoffatom oder eine Niedrigalkylgruppe,

$R_3$ ein Wasserstoffatom oder eine geradkettige oder verzweigte Niedrigalkylgruppe oder eine Phenylgruppe, die gegebenenfalls durch eine oder mehrere geradkettige oder verzweigte Niedrigalkylgruppen substituiert ist,

n und m, die gleichartig oder verschieden sein können, jeweils 0 oder 1,

$R_1$ und $R_2$, die gleichartig oder verschieden sein können, jeweils ein Wasserstoffatom oder eine geradkettige oder verzweigte Niedrigalkylgruppe

oder

$R_1$ und $R_2$ gemeinsam mit dem Stickstoffatom, an das sie gebunden sein, ein mono- oder bicyclisches, gesättigtes oder ungesättigtes stickstoffhaltiges heterocyclisches System, wobei jeder Ring sechs oder sieben Ringglieder aufweist und gegebenenfalls eines oder zwei weitere Heteroatome, ausgewählt aus Stickstoff-, Sauerstoff- und Schwefelatomen, umfaßt und gegebenenfalls durch eine oder mehrere geradkettige oder verzweigte Niedrigalkyl- oder Niedrigalkoxygruppen oder durch eine ihrerseits gegebenenfalls durch eine oder mehrere Niedrigalkylgruppen, Niedrigalkoxygruppen, Trifluormethylgruppen oder eines oder mehrere Halogenatome substituierte Arylgruppe substituiert ist,

oder

$R_1$ eine Gruppe

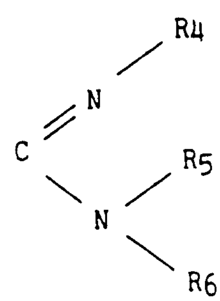

in der $R_4$, $R_5$ und $R_6$, die gleichartig oder verschieden sein können, jeweils ein Wasserstoffatom oder eine geradkettige oder verzweigte Niedrigalkylgruppe darstellen oder $R_4$ zusammen mit $R_5$ eine Brücke $(CH_2)_p$ darstellt, worin p zwischen 2 und 4 liegt,
oder $R_1$ eine 10,11-Dihydro[5H]dibenzo[a,b]cyclohepten-5-yl-gruppe und $R_2$ ein Wasserstoffatom bedeuten,

und gegebenenfalls von deren Isomeren sowie deren Additionssalzen mit einer pharmazeutisch annehmbaren Säure,
wobei unter Niedrigalkylgruppen oder Niedrigalkoxygruppen Gruppen mit 1 bis 6 Kohlenstoffatomen zu verstehen sind, **dadurch gekennzeichnet**, daß man ein Derivat der Formel (II):

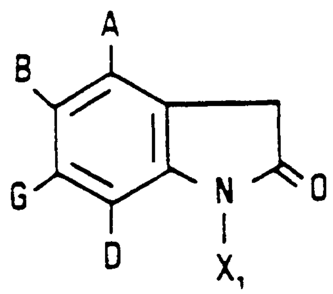

(II)

in der A, B, G und D die bezuglich der Formel (I) angegebenen Bedeutungen besitzen und
$X_1$ ein Wasserstoffatom oder eine Niedrigalkylgruppe bedeutet, in Gegenwart eines Thionierungsmittels, wie Phosphorpentasulfid, kondensiert, so daß man ein Derivat der Formel (III):

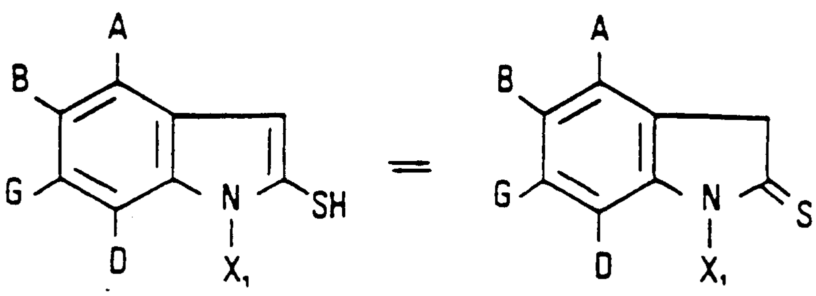

(III)

erhält, in der A, B, G, D und $X_1$ die oben angegebenen Bedeutungen besitzen, welches man mit einem Derivat der Formel (IV):

$JCHR_3\text{-}(CH_2)_m\text{-}CTK\text{-}COOR$ (IV)

in der
entweder m 0 und in diesem Fall J und K gemeinsam eine $\pi$-Bindung,
oder

m 1 und in diesem Fall J ein Halogenatom und K ein Wasserstoffatom und

R ein Wasserstoffatom oder eine geradkettige oder verzweigte Niedrigalkylgruppe bedeuten und T und $R_3$ die bezüglich der Formel (I) angegebenen Bedeutungen besitzen,

behandelt, so daß man ein Derivat der Formel (V):

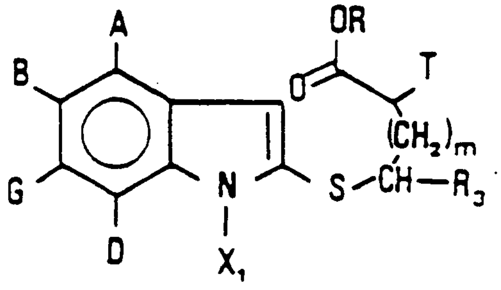

(V)

erhält, in der A, B, G, D, T, R, $R_3$, $X_1$ und m die oben angegebenen Bedeutungen besitzen,
welches, wenn R von H verschieden ist, der Einwirkung eines alkalischen Mittels unterworfen wird, so daß man ein Derivat der Formel (Va) erhält, einem Sonderfall der Derivate der Formel (V), worin R H bedeutet,

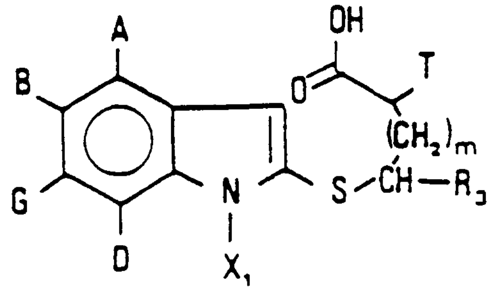

(Va)

welches man der Einwirkung eines vorzugsweise unter einer Stickstoffatmosphäre hergestellten Polyphosphatesters unterwirft, so daß man ein Derivat der Formel (VI) erhält:

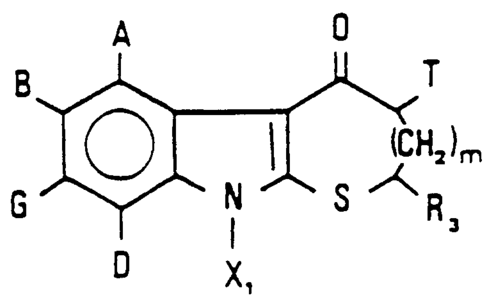

(VI)

in der A, B, G, D, T, m, $X_1$ und $R_3$ die oben angegebenen Bedeutungen besitzen, welches man anschließend vorzugsweise unter einer Stickstoffatomosphäre in Gegenwart eines Dialkylamins der Formel

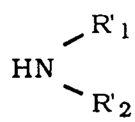

in der $R'_1$ und $R'_2$ Niedrigalkylgruppen bedeuten, oder eines seiner Salze mit einer starken Säure mit Paraformaldehyd behandelt,
so daß man ein Derivat der Formel (I/A) erhält, einem Sonderfall der Derivate der Formel (I):

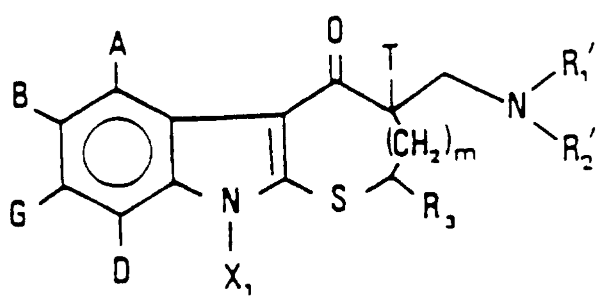

(I/A)

in der A, B, G, D, $R_3$, T, m und $X_1$ die oben angegebenen Bedeutungen besitzen und $R'_1$ und $R'_2$ Niedrigalkylgruppen bedeuten, welches man erforderlichenfalls durch Chromatographie und/oder Kristallisation reinigt, welches man

- wenn in dem Derivat der Formel (I), welches hergestellt werden soll, $R_1$ und $R_2$ zusammen mit dem Stickstoffatm, an das sie gebunden sind, ein heterocyclisches System bilden, wie es bezüglich der Formel (I) definiert ist, anschließend mit einer mono- oder bicyclischen, gesättigten oder ungesättigten stickstoffhaltigen heterocyclischen Verbindung, an dessen Stickstoffatom ein Wasserstoffatom gebunden ist, wobei jeder Ring fünf oder sechs Ringglieder und gegebenenfalls eines oder zwei weitere Heteroatome, die aus Stickstoff-, Sauerstoff- oder Schwefelatomen ausgewählt sind, aufweist und gegebenenfalls durch eine oder mehrere geradkettige oder verzweigte Niedrigalkyl- oder Niedrigalkoxygruppen oder eine ihrerseits gegebenenfalls durch eine oder mehrere Niedrigalkylgruppen, Niedrigalkoxygruppen oder einem oder mehrere Halogenatome oder Trifluormethylgruppen substituierte Arylgruppe substituiert ist, behandelt, so daß man eine Verbindung der Formel (I/B) erhält:

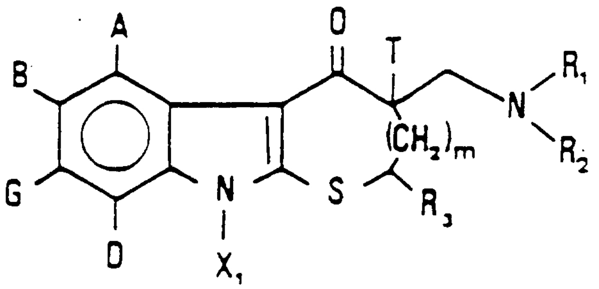

(I/B)

einem Sonderfall der Derivate der Formel (I), in der A, B, G, D, $R_3$, T, m und $X_1$ die oben angegebenen Bedeutungen besitzen und $R_1$ und $R_2$ zusammen mit dem Stickstoffatom, an das sie gebunden sind, ein heterocyclisches System bilden, wie es oben definiert ist,

- dann, wenn $R_1$ und $R_2$ in dem Derivat der Formel (I) welches hergestellt werden soll, jeweils ein Wasserstoffatom bedeuten, mit 10,11-Dihydro-[5H]-dibenzo[a,b]cyclohepten-5-yl-amin behandelt, so daß man nach der Reinigung durch Chromatographie und Kristallisation ein Derivat der Formel (I/C1) erhält:

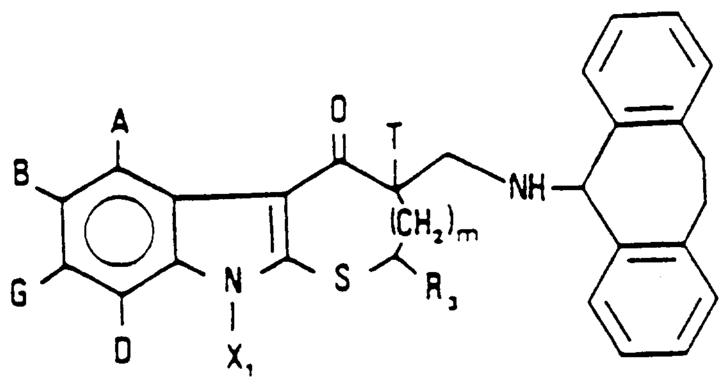

(I/C1)

einem Sonderfall der Formel (I), in der A, B, G, D, $R_3$, T, m und $X_1$ die oben angegebenen Bedeutungen besitzen, welches man in gegebenenfalls verdünnter Essigsäure erhitzt, so daß man nach der eventuellen Kristallisation ein Derivat der Formel (I/C) erhält:

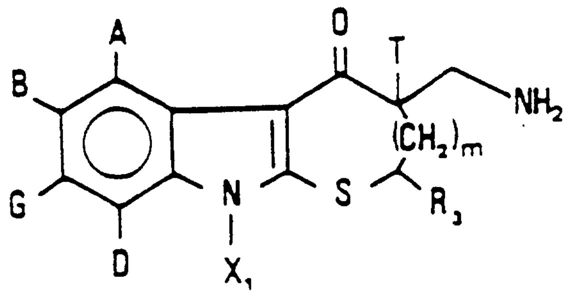

(I/C)

einem Sonderfall der Derivate der Formel (I), in der A, B, G, D, $R_3$, T, m und $X_1$ die oben angegebenen Bedeutungen besitzen,
welches man erforderlichenfalls durch Chromatographie und/oder Kristallisation reinigt und welches dann, wenn $R_1$ in dem Derivat der Formel (I), welches hergestellt werden soll, eine Gruppe der Formel:

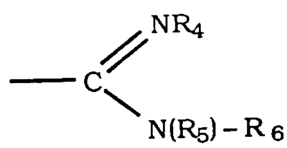

darstellt, in der $R_4$, $R_5$ und $R_6$ die bezuglich der Formel (I) angegebenen Bedeutungen besitzen, mit einem Salz eines Derivats der Formel (VII):

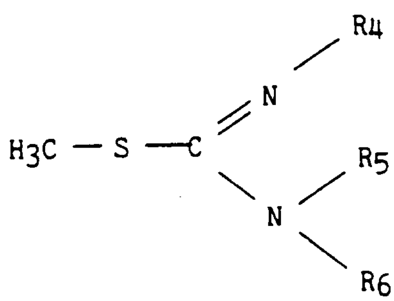

(VII)

in der $R_4$, $R_5$ und $R_6$ die bezuglich der Formel (I) angegebenen Bedeutungen besitzen, mit einer starken Säure behandelt,
so daß man nach der eventuellen Reinigung durch Chromatographie, der Behandlung mit einem Schwermetallsalz und gegebenenfalls der Kristallisation ein Derivat der Formel (I/D) erhält:

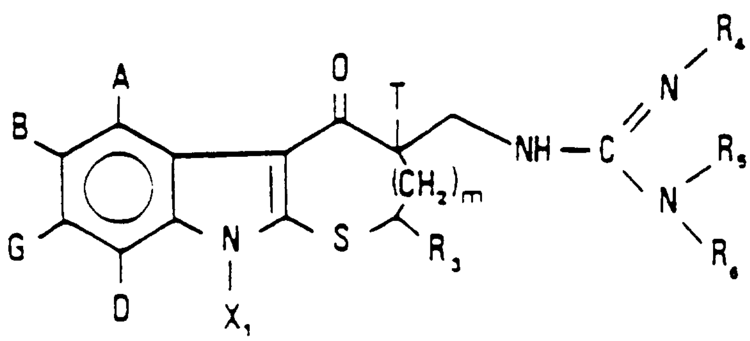

(I/D)

einem Sonderfall der Derivate der Formel (I), in der $R_1$ eine Gruppe der Formel:

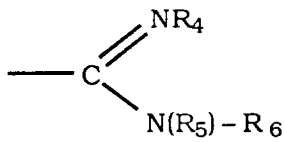

und $R_2$ ein Wasserstoffatom bedeuten,
und worin A, B, G, D, $R_3$, T, m und $X_1$ die oben angegebenen Bedeutungen besitzen,
welches dann, wenn $X_1$ ein Wasserstoffatom darstellt, in Abhängigkeit von der Verbindung der Formel (I), die hergestellt werden soll, der Einwirkung einer Verbindung der Formel (VIII):

Hal-$SO_2$E (VIII)

in der E die bezüglich der Formel (I) angegebenen Bedeutungen besitzt und Hal ein Halogenatom darstellt, in Gegenwart von Natriumhydrid unterworfen werden kann, so daß man nach der Extraktion und der eventuellen Reinigung durch Säulenchromatographie ein Derivat der Formel (I/E) erhält:

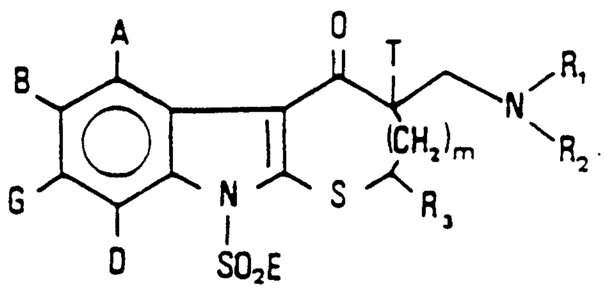

(I/E)

einem Sonderfall der Derivate der Formel (I), in der X eine Gruppe der Formel $SO_2$E darstellt und A, B, G, D, $R_1$, $R_2$, $R_3$, T, m und E die bezuglich der Formel (I) angegebenen Bedeutungen besitzen,
welches Derivat der Formel (I/A), (I/B), (I/C1), (I/C), (I/D) oder (I/E) dann, wenn n in der Verbindung der Formel (I), die hergestellt werden soll, 1 bedeutet, mit einem Oxidationsmittel, vorzugsweise m-Chlorperbenzoesäure, behandelt wird, so daß man nach der eventuellen Reinigung durch Chromatographie und/oder Kristallisation ein Derivat der Formel (I/F) erhält:

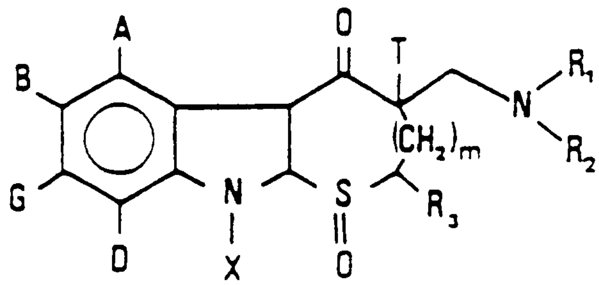

(I/F)

einem Sonderfall der Derivate der Formel (I), in der A, B, G, D, X, T, $R_1$, $R_2$, $R_3$ und m die bezüglich der Formel (I) angegebenen Bedeutungen besitzen und n 1 bedeutet,
welches Derivat der Formel (I/A), (I/B), (I/C1), (I/C), (I/D), (I/E) oder (I/F) gewünschtenfalls in seine Isomeren aufgetrennt und/oder in ein Salz überführt werden kann zur Erleichterung der Reinigung bei den eventuellen späteren Stufen, oder auch mit einer pharmazeutisch annehmbaren Säure in Salz überführt werden und gegebenenfalls für die therapeutische Anwendung kristallisiert werden kann.

2. Verfahren nach Anspruch 1 zur Herstellung von Verbindungen der allgemeinen Formel (I/B1):

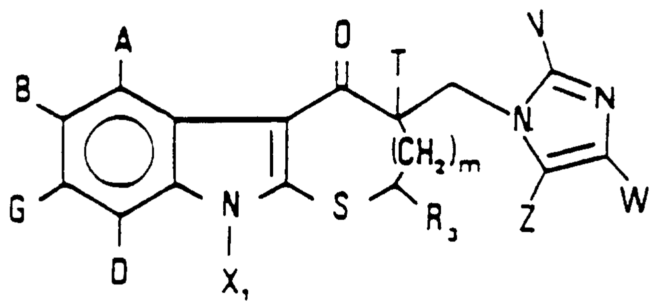

(I/B1)

in der

A, B, G, D, T, $R_3$ und m die bezuglich der Formel (I) in Anspruch 1 angegebenen Bedeutungen besitzen,

$X_1$ ein Wasserstoffatom oder eine Niedrigalkylgruppe darstellt und

V, W und Z, die gleichartig oder verschieden sein können, jeweils unabhängig voneinander eine geradkettige oder verzweigte Niedrigalkyl- oder Niedrigalkoxygruppe oder eine ihrerseits gegebenenfalls durch eine Niedrigalkylgruppe, eine Niedrigalkoxygruppe, ein Halogenatom oder eine Trifluormethylgruppe substituierte Phenylgruppe bedeuten,

**dadurch gekennzeichnet**, daß man eine Verbindung der Formel (I/A):

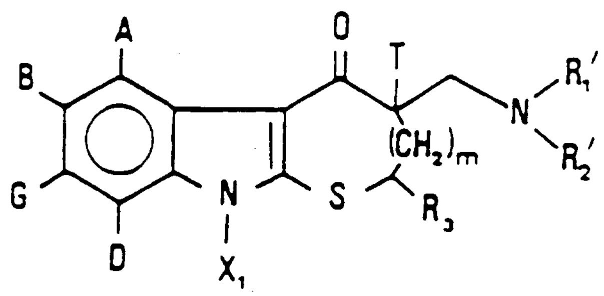

(I/A)

einem Sonderfall der Derivate der Formel (I),
in der A, B, G, D, $R_3$, T, m und $X_1$ die in Anspruch 1 angegebenen Bedeutungen besitzen und $R'_1$ und $R'_2$ Niedrigalkylgruppen bedeuten, welches man erforderlichenfalls durch Chromatographie und/oder Kristallisation gereinigt hat, mit einem Derivat der Formel (M):

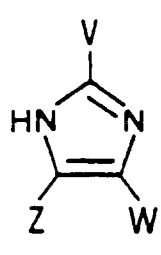

(M)

in der V, W und Z die oben angegebenen Bedeutungen besitzen, behandelt, so daß man eine Verbindung der Formel (I/B1) erhält, die man gewünschtenfalls in seine Isomeren auftrennen oder mit einer pharmazeutisch annehmbaren Säure in ein Salz überführen und gegebenenfalls kristallisieren kann.

**Patentsansprüche für folgenden Vertragsstaat : GR**

**1.** Verfahren zur Herstellung von Verbindungen der allgemeinen Formel (I):

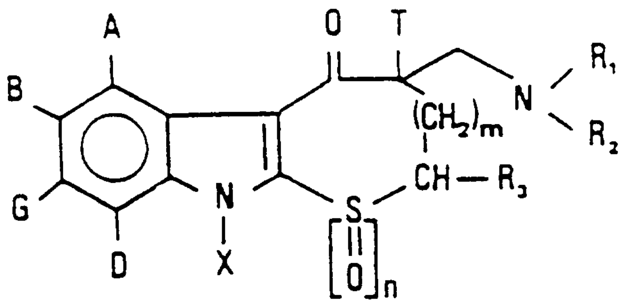

(I)

in der

A, B, G, D, die gleichartig oder verschieden sein können, jeweils ein Wasserstoffatom oder ein Halogenatom, eine Niedrigalkoxygruppe oder eine geradkettige oder verzweigte Niedrigalkylgruppe, die gegebenenfalls durch eines oder mehrere Halogenatome substituiert ist,

X ein Wasserstoffatom, eine geradkettige oder verzweigte Niedrigalkylgruppe oder eine Gruppe $SO_2E$, worin E eine geradkettige oder verzweigte Niedrigalkylgruppe oder Phenylgruppe, die gegebenenfalls durch eine geradkettige oder verzweigte Niedrigalkylgruppe substituiert ist, darstellt,

T ein Wasserstoffatom oder eine Niedrigalkylgruppe,

$R_3$ ein Wasserstoffatom oder eine geradkettige oder verzweigte Niedrigalkylgruppe oder eine Phenylgruppe, die gegebenenfalls durch eine oder mehrere geradkettige oder verzweigte Niedrigalkylgruppen substituiert ist,

n und m, die gleichartig oder verschieden sein können, jeweils 0 oder 1,

$R_1$ und $R_2$, die gleichartig oder verschieden sein können, jeweils ein Wasserstoffatom oder eine geradkettige oder verzweigte Niedrigalkylgruppe

oder

$R_1$ und $R_2$ gemeinsam mit dem Stickstoffatom, an das sie gebunden sein, ein mono- oder bicyclisches, gesättigtes oder ungesättigtes stickstoffhaltiges heterocyclisches System, wobei jeder Ring sechs oder sieben Ringglieder aufweist und gegebenenfalls eines oder zwei weitere Heteroatome, ausgewählt aus Stickstoff-, Sauerstoff- und Schwefelatomen, umfaßt und gegebenenfalls durch eine oder mehrere geradkettige oder verzweigte Niedrigalkyl- oder Niedrigalkoxygruppen oder durch eine ihrerseits gegebenenfalls durch eine oder mehrere Niedrigalkylgruppen, Niedrigalkoxygruppen, Trifluormethylgruppen oder eines oder mehrere Halogenatome substituierte Arylgruppe substituiert ist,

oder

$R_1$ eine Gruppe

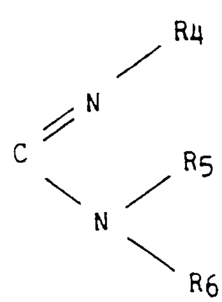

in der $R_4$, $R_5$ und $R_6$, die gleichartig oder verschieden sein können, jeweils ein Wasserstoffatom oder eine geradkettige oder verzweigte Niedrigalkylgruppe darstellen oder $R_4$ zusammen mit $R_5$ eine Brücke $(CH_2)_p$ darstellt, worin p zwischen 2 und 4 liegt,
oder $R_1$ eine 10,11-Dihydro[5H]dibenzo[a,b]cyclohepten-5-yl-gruppe und $R_2$ ein Wasserstoffatom bedeuten,

und gegebenenfalls von deren Isomeren sowie deren Additionssalzen mit einer pharmazeutisch annehmbaren Säure,
wobei unter Niedrigalkylgruppen oder Niedrigalkoxygruppen Gruppen mit 1 bis 6 Kohlenstoffatomen zu verstehen sind, **dadurch gekennzeichnet**, daß man ein Derivat der Formel (II):

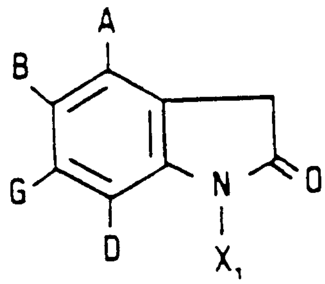

(II)

in der A, B, G und D die bezüglich der Formel (I) angegebenen Bedeutungen besitzen und
$X_1$ ein Wasserstoffatom oder eine Niedrigalkylgruppe bedeutet, in Gegenwart eines Thionierungsmittels, wie Phosphorpentasulfid, kondensiert, so daß man ein Derivat der Formel (III):

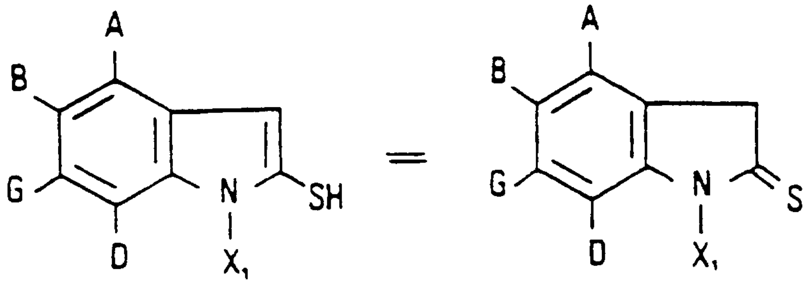

(III)

erhält, in der A, B, G, D und $X_1$ die oben angegebenen Bedeutungen besitzen, welches man mit einem Derivat der Formel (IV):

$JCHR_3\text{-}(CH_2)_m\text{-}CTK\text{-}COOR$ (IV)

in der
entweder m 0 und in diesem Fall J und K gemeinsam eine $\pi$-Bindung,
oder

m 1 und in diesem Fall J ein Halogenatom und K ein Wasserstoffatom und
R ein Wasserstoffatom oder eine geradkettige oder verzweigte Niedrigalkylgruppe bedeuten und T und $R_3$ die bezüglich der Formel (I) angegebenen Bedeutungen besitzen,

behandelt, so daß man ein Derivat der Formel (V):

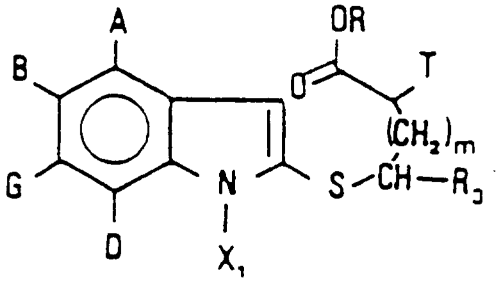

(V)

erhält, in der A, B, G, D, T, R, $R_3$, $X_1$ und m die oben angegebenen Bedeutungen besitzen,

welches, wenn R von H verschieden ist, der Einwirkung eines alkalischen Mittels unterworfen wird, so daß man ein Derivat der Formel (Va) erhält, einem Sonderfall der Derivate der Formel (V), worin R H bedeutet,

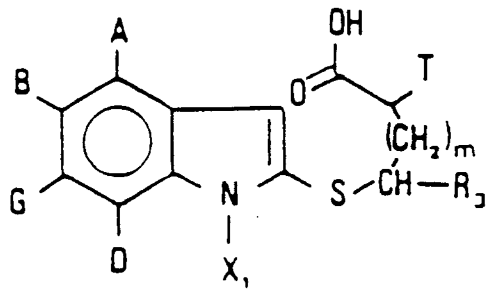

(Va)

welches man der Einwirkung eines vorzugsweise unter einer Stickstoffatmosphäre hergestellten Polyphosphatesters unterwirft, so daß man ein Derivat der Formel (VI) erhält:

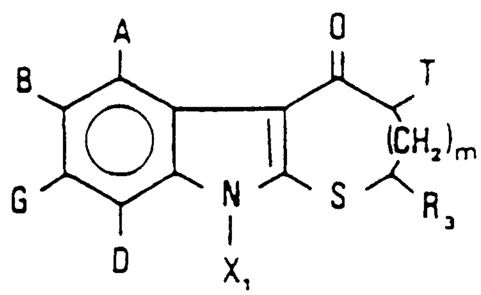

(VI)

In der A, B, G, D, T, m, $X_1$ und $R_3$ die oben angegebenen Bedeutungen besitzen, welches man anschließend vorzugsweise unter einer Stickstoffatomosphäre in Gegenwart eines Dialkylamins der Formel

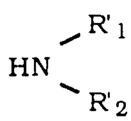

in der $R'_1$ und $R'_2$ Niedrigalkylgruppen bedeuten, oder eines seiner Salze mit einer starken Säure mit Paraformaldehyd behandelt,

so daß man ein Derivat der Formel (I/A) erhält, einem Sonderfall der Derivate der Formel (I):

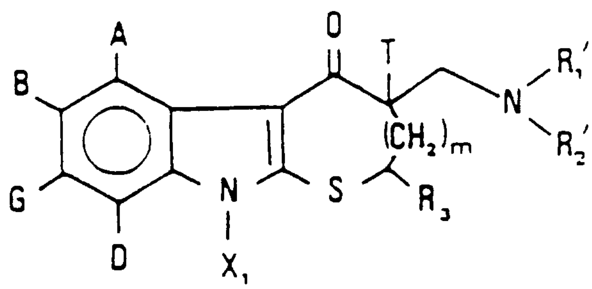

(I/A)

in der A, B, G, D, $R_3$, T, m und $X_1$ die oben angegebenen Bedeutungen besitzen und $R'_1$ und $R'_2$ Niedrigalkylgruppen bedeuten, welches man erforderlichenfalls durch Chromatographie und/oder Kristallisation reinigt, welches man

- wenn in dem Derivat der Formel (I), welches hergestellt werden soll, $R_1$ und $R_2$ zusammen mit dem Stickstoffatm, an das sie gebunden sind, ein heterocyclisches System bilden, wie es bezüglich der Formel (I) definiert ist, anschließend mit einer mono- oder bicyclischen, gesättigten oder ungesättigten stickstoffhaltigen heterocyclischen Verbindung, an dessen Stickstoffatom ein Wasserstoffatom gebunden ist, wobei jeder Ring fünf oder sechs Ringglieder und gegebenenfalls eines oder zwei weitere Heteroatome, die aus Stickstoff-, Sauerstoff- oder Schwefelatomen ausgewählt sind, aufweist und gegebenenfalls durch eine oder mehrere geradkettige oder verzweigte Niedrigalkyl- oder Niedrigalkoxygruppen oder eine ihrerseits gegebenenfalls durch eine oder mehrere Niedrigalkylgruppen, Niedrigalkoxygruppen oder einem oder mehrere Halogenatome oder Trifluormethylgruppen substituierte Arylgruppe substituiert ist, behandelt, so daß man eine Verbindung der Formel (I/B) erhält:

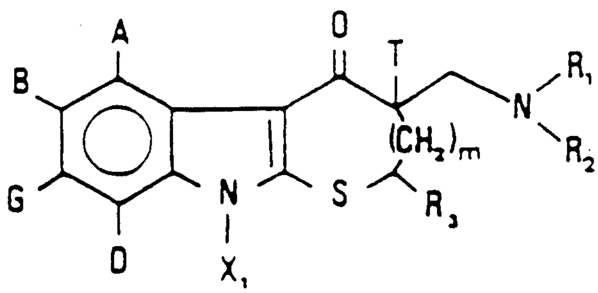

(I/B)

einem Sonderfall der Derivate der Formel (I), in der A, B, G, D, $R_3$, T, m und $X_1$ die oben angegebenen Bedeutungen besitzen und $R_1$ und $R_2$ zusammen mit dem Stickstoffatom, an das sie gebunden sind, ein heterocyclisches System bilden, wie es oben definiert ist,

- dann, wenn $R_1$ und $R_2$ in dem Derivat der Formel (I), welches hergestellt werden soll, jeweils ein Wasserstoffatom bedeuten, mit 10,11-Dihydro-[5H]-dibenzo[a,b]cyclohepten-5-yl-amin behandelt, so daß man nach der Reinigung durch Chromatographie und Kristallisation ein Derivat der Formel (I/C1) erhält:

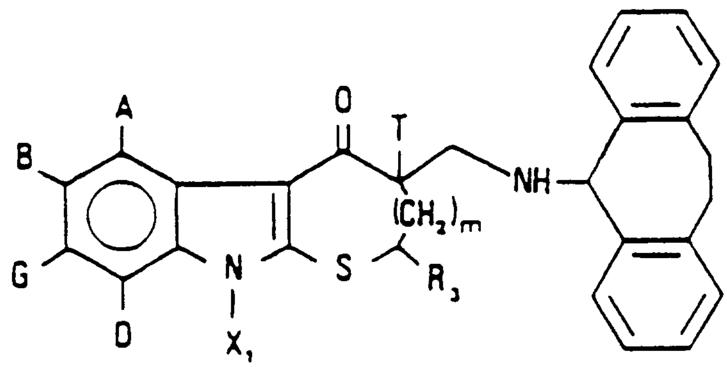

(I/C1)

einem Sonderfall der Formel (I), in der A, B, G, D, $R_3$, T, m und $X_1$ die oben angegebenen Bedeutungen besitzen, welches man in gegebenenfalls verdünnter Essigsäure erhitzt, so daß man nach der eventuellen Kristallisation ein Derivat der Formel (I/C) erhält:

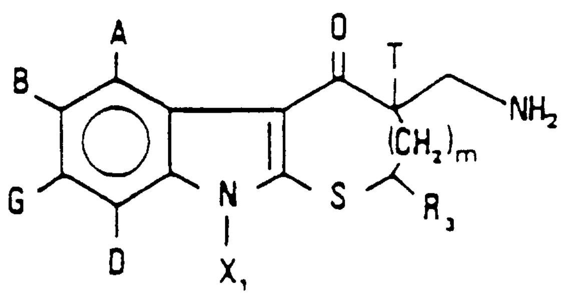

(I/C)

einem Sonderfall der Derivate der Formel (I), in der A, B, G, D, $R_3$, T, m und $X_1$ die oben angegebenen Bedeutungen besitzen,
welches man erforderlichenfalls durch Chromatographie und/oder Kristallisation reinigt und welches dann, wenn $R_1$ in dem Derivat der Formel (I), welches hergestellt werden soll, eine Gruppe der Formel:

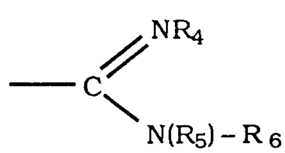

darstellt, in der $R_4$, $R_5$ und $R_6$ die bezüglich der Formel (I) angegebenen Bedeutungen besitzen, mit einem Salz eines Derivats der Formel (VII):

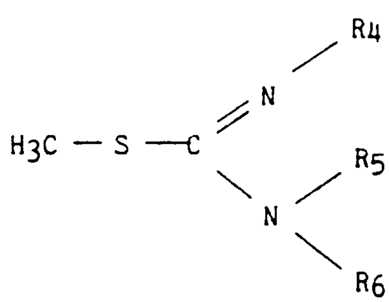

(VII)

in der $R_4$, $R_5$ und R6 die bezüglich der Formel (I) angegebenen Bedeutungen besitzen, mit einer

starken Säure behandelt,
so daß man nach der eventuellen Reinigung durch Chromatographie, der Behandlung mit einem Schwermetallsalz und gegebenenfalls der Kristallisation ein Derivat der Formel (I/D) erhält:

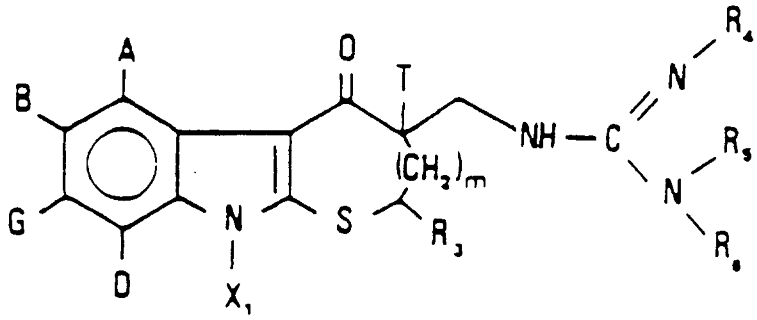

(I/D)

einem Sonderfall der Derivate der Formel (I), in der $R_1$ eine Gruppe der Formel:

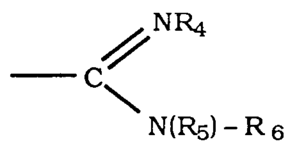

und $R_2$ ein Wasserstoffatom bedeuten,
und worin A, B, G, D, $R_3$, T, m und $X_1$ die oben angegebenen Bedeutungen besitzen,
welches dann, wenn $X_1$ ein Wasserstoffatom darstellt, in Abhängigkeit von der Verbindung der Formel (I), die hergestellt werden soll, der Einwirkung einer Verbindung der Formel (VIII):

$Hal\text{-}SO_2E$ (VIII)

In der E die bezüglich der Formel (I) angegebenen Bedeutungen besitzt und Hal ein Halogenatom darstellt, in Gegenwart von Natriumhydrid unterworfen werden kann, so daß man nach der Extraktion und der eventuellen Reinigung durch Säulenchromatographie ein Derivat der Formel (I/E) erhält:

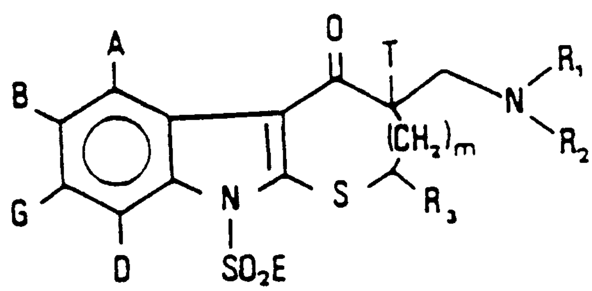

(I/E)

einem Sonderfall der Derivate der Formel (I) in der X eine Gruppe der Formel $SO_2E$ darstellt und A, B, G, D, $R_1$, $R_2$, $R_3$, T, m und E die bezüglich der Formel (I) angegebenen Bedeutungen besitzen,
welches Derivat der Formel (I/A), (I/B), (I/C1), (I/C), (I/D) oder (I/E) dann, wenn n in der Verbindung der Formel (I), die hergestellt werden soll, 1 bedeutet, mit einem Oxidationsmittel, vorzugsweise m-Chlorperbenzoesäure, behandelt wird, so daß man nach der eventuellen Reinigung durch Chromatographie und/oder Kristallisation ein Derivat der Formel (I/F) erhält:

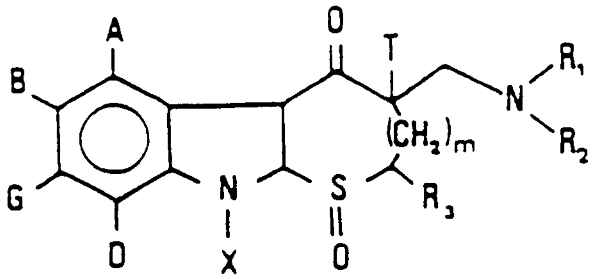

(I/F)

einem Sonderfall der Derivate der Formel (I), in der A, B, G, D, X, T, $R_1$, $R_2$, $R_3$ und m die bezuglich der Formel (I) angegebenen Bedeutungen besitzen und n 1 bedeutet,

welches Derivat der Formel (I/A), (I/B), (I/C1), (I/C), (I/D), (I/E) oder (I/F) gewünschtenfalls in seine Isomeren aufgetrennt und/oder in ein Salz überführt werden kann zur Erleichterung der Reinigung bei den eventuellen späteren Stufen, oder auch mit einer pharmazeutisch annehmbaren Säure in Salz überführt werden und gegebenenfalls für die therapeutische Anwendung kristallisiert werden kann.

2. Verfahren nach Anspruch 1 zur Herstellung von Verbindungen der allgemeinen Formel (I/B1):

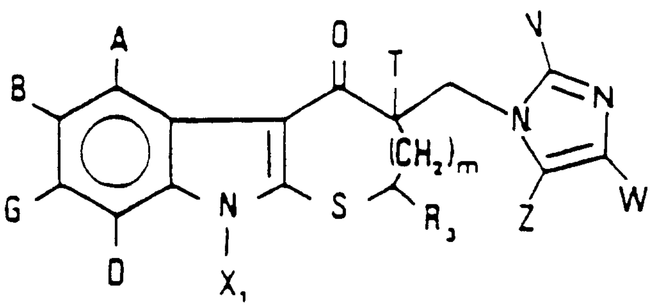

(I/B1)

in der

A, B, G, D, T, $R_3$ und m die bezüglich der Formel (I) in Anspruch 1 angegebenen Bedeutungen besitzen,

$X_1$ ein Wasserstoffatom oder eine Niedrigalkylgruppe darstellt und

V, W und Z, die gleichartig oder verschieden sein können, jeweils unabhängig voneinander eine geradkettige oder verzweigte Niedrigalkyl- oder Niedrigalkoxygruppe oder eine ihrerseits gegebenenfalls durch eine Niedrigalkylgruppe, eine Niedrigalkoxygruppe, ein Halogenatom oder eine Trifluormethylgruppe substituierte Phenylgruppe bedeuten,

**dadurch gekennzeichnet**, daß man eine Verbindung der Formel (I/A):

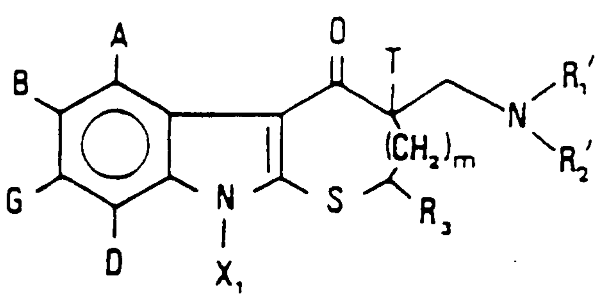

(I/A)

einem Sonderfall der Derivate der Formel (I),

in der A, B, G, D, $R_3$, T, m und $X_1$ die in Anspruch 1 angegebenen Bedeutungen besitzen und $R'_1$ und $R'_2$ Niedrigalkylgruppen bedeuten, welches man erforderlichenfalls durch Chromatographie und/oder Kristallisation gereinigt hat, mit einem Derivat der Formel (M):

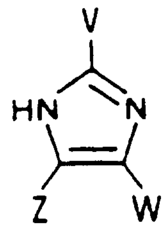

(M)

in der V, W und Z die oben angegebenen Bedeutungen besitzen, behandelt, so daß man eine Verbindung der Formel (I/B1) erhält, die man gewünschtenfalls in seine Isomeren auftrennen oder mit einer pharmazeutisch annehmbaren Säure in ein Salz überführen und gegebenenfalls kristallisieren kann.